# EUROPEAN PATENT APPLICATION

(11) **EP 4 755 891 A1**
(43) Date of publication of application: **10.06.2026**
(21) Application number: 24848166.5
(22) Date of filing: 26.07.2024
(51) Int. Cl.: C07D 495/04, C07D 333/76, A61K 31/15, A61K 31/431, A61P 35/04, A61P 35/00

(54) **ARF1 INHIBITOR AND USE THEREOF**

(30) Priority: 28.07.2023 CN 202310944525
(71) Applicant: AOBIO PHARMACEUTICAL CO., LTD., Shanghai 201800 (CN)
(72) Inventor: HOU, Steven Xianyu, Shanghai 201800 (CN); WANG, Yuetong, Shanghai 201800 (CN); LUO, Chenfei, Shanghai 201800 (CN); LI, Haisen, Shanghai 201800 (CN); HUANG, Zhigang, Shanghai 201800 (CN)
(74) Representative: CAPRI
(86) International application number: PCT/CN2024/107778
(87) International publication number: WO 2025/026209

(57) **Abstract**

A compound of formula (I), or a stereoisomer, isotopic variant, pharmaceutically acceptable salt or solvate thereof, wherein R₁, R₂, R₃, R₄, X₁, X₂, X₃, X₄, X₅, X₆, X₇, X₈, X₉, X₁₀, L, ring A, ring B, ring C, and ring D are as described herein. The compound of formula (I), or the stereoisomer, isotopic variant, pharmaceutically acceptable salt or solvate thereof can be used as an Arf1 inhibitor. Further provided are a pharmaceutical composition containing the compound of formula (I), or the stereoisomer, isotopic variant, pharmaceutically acceptable salt or solvate thereof, and a use in the treatment or prevention of a disease, such as cancer or tumor, associated with Arf1 pathway activity in a subject. Further provided is a use of the compound of formula (I), or the stereoisomer, isotopic variant, pharmaceutically acceptable salt or solvate thereof in the preparation of a therapeutic vaccine for retarding tumor development.

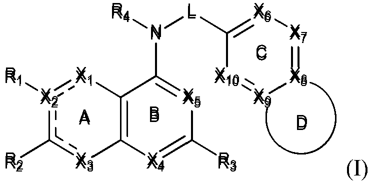

## Description

### TECHNICAL FIELD

The present disclosure relates to a class of heterocyclic compounds, pharmaceutically acceptable salts thereof, solvates thereof, stereoisomers thereof, or isotopic variants thereof, which can be used as a novel class of COPI/Arf1-lipolysis-β-oxidation pathway inhibitors (also referred to as Arf1 inhibitors). The present disclosure also relates to a pharmaceutical composition comprising these compounds as active ingredients, and the use thereof in treating or preventing a disease associated with Arf1 pathway activity in a subject, such as cancer or a tumor, and the use of these compounds in the manufacture of a therapeutic vaccine for blocking tumor development.

### BACKGROUND

Cancer is a leading cause of death worldwide, which leads to nearly 10 million deaths in 2021. Despite advances in treating some types of cancer through treatments like surgery, radiotherapy, and chemotherapy, many types of cancer remain essentially incurable. Even when an effective treatment exists for a specific cancer, the side effects of such therapy may be severe, leading to a significant decline in quality of life.

Immunotherapies employing immune checkpoint blockade (ICB) and adoptive T-cell therapy (ACT) have achieved landmark clinical efficacy in several advanced cancers, including melanoma, renal cancer, and lung cancer. ICB therapies promote the proliferation of PD-1⁺TCF1⁺CD8⁺ tumor-infiltrating stem-like T cells (Im *et al.*, 2016; Siddiqui *et al.*, 2019) and act on different immune cell types within the tumor microenvironment (TME) to enhance the anti-tumor effects of CD8⁺ T cells (Kurtulus *et al.*, 2019). However, most patients do not benefit from these interventions, particularly those with poorly immunogenic tumors such as liver cancer and triple-negative breast cancer (TNBC) patients (Schmid *et al.*, 2020; Yu *et al.*, 2021; Zhao *et al.*, 2020). This is due to a range of underlying factors, including the systemic loss of CD8⁺ T cells, suppression of T cell activation, poor T cell persistence, or T cell exhaustion (Bruni *et al.*, 2020; Chen and Mellman, 2017; *et al.*, 2019; Sharma *et al.*, 2017; Spranger *et al.*, 2018; Thorsson *et al.,* 2018; Tumeh *et al.,* 2014). These immunosuppressive factors are primarily influenced by the TME (Gupta *et al.,* 2022). The tumor community consists of heterogeneous tumor cells, stromal cells (such as adipocytes and fibroblasts), immune cells, and other cells. A major ongoing effort is to improve the immunotherapy by modifying the TME (Binnewies *et al.,* 2018). Cancer cells are the primary players in regulating the TME (Wellenstein and de Visser, 2018). Tumors exhibit significant intratumoral heterogeneity. The groundbreaking research by John Dick and his colleagues revealed that leukemia and many other solid cancers have been shown to maintain a hierarchical structure. In most cases, a small number of leukemia stem cells (LSCs) or cancer stem cells (CSCs), also namely tumor-initiating cells, are known to reside at the apex and exhibit unlimited self-renewal capacity. This characteristic enables tumor regeneration and recurrence even after seemingly successful treatment (Trumpp and Haas, 2022). The CSCs may serve as the cellular source driving tumor recurrence, as they possess the ability to enter a reversible quiescent/dormant state, enabling their resistance to standard chemotherapy and the formation of a cell reservoir. The TME is considered to be the niche for CSCs and regulates their phenotypic plasticity. The TME forms an interactive and immunosuppressive environment with tumor cells, immune cells such as tumor-associated macrophages (TAMs), *etc.* (Hass *et al.,* 2020). A stem-like subpopulation inside the tumor hemisphere of solid tumors has been found to maintain an immunosuppressive and therapy-evading tumor niche (Jain *et al.,* 2021). In TNBC, quiescent cancer stem cells (QCSCs) were recently shown to constitute an immunosuppressive niche by orchestrating a locally hypoxic immunosuppressive environment characterized by dysfunctional dendritic cells (DCs), suppressive fibroblasts, reduced T cell infiltration, and increased T cell exhaustion (Baldominos *et al.,* 2022). To activate cytotoxic CD8⁺ T cells for tumor elimination, tumor-specific antigen peptides must be presented on the cell surface via human leukocyte antigen (HLA) class I molecules. Several types of CSCs have been found to downregulate the expression of HLA class I molecules (Muller *et al.,* 2020). The CSCs also express CD47α, which inhibits macrophage phagocytosis by interacting with signal regulatory proteins (Muller *et al.,* 2020). Therefore, developing novel strategies to eradicate CSCs and alter their immunosuppressive environment will be crucial for improving the immunotherapy responses in cancer treatment.

For many years, the inventors have been dedicated to research on stem cells and cancer stem cells. Recently, the inventors conducted a genome-wide RNAi screen in Drosophila intestinal stem cells (ISCs) and identified a set of genes (including genes in the Arf1-mediated lipid metabolism pathway) whose knockdown can induce cytokine release, affect their microenvironment, and subsequently lead to stem cell death. The specific action mechanisms of these genes vary, but they share a common feature. This feature is that their knockout first releases factors from stem cells and CSCs, alters the microenvironment, and activates neighboring cells or immune cells, which then feed back to kill the stem cells or CSCs (Aggarwal *et al.,* 2022; Singh *et al.,* 2016; Wang *et al.,* 2020; Zeng *et al.,* 2015). The absence of these genes in the CSCs releases cytokines, converting the TME from an immunosuppressive to an immunostimulatory microenvironment (Wang *et al.,* 2020).

ADP-ribosylation factor 1 (Arf1) is a member of the human Arf gene family, encoding small guanine nucleotide-binding proteins that play key roles in vesicular trafficking and lipid metabolism (D'Souza-Schorey and Chavrier, 2006; Donaldson and Jackson, 2011; Kazmalek *et al.,* 2017; Wilfling *et al.,* 2014). Previous studies have shown that the Arf1 is highly expressed in various human cancers, including but not limited to breast cancer, hepatocellular carcinoma, colorectal cancer, and prostate cancer (Casalou *et al.,* 2016; Wang *et al.,* 2020). Upregulation of Arf1 is negatively correlated with the prognosis of cancer patients (Lang *et al.,* 2017; Wang *et al.,* 2020).

Knocking out the Arf1 pathway in the CSCs triggers a metabolic stress cascade, starting with mitochondrial damage and endoplasmic reticulum (ER) stress, followed by the exposure of damage-associated molecular patterns (DAMPs: CALR, HMGB1, ATP), which thereby alter the tumor microenvironment and activate systemic anti-tumor immune responses, ultimately leading to tumor destruction (Wang *et al.,* 2020). Recently, the inventors tested two novel Arf1 inhibitors (DU101, DU102; described in our previous patent: WO2022/028429A1, PCT/CN2021/110373) and disclosed their new anti-tumor mechanisms. The inventors also demonstrated that these two newly designed Arf1 inhibitors exhibit low toxicity and potent anti-tumor activity in both mouse and patient-derived liver and melanoma tumor xenograft (PDX) models. Their application in preclinical tumor models not only significantly increased the infiltration and activation of cytotoxic CD8⁺ T cells in tumors via ATP, but also induced the formation of tumor-associated stem-like TCF1⁺CD4⁺CD8⁺ double-positive T (DPT) cells through the S1P-S1PR pathway.

Two recent studies have found that tumor-associated CD4⁺CD8⁺ DPT cells can exhibit potent anti-tumor activity in hepatocellular carcinoma (HCC) and other tumors (Schad *et al.,* 2022; Zheng *et al.,* 2020). Since DPT cells primarily exist in the thymus during early development, tumor-associated DPT cells may originate from infiltrating CD4⁺ or CD8⁺ single-positive T cells, expressing both exhaustion and activation markers. DPT cells exhibit robust immune responses and are in an active anti-tumor state. Furthermore, HCC patients enriched in DPT cells show a positive correlation with better treatment outcomes, indicating that DPT cells are superior anti-tumor T cells.

If the tumor persists, active CD8⁺ T cells often become exhausted, so that it is crucial to maintain a functional pool of tumor-targeting CD8⁺ T cell before all tumor cells are eradicated. In cancer patients receiving immunotherapy, the duration of functional anti-tumor T cells is directly correlated with better treatment outcomes (Robbins *et al.,* 2004). ICBs have been shown to inhibit tumors not by reversing T cell exhaustion but by promoting the proliferation of stem-like T cell subsets. Subsequently, these stem-like T cells generate fresh and effective T cells to replenish the intratumoral exhausted T cells. Stem-like CD8⁺ T cells exhibit superior responses to ICBs and ACT in tumor animal models and cancer patients (Baharon *et al.,* 2021; Eberhardt *et al.,* 2021; Galletti *et* al.,2020; Hashimoto *et* al.,2022; Im *et al*.,2016; Jansen *et al.*,2019; Kurtulus *et al.,* 2019; Krishna *et al*.,2020; Mo *et al.*,2021; Prokhnevska *et al.,* 2023; Siddiqui *et al.,* 2019; Wu *et al.,* 2020; Yost *et al.,* 2019).

The inventors' data demonstrate that treatment with an Arf1 inhibitor enriches the stem-like CD4⁺CD8⁺DPT cell population, which combines the superior properties of both stem-like T cells and CD4⁺CD8⁺ DPT cell subsets, potentially exhibiting more potent anti-tumor activity. Exploring the properties of this unique T cell population have chances to further improve the efficiency of T cell immunotherapy. Thus, treatment with a single agent can overcome three barriers in cancer immunotherapy: increasing the infiltration of CD8⁺ T cells, promoting their activation, and blocking T cell dysfunction or exhaustion. Treatment with these Arf1 inhibitors can transform the tumor immune microenvironment from cold to hot by reprograming lipid metabolism. These Arf1 inhibitors may offer novel cancer immunotherapy strategies and significantly increase the proportion of cancer patients suitable for immunotherapy. The novel Arf1 inhibitors can not only be used to treat poorly immunogenic tumors but also enable the development of more effective T cell-based immunotherapies by combining the inhibitors with ICBs or ACT.

However, the poor water solubility and permeability of DU101 and DU102 greatly limit their transformation potential. Therefore, there is an urgent need to further develop novel Arf1 inhibitors with good water solubility and drugability, and induce potent anti-tumor immunity.

### SUMMARY

The present disclosure is based on the inventors' unexpected discovery that ablating the COPI/Arf1-lipolysis-β-oxidation pathway can eradicate CSCs and induce DAMPs-mediated anti-tumor immune responses, as well as the development and identification of a novel class of Arf1 inhibitors with good water solubility and potent anti-tumor immunity. Therefore, the Arf1 inhibitors of the present disclosure (*i.e.,* the compounds of Formula (I)) are particularly suitable for treating or preventing diseases associated with Arf1 pathway activity; treating refractory, recurrent, or metastatic cancers; selectively killing cancer cells through specific administration regimens; and targeting cancer stem cells (CSCs) by inhibiting the Arf1 pathway, particularly the COPI/Arf1-lipolysis-β-oxidation pathway, to induce anti-tumor immune responses.

In one aspect, the present disclosure provides a compound of Formula (I), a stereoisomer thereof, an isotopic variant thereof, or a pharmaceutically acceptable salt or solvate of any one of the foregoing: 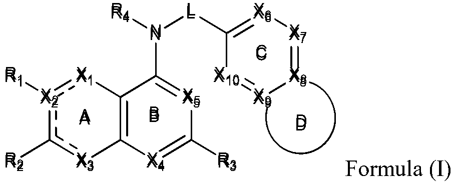 wherein
X₁ is absent or is CH;
X₂ is C or N;
X₃ is S, O, CH, or N;
- - - represents a single bond or a double bond;
X₄, X₅, X₆, X₇, X₈, X₉, and X₁₀ are each independently C(Rₓ) or N, wherein Rₓ is independently selected from the group consisting of H, D (deuterium), CN, OH, halogen, C₁₋₆ alkyl, C₁₋₆ haloalkyl, C₁₋₆ alkoxy, and C₁₋₆ haloalkoxy, for example, the group consisting of H, D, CN, OH, halogen, C₁₋₄ alkyl, C₁₋₄ haloalkyl, C₁₋₄ alkoxy, and C₁₋₄ haloalkoxy, wherein the halogen or halo is F, Cl, Br, or I;
R₁ and R₂ are each independently absent or selected from the group consisting of H, D, CN, OH, halogen, C₁₋₆ alkyl optionally substituted with 1-3 Rₐ, and C₁₋₆ alkoxy optionally substituted with 1-3 R_{b}, or R₁ and R₂ together with the carbon atom to which they are attached form a C₅₋₈ carbocycle optionally substituted with 1-3 R_{c} or a 5- to 8-membered heterocycle optionally substituted with 1-3 R_{c}, wherein the 5- to 8-membered heterocycle optionally contains one or more heteroatoms selected from N, O, and S, for example, 1, 2, or 3 heteroatoms selected from N, O, and S;
R₃ is selected from H, D, CN, OH, halogen, C₁₋₆ alkyl optionally substituted with 1-3 Rₐ, C₁₋₆ alkoxy optionally substituted with 1-3 R_{b}, and N(Rₙ)₂, wherein each Rₙ is independently H, D, C₁₋₆ alkyl optionally substituted with 1-3 Rₐ, or C₆₋₁₄ aryl optionally substituted with 1-3 R_{g};
R₄ is selected from H and C₁₋₆ alkyl optionally substituted with 1-3 Rₐ, such as C₁₋₄ alkyl optionally substituted with 1, 2, or 3 Rₐ;
L is absent or is selected from C₁₋₆ hydrocarbylene optionally substituted with 1-3 R_{d} and C₁₋₆ heterohydrocarbylene optionally substituted with 1-3 Rₑ, wherein the heterohydrocarbylene optionally contains one or more heteroatoms selected from N, O, and S, such as C₁₋₄ hydrocarbylene optionally substituted with 1-3 R_{d} or C₁₋₄ heterohydrocarbylene optionally substituted with 1-3 Rₑ, and further such as C₁₋₂ hydrocarbylene optionally substituted with 1-3 R_{d} or C₁₋₂ heterohydrocarbylene optionally substituted with 1-3 Rₑ;
ring D is absent or is a C₅₋₆ carbocycle optionally substituted with 1-3 R_{f} or a 5- to 6-membered heterocycle optionally substituted with 1-3 R_{f};
each occurrence of Rₐ, R_{b}, R_{c}, R_{d}, Rₑ, R_{f}, and R_{g} is independently selected from the group consisting of D, halogen, C₁₋₆ alkyl, C₁₋₆ haloalkyl, C₁₋₆ alkoxy, C₁₋₆ haloalkoxy, -C(O)-C₁₋₆ alkyl, oxo (=O), and -NH₂, for example, the group consisting of D, halogen, C₁₋₄ alkyl, C₁₋₄ haloalkyl, C₁₋₄ alkoxy, C₁₋₄ haloalkoxy, -C(O)-C₁₋₄ alkyl, oxo (=O), and -NH₂, wherein the halogen or halo is F, Cl, Br, or I;
alternatively, each occurrence of Rₐ, R_{b}, R_{c}, R_{d}, Rₑ, R_{f}, and R_{g} is independently selected from the group consisting of D, halogen, C₁₋₆ alkyl, C₁₋₆ haloalkyl, C₁₋₆ alkoxy, C₁₋₆ haloalkoxy, C₁₋₆ hydroxyalkyl, C₁₋₆ aminoalkyl, -C(O)-C₁₋₆ alkyl, -C(O)-C₁₋₆ haloalkyl, -C(O)-C₁₋₆ hydroxyalkyl, -C(O)-C₁₋₆ aminoalkyl, -C(O)-C₅₋₁₄ aryl, -C(O)-C₅₋₁₄ heteroaryl, oxo (=O), and -NH₂, wherein the C₅₋₁₄ aryl and C₅₋₁₄ heteroaryl are optionally substituted with 1-3 substituents selected from the group consisting of D, halogen, -OH, and -NH₂, for example, the group consisting of D, halogen, C₁₋₄ alkyl, C₁₋₄ haloalkyl, C₁₋₄ alkoxy, C₁₋₄ haloalkoxy, C₁₋₄ hydroxyalkyl, C₁₋₄ aminoalkyl, -C(O)-C₁₋₄ alkyl, -C(O)-C₁₋₄ haloalkyl, -C(O)-C₁₋₄ hydroxyalkyl, -C(O)-C₁₋₄ aminoalkyl, -C(O)-optionally substituted C₅₋₆ aryl, - C(O)-optionally substituted C₅₋₆ heteroaryl, oxo (=O), and -NH₂, wherein the optionally substituted C₅₋₆ aryl and optionally substituted C₅₋₆ heteroaryl are optionally substituted with 1-3 substituents selected from the group consisting of D, halogen, -OH, and -NH₂, wherein the halogen or halo is F, Cl, Br, or I;
alternatively, each occurrence of Rₐ, R_{b}, R_{c}, R_{d}, Rₑ, R_{f}, and R_{g} is independently selected from the group consisting of C₁₋₆ hydroxyalkyl, C₁₋₆ aminoalkyl, -C(O)-C₁₋₆ haloalkyl, -C(O)-C₁₋₆ hydroxyalkyl, -C(O)-C₁₋₆ aminoalkyl, -C(O)-C₅₋₁₄ aryl, and -C(O)-C₅₋₁₄ heteroaryl, wherein the C₅₋₁₄ aryl and C₅₋₁₄ heteroaryl are optionally substituted with 1-3 substituents selected from the group consisting of D, halogen, -OH, and -NH₂, for example, the group consisting of C₁₋₄ hydroxyalkyl, C₁₋₄ aminoalkyl, -C(O)-C₁₋₄ haloalkyl, -C(O)-C₁₋₄ hydroxyalkyl, -C(O)-C₁₋₄ aminoalkyl, -C(O)-optionally substituted C₅₋₆ aryl, and -C(O)-optionally substituted C₅₋₆ heteroaryl, wherein the optionally substituted C₅₋₆ aryl and optionally substituted C₅₋₆ heteroaryl are optionally substituted with 1-3 substituents selected from the group consisting of D, halogen, -OH, and -NH₂, wherein the halogen or halo is F, Cl, Br, or I.

In some embodiments, each occurrence of Rₐ, R_{b}, R_{c}, R_{d}, Rₑ, R_{f}, and R_{g} is independently optionally substituted with 1-3 substituents, for example, 1, 2, or 3 substituents selected from the group consisting of D, halogen, -OH, and -NH₂.

As used herein, "- - -" represents a single bond or a double bond, and those skilled in the art should understand that the resulting compound molecule should satisfy the valence bond theory when "- - -" is a single bond or a double bond.

In some embodiments, the isotopic variant of the compound of Formula (I) is a deuterated derivative, wherein the compound of Formula (I) is optionally substituted with one or more deuterium atoms.

In some embodiments, X₁ is absent, in which case ring A is a C₅ carbocycle or a 5-membered heterocycle, having the structure shown below:

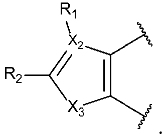

In some embodiments, X₁ is CH, in which case ring A is a C₆ carbocycle or a 6-membered heterocycle, having the structure shown below:

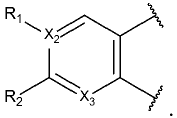

In some embodiments, ring A is selected from the following ring structures:

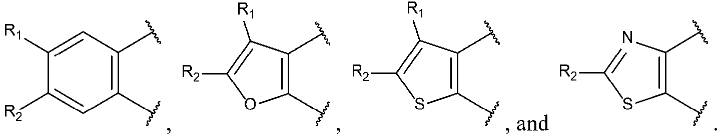

In some embodiments, R₁ and R₂ are each independently absent or selected from the group consisting of H, D (deuterium), CN, OH, halogen, C₁₋₄ alkyl optionally substituted with 1-3 Rₐ such as halo C₁₋₄ alkyl, and C₁₋₄ alkoxy optionally substituted with 1-3 R_{b} such as halo C₁₋₄ alkoxy, for example, H, D, CN, OH, F, Cl, Br, I, methyl, ethyl, propyl, isopropyl, butyl, isobutyl, *tert*-butyl, trifluoromethyl, methoxy, ethoxy, propoxy, butoxy, and the like.

In some embodiments, R₁ and R₂ are each independently absent or selected from the group consisting of H, D (deuterium), CN, OH, halogen, C₁₋₄ alkyl optionally substituted with 1-3 (*e.g.,* 1, 2, or 3) Rₐ, and C₁₋₄ alkoxy optionally substituted with 1-3 (*e.g.,* 1, 2, or 3) R_{b}. In some embodiments, each occurrence of Rₐ is independently selected from D and halogen *(e.g.,* F, Cl, Br, or I), and each occurrence of R_{b} is independently selected from D and halogen (*e.g.,* F, Cl, Br, or I).

In some embodiments, R₁ and R₂ are each independently selected from the group consisting of H, D, CN, OH, halogen, C₁₋₄ alkyl, C₁₋₄ haloalkyl, C₁₋₄ alkoxy, C₁₋₄ haloalkoxy, and the like. In some embodiments, R₁ and R₂ are each independently selected from the group consisting of H, D, CN, OH, F, Cl, Br, I, methyl, ethyl, propyl, isopropyl, butyl, isobutyl, *tert*-butyl, trifluoromethyl, methoxy, ethoxy, propoxy, butoxy, and the like. In some embodiments, R₁ and R₂ are each independently selected from the group consisting of H, D, Cl, methyl, ethyl, trifluoromethyl, methoxy, and the like.

In some specific embodiments, ring A is selected from the following ring structures:

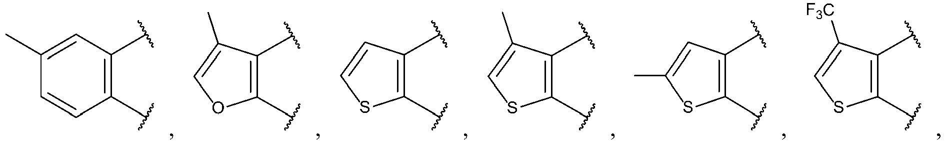

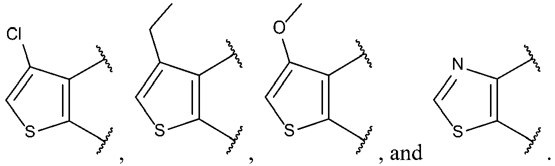

In some embodiments, R₁ and R₂ together with the carbon atom to which they are attached form a C₅₋₈ carbocycle or 5- to 8-membered heterocycle selected from the following ring structures:

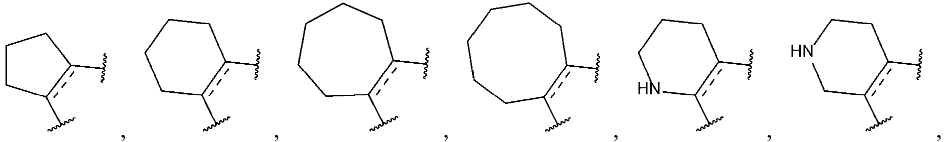

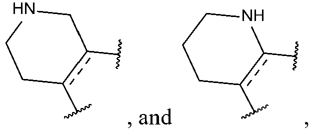

wherein - - - represents a single bond or a double bond, and the C₅₋₈ carbocycle or 5- to 8-membered heterocycle is optionally substituted with 1-3 R_{c}. each occurrence of R_{c} is independently selected from the group consisting of D, halogen, C₁₋₆ alkyl, C₁₋₆ haloalkyl, C₁₋₆ alkoxy, C₁₋₆ haloalkoxy, C₁₋₆ hydroxyalkyl, C₁₋₆ aminoalkyl, -C(O)-C₁₋₆ alkyl, -C(O)-C₁₋₆ haloalkyl, -C(O)-C₁₋₆ hydroxyalkyl, - C(O)-C₁₋₆ aminoalkyl, -C(O)-C₅₋₁₄ aryl, -C(O)-C₅₋₁₄ heteroaryl, oxo (=O), and -NH₂, wherein the C₅₋₁₄ aryl and C₅₋₁₄ heteroaryl are optionally substituted with 1-3 substituents selected from the group consisting of D, halogen, -OH, and -NH₂; for example, the group consisting of halogen, C₁₋₄ alkyl, C₁₋₄ haloalkyl, C₁₋₄ alkoxy, C₁₋₄ haloalkoxy, C₁₋₄ hydroxyalkyl, C₁₋₄ aminoalkyl, -C(O)-C₁₋₄ alkyl, -C(O)-C₁₋₄ haloalkyl, -C(O)-C₁₋₄ hydroxyalkyl, -C(O)-C₁₋₄ aminoalkyl, -C(O)-optionally substituted C₅₋₆ aryl, - C(O)-optionally substituted C₅₋₆ heteroaryl, oxo (=O), and -NH₂, wherein the optionally substituted C₅₋₆ aryl and optionally substituted C₅₋₆ heteroaryl are optionally substituted with 1-3 substituents selected from the group consisting of D, halogen, -OH, and -NH₂. In some embodiments, the optionally substituted aryl and optionally substituted heteroaryl are each independently optionally substituted with 1, 2, or 3 substituents selected from the group consisting of D, halogen, -OH, and -NH₂. In some embodiments, the halogen or halo is F, Cl, Br, or I.

In some embodiments, each occurrence of R_{c} is independently optionally substituted with 1-3 substituents selected from the group consisting of D, halogen, -OH, and -NH₂, for example, the alkyl moiety, aryl moiety, or heteroaryl moiety in R_{c} is further optionally substituted with 1-3 substituents selected from the group consisting of D, halogen, -OH, and -NH₂, such as deuterated alkyl, hydroxyalkyl, deuterated aryl, haloaryl, deuterated heteroaryl, and haloheteroaryl.

In some embodiments, R_{c} is optionally attached to a nitrogen atom on a nitrogen-containing 5-to 8-membered heterocycle. In some embodiments, when R₁ and R₂ together with the carbon atom to which they are attached form a C₅₋₈ carbocycle or a 5- to 8-membered heterocycle, the fused ring structure formed together with ring A is selected from the following structures: 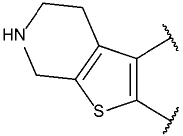 optionally substituted with 1-3 R_{c} and 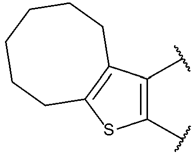 optionally substituted with 1-3 R_{c}, wherein each occurrence of R_{c} is independently selected from the group consisting of D, halogen, C₁₋₆ alkyl such as C₁₋₅ alkyl or C₁₋₄ alkyl, C₁₋₆ haloalkyl such as C₁₋₄ haloalkyl, C₁₋₆ alkoxy such as C₁₋₄ alkoxy, C₁₋₆ haloalkoxy such as C₁₋₄ haloalkoxy, C₁₋₆ hydroxyalkyl such as C₁₋₄ hydroxyalkyl, C₁₋₆ aminoalkyl such as C₁₋₄ aminoalkyl, -C(O)-C₁₋₆ alkyl such as -C(O)-C₁₋₄ alkyl, - C(O)-C₁₋₆ hydroxyalkyl such as -C(O)-C₁₋₄ hydroxyalkyl, -C(O)-C₁₋₆ aminoalkyl such as -C(O)-C₁₋₄ aminoalkyl, -C(O)-optionally substituted C₅₋₁₄ aryl such as -C(O)-optionally substituted C₅₋₆ aryl, - C(O)-optionally substituted C₅₋₁₄ heteroaryl such as -C(O)-optionally substituted C₅₋₆ heteroaryl, oxo (=O), and -NH₂, wherein the optionally substituted aryl and optionally substituted heteroaryl are each independently optionally substituted with 1, 2, or 3 substituents selected from the group consisting of D, halogen, -OH, and -NH₂. In some embodiments, each R_{c} is independently optionally substituted with 1-3 (*e.g.,* 1, 2, or 3) substituents selected from the group consisting of D, halogen, -OH, and -NH₂, for example, the alkyl moiety, aryl moiety, or heteroaryl moiety in R_{c} is further optionally substituted with 1-3 substituents selected from the group consisting of D, halogen, -OH, and -NH₂, such as deuterated alkyl, hydroxyalkyl, deuterated aryl, haloaryl, deuterated heteroaryl, and haloheteroaryl. In some embodiments, R_{c} is optionally attached to a nitrogen atom on a nitrogen-containing 5- to 8-membered heterocycle.

In some specific embodiments, when R₁ and R₂ together with the carbon atom to which they are attached form a C₅₋₈ carbocycle or a 5- to 8-membered heterocycle, the fused ring structure formed together with ring A is selected from the following structures:

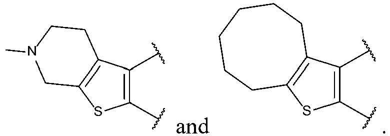

In some specific embodiments, when R₁ and R₂ together with the carbon atom to which they are attached form a C₅₋₈ carbocycle or a 5- to 8-membered heterocycle, the fused ring structure formed together with ring A is selected from the following structures:

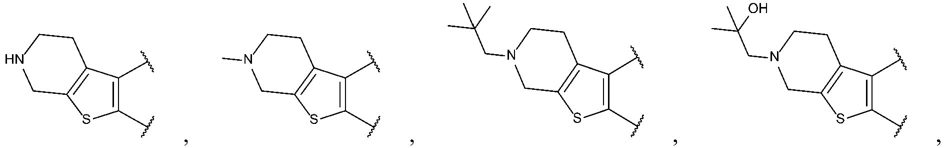

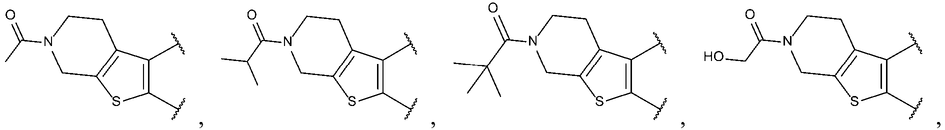

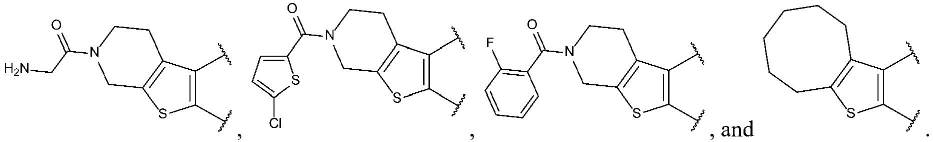

In some specific embodiments, when R₁ and R₂ together with the carbon atom to which they are attached form a C₅₋₈ carbocycle or a 5- to 8-membered heterocycle, the fused ring structure formed together with ring A is selected from the following structures:

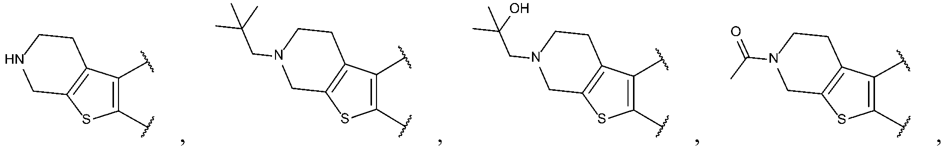

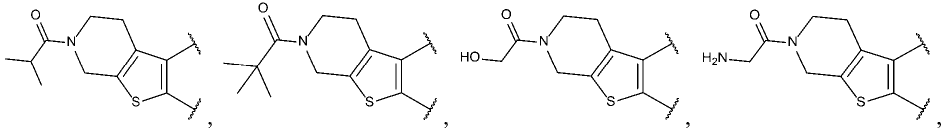

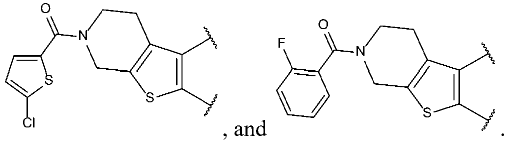

In some embodiments, ring B is selected from the following structures:

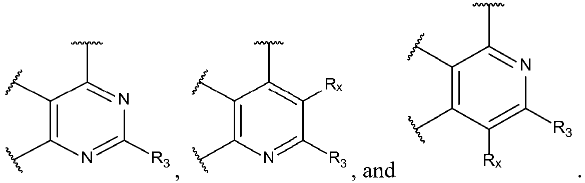

In some specific embodiments, Rₓ substituted on ring B is selected from the group consisting of H, D (deuterium), CN, OH, halogen, C₁₋₄ alkyl, C₁₋₄ haloalkyl, C₁₋₄ alkoxy, and C₁₋₄ haloalkoxy, wherein the halogen or halo is F, Cl, Br, or I.

In some specific embodiments, R₃ is H, D (deuterium), CN, OH, halogen, C₁₋₄ alkyl, C₁₋₄ alkoxy, NH₂, NHRₙ, or N(Rₙ)₂, wherein Rₙ is selected from C₁₋₄ alkyl and phenyl optionally substituted with 1-3 (*e.g.,* 1, 2, or 3) R_{g}, wherein each occurrence of R_{g} is independently selected from the group consisting of halogen, C₁₋₄ alkyl, C₁₋₄ haloalkyl, C₁₋₄ alkoxy, C₁₋₄ haloalkoxy, and -NH₂, wherein the halogen or halo is F, Cl, Br, or I.

In some specific embodiments, ring B is selected from the following ring structures:

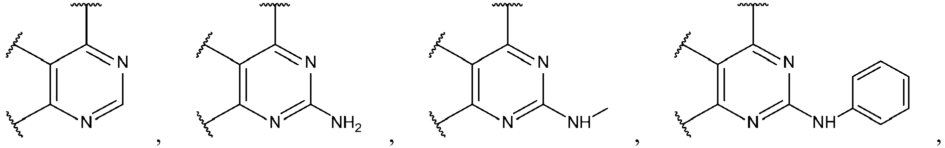

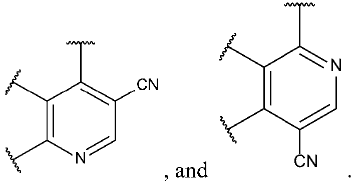

In some embodiments, ring A and ring B together form a ring structure selected from: 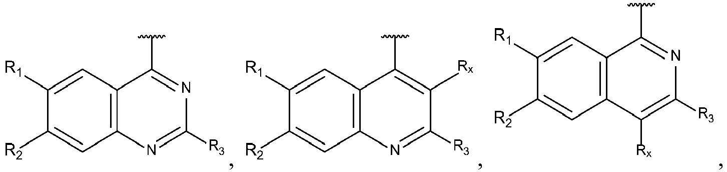 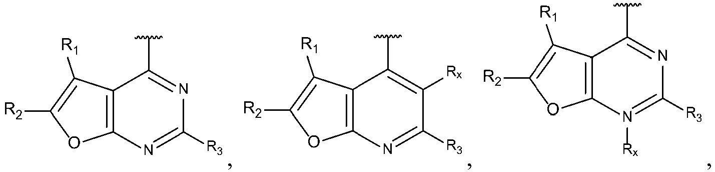 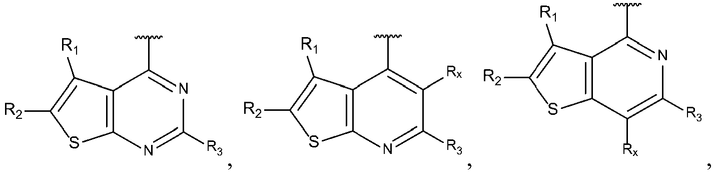 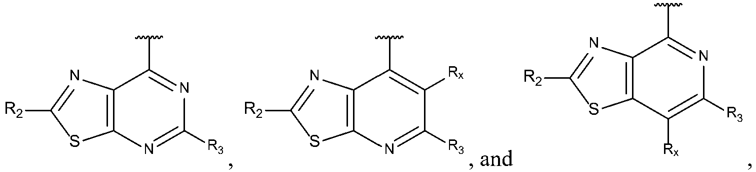 wherein R₁, R₂, R₃, and Rₓ are each independently as defined herein.

In some embodiments, R₁ and R₂ are each independently absent or selected from the group consisting of H, D (deuterium), CN, OH, halogen, C₁₋₄ alkyl optionally substituted with 1-3 (*e.g.,* 1, 2, or 3) Rₐ, and C₁₋₄ alkoxy optionally substituted with 1-3 (*e.g.,* 1, 2, or 3) R_{b}. In some embodiments, R₁ and R₂ are each independently selected from the group consisting of H, D, CN, OH, halogen, C₁₋₄ alkyl, C₁₋₄ haloalkyl, C₁₋₄ alkoxy, C₁₋₄ haloalkoxy, and the like. In some embodiments, R₁ and R₂ are each independently selected from, for example, H, D, CN, OH, F, Cl, Br, I, methyl, ethyl, propyl, isopropyl, butyl, isobutyl, *tert*-butyl, trifluoromethyl, methoxy, ethoxy, propoxy, and butoxy. In some embodiments, Rₓ substituted on ring B is selected from the group consisting of H, D (deuterium), CN, OH, halogen, C₁₋₄ alkyl, C₁₋₄ haloalkyl, C₁₋₄ alkoxy, and C₁₋₄ haloalkoxy, wherein the halogen or halo is F, Cl, Br, or I. In some embodiments, R₃ is H, D (deuterium), CN, OH, halogen, C₁₋₄ alkyl, C₁₋₄ alkoxy, NH₂, NHRₙ, or N(Rₙ)₂, wherein Rₙ is selected from C₁₋₄ alkyl and phenyl optionally substituted with 1-3 (*e.g.,* 1, 2, or 3) R_{g}, wherein each occurrence of R_{g} is independently selected from the group consisting of halogen, C₁₋₄ alkyl, C₁₋₄ haloalkyl, C₁₋₄ alkoxy, C₁₋₄ haloalkoxy, and -NH₂, wherein the halogen or halo is F, Cl, Br, or I.

In some specific embodiments, ring A and ring B together form a ring structure selected from: 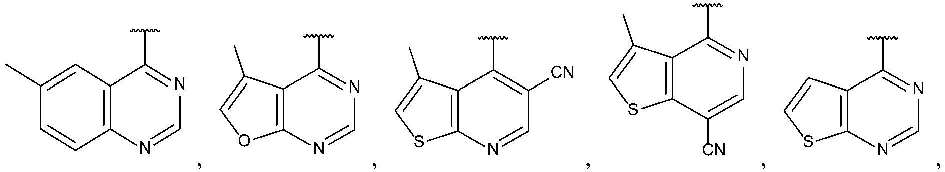 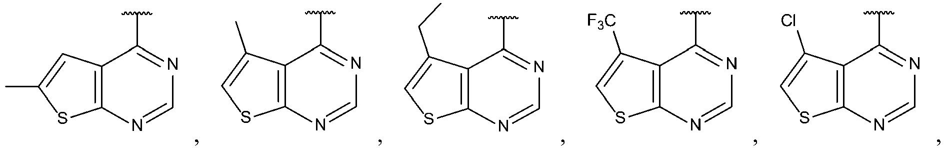 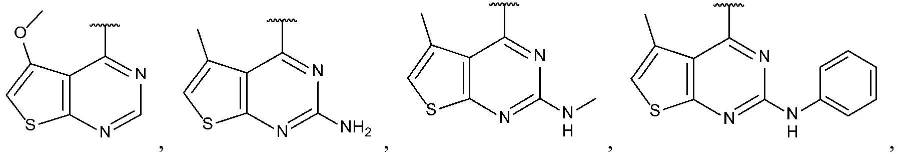 and

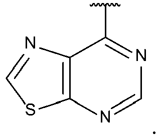

In some embodiments, when R₁ and R₂ together with the carbon atom to which they are attached form a C₅₋₈ carbocycle or a 5- to 8-membered heterocycle, the C₅₋₈ carbocycle or the 5- to 8-membered heterocycle together with ring A and ring B form a fused ring structure selected from the following structures: 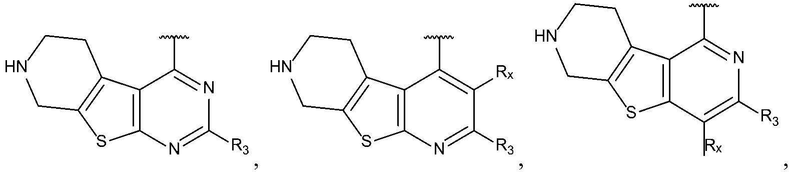 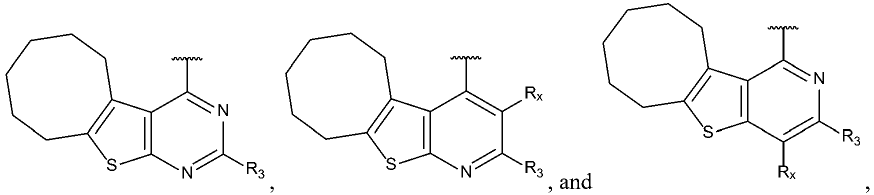 wherein the C₅₋₈ carbocycle or the 5- to 8-membered heterocycle is optionally substituted with 1-3 R_{c}, and each occurrence of R_{c} is independently selected from the group consisting of D, halogen, C₁₋₆ alkyl such as C₁₋₅ alkyl or C₁₋₄ alkyl, C₁₋₆ haloalkyl such as C₁₋₄ haloalkyl, C₁₋₆ alkoxy such as C₁₋₄ alkoxy, C₁₋₆ haloalkoxy such as C₁₋₄ haloalkoxy, C₁₋₆ hydroxyalkyl such as C₁₋₄ hydroxyalkyl, C₁₋₆ aminoalkyl such as C₁₋₄ aminoalkyl, -C(O)-C₁₋₆ alkyl such as -C(O)-C₁₋₄ alkyl, -C(O)-C₁₋₆ hydroxyalkyl such as -C(O)-C₁₋₄ hydroxyalkyl, -C(O)-C₁₋₆ aminoalkyl such as -C(O)-C₁₋₄ aminoalkyl, -C(O)-optionally substituted C₅₋₁₄ aryl such as -C(O)-optionally substituted C₅₋₆ aryl, -C(O)-optionally substituted C₅₋₁₄ heteroaryl such as -C(O)-optionally substituted C₅₋₆ heteroaryl, oxo (=O), and -NH₂, wherein the optionally substituted aryl and optionally substituted heteroaryl are each independently optionally substituted with 1, 2, or 3 substituents selected from the group consisting of D, halogen, - OH, and -NH₂. In some embodiments, each R_{c} is independently optionally substituted with 1-3 (*e.g.,* 1, 2, or 3) substituents selected from the group consisting of D, halogen, -OH, and -NH₂, for example, the alkyl moiety, aryl moiety, or heteroaryl moiety in R_{c} is further optionally substituted with 1-3 substituents selected from the group consisting of D, halogen, -OH, and -NH₂, such as deuterated alkyl, hydroxyalkyl, deuterated aryl, haloaryl, deuterated heteroaryl, and haloheteroaryl. In some embodiments, R_{c} is optionally attached to a nitrogen atom on a nitrogen-containing 5- to 8-membered heterocycle. In some embodiments, Rₓ substituted on ring B is selected from the group consisting of H, D (deuterium), CN, OH, halogen, C₁₋₄ alkyl, C₁₋₄ haloalkyl, C₁₋₄ alkoxy, and C₁₋₄ haloalkoxy, wherein the halogen or halo is F, Cl, Br, or I. In some embodiments, R₃ is H, D (deuterium), CN, OH, halogen, C₁₋₄ alkyl, C₁₋₄ alkoxy, NH₂, NHRₙ, or N(Rₙ)₂, wherein Rₙ is selected from C₁₋₄ alkyl and phenyl optionally substituted with 1-3 (*e.g.,* 1, 2, or 3) R_{g}, wherein each occurrence of R_{g} is independently selected from the group consisting of halogen, C₁₋₄ alkyl, C₁₋₄ haloalkyl, C₁₋₄ alkoxy, C₁₋₄ haloalkoxy, and -NH₂, wherein the halogen or halo is F, Cl, Br, or I.

In some specific embodiments, ring A and ring B together form a ring structure selected from:

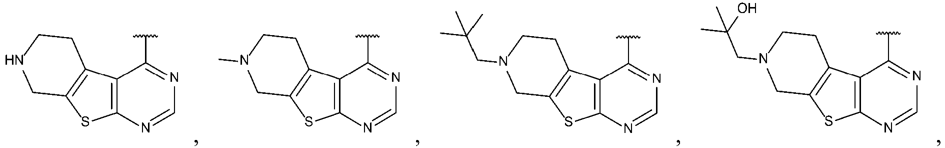

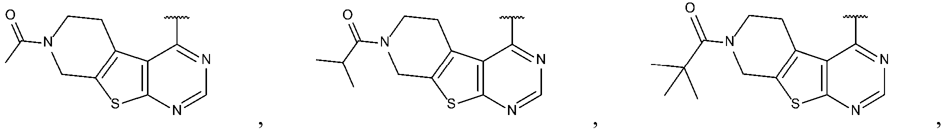

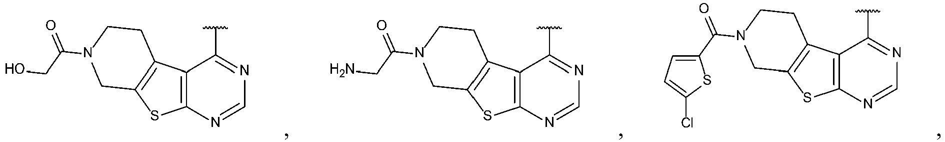

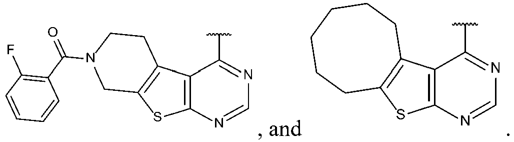

In some embodiments, R₄ is selected from H and C₁₋₆ alkyl optionally substituted with 1-3 Rₐ, such as C₁₋₄ alkyl optionally substituted with 1, 2, or 3 Rₐ, such as methyl, ethyl, propyl, butyl, halomethyl, haloethyl, halopropyl, or halobutyl.

In some embodiments, X₆, X₇, X₈, X₉, and X₁₀ are each independently C(Rₓ).

In some embodiments, when ring D is absent, ring C is a benzene ring optionally substituted with 1-5 Rₓ, such as 1, 2, 3, 4, or 5 Rₓ.

In some embodiments, when ring D is present, ring C is a benzene ring optionally substituted with 1-3 Rₓ, such as 1, 2, or 3 Rₓ.

In some embodiments, any one of X₆, X₇, X₈, X₉, and X₁₀ is N, and the rest are C(Rₓ).

In some embodiments, when ring D is absent, ring C is a pyridine ring optionally substituted with 1-4 Rₓ, such as 1, 2, 3, or 4 Rₓ.

In some embodiments, when ring D is present, ring C is a pyridine ring optionally substituted with 1-2 Rₓ, such as 1 or 2 R_{y}.

In some specific embodiments, the Rₓ substituted on ring C is selected from the group consisting of H, D (deuterium), CN, OH, halogen, C₁₋₄ alkyl, C₁₋₄ haloalkyl, C₁₋₄ alkoxy, and C₁₋₄ haloalkoxy, wherein the halogen or halo is F, Cl, Br, or I.

In some specific embodiments, ring C is selected from the following ring structures:

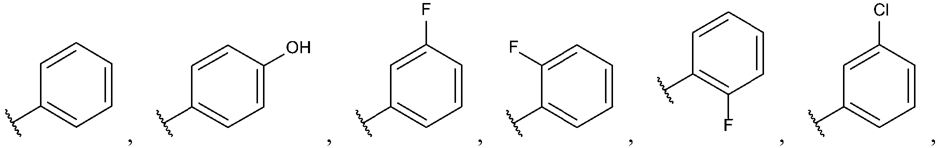

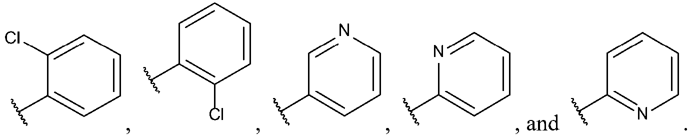

In some specific embodiments, when ring D is present, ring C is selected from the following ring structures:

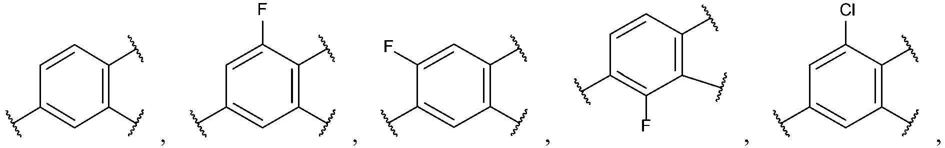

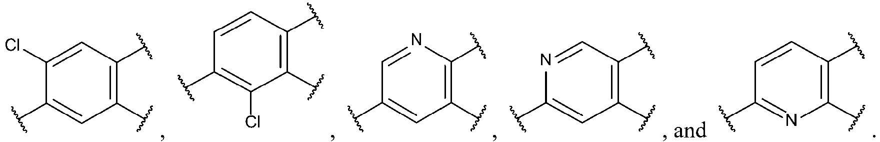

In some embodiments, ring D is selected from the following ring structures optionally substituted with 1-3 R_{f}, such as 1, 2, or 3 R_{f}: 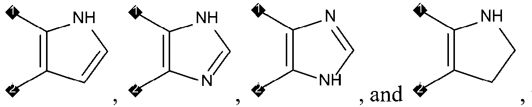 wherein "1" indicates the connection direction to X₈ of ring C, and "2" indicates the connection direction to X₉ of ring C.

In some embodiments, ring D is selected from the following ring structures optionally substituted with 1-3 R_{f}, such as 1, 2, or 3 R_{f}: 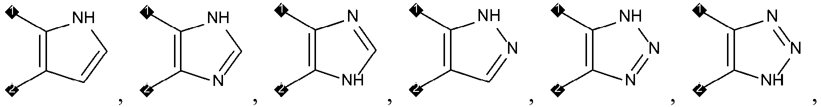 and 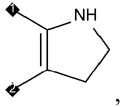 wherein "1" indicates the connection direction to X₈ of ring C, and "2" indicates the connection direction to X₉ of ring C.

In some embodiments, ring D is selected from the following ring structures optionally substituted with 1-3 R_{f}, such as 1, 2, or 3 R_{f}: 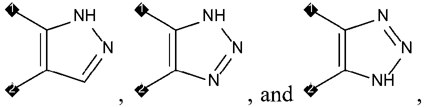 wherein "1" indicates the connection direction to X₈ of ring C, and "2" indicates the connection direction to X₉ of ring C.

In some embodiments, each occurrence of R_{f} is independently selected from the group consisting of D, halogen, C₁₋₆ alkyl, C₁₋₆ haloalkyl, C₁₋₆ alkoxy, and C₁₋₆ haloalkoxy.

In some specific embodiments, each occurrence of R_{f} is independently selected from halogen, C₁₋₄ alkyl (*e.g.,* methyl, ethyl, propyl, isopropyl, butyl, isobutyl, or *tert*-butyl), C₁₋₄ haloalkyl (*e.g.,* trifluoromethyl, trifluoroethyl, or -CH₂CF₃), C₁₋₄ alkoxy (*e.g.*, methoxy, ethoxy, propoxy, or butoxy), C₁₋₄ haloalkoxy, oxo (=O), and the like.

In some specific embodiments, ring D is selected from the following ring structures:

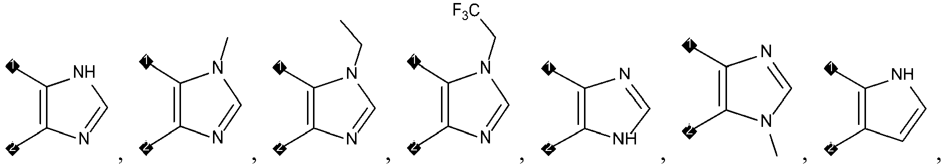

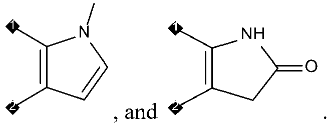

In some specific embodiments, ring D is selected from the following ring structures:

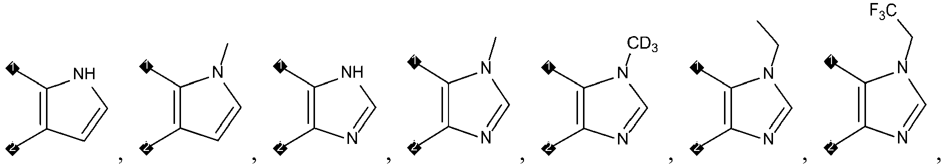

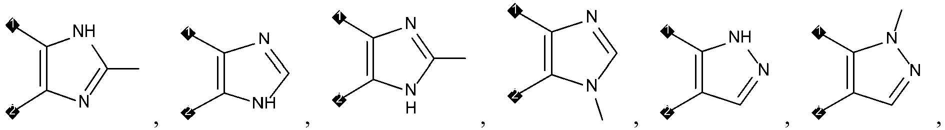

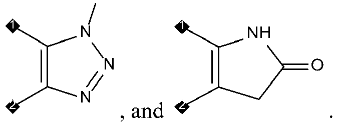

In some specific embodiments, ring D is selected from the following ring structures:

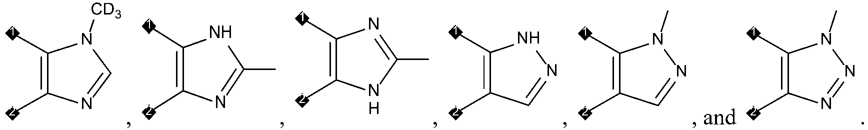

In some embodiments, ring C and ring D together form the following fused ring structure: 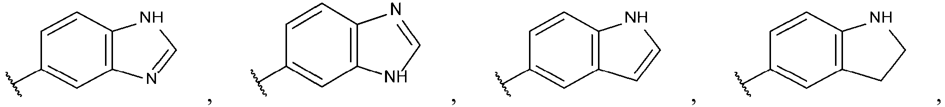 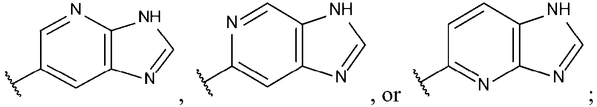 ring C is optionally substituted with 1-3 Rₓ, and ring D is optionally substituted with 1-3 R_{f}.

In some embodiments, ring C and ring D together form the following fused ring structure: 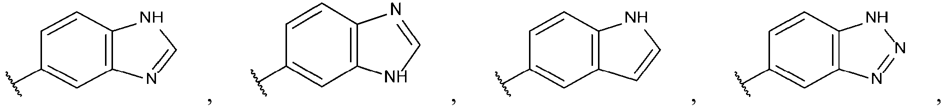 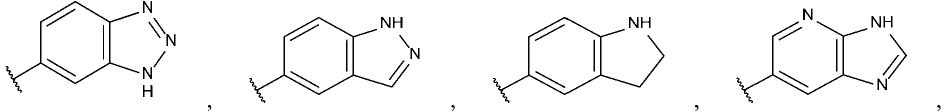 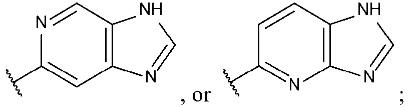 ring C is optionally substituted with 1-3 Rₓ, and ring D is optionally substituted with 1-3 R_{f}.

In some embodiments, ring C and ring D together form the following fused ring structure: 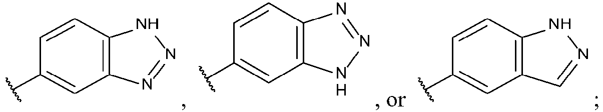 ring C is optionally substituted with 1-3 Rₓ, and ring D is optionally substituted with 1-3 R_{f}.

In some specific embodiments, ring C and ring D together form the following fused ring structure:

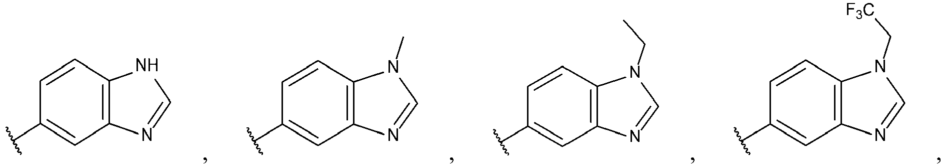

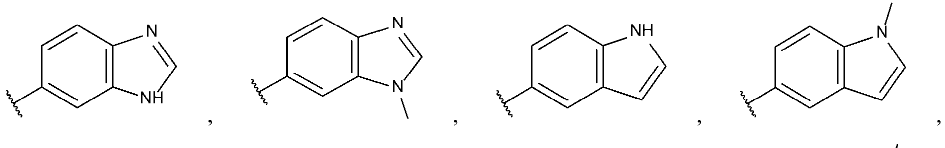

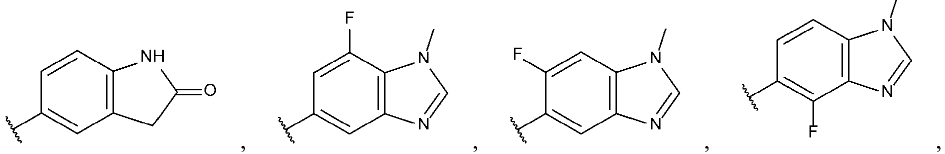

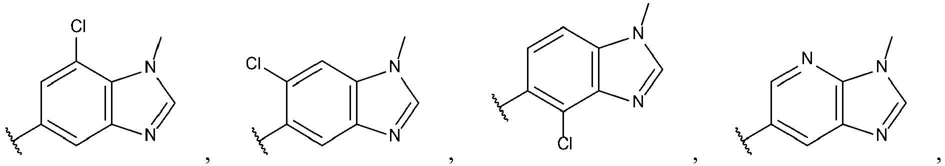

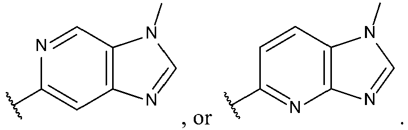

In some specific embodiments, ring C and ring D together form the following fused ring structure:

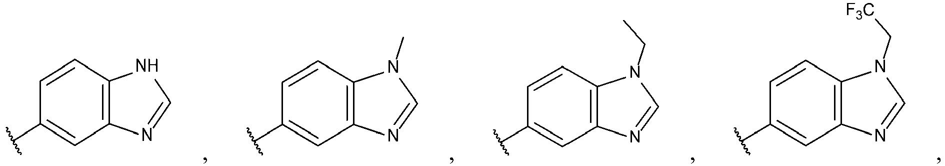

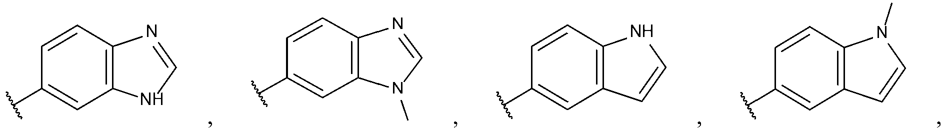

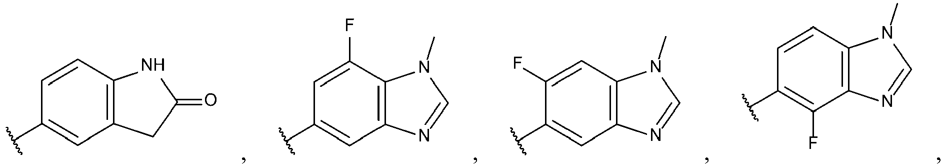

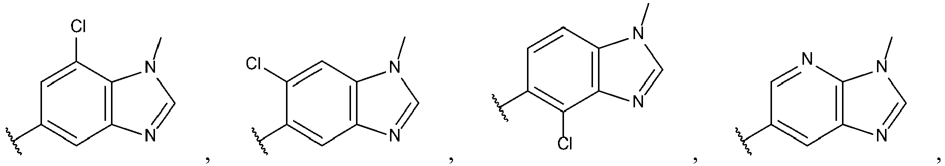

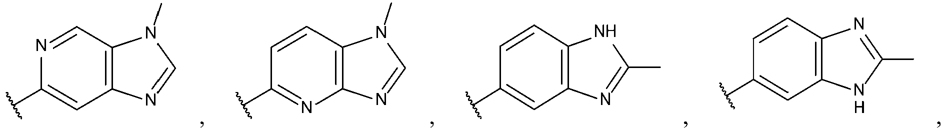

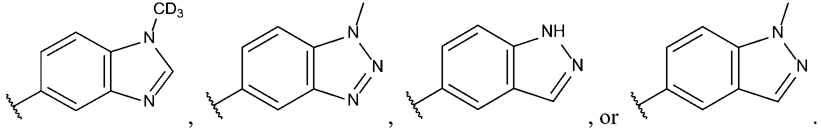

In some specific embodiments, ring C and ring D together form the following fused ring structure:

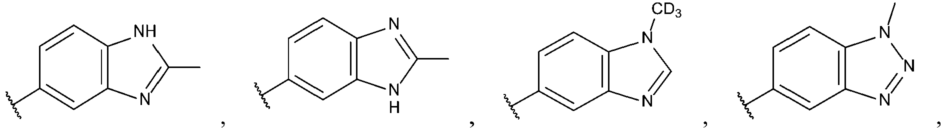

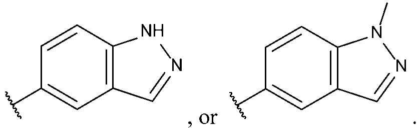

In some embodiments, when present, L is selected from C₁₋₂ hydrocarbylene optionally substituted with 1-3 R_{d} (*e.g.,* 1, 2, or 3 R_{d}) and C₁₋₂ heterohydrocarbylene optionally substituted with 1-3 Rₑ (*e.g.,* 1, 2, or 3 Rₑ), wherein the heterohydrocarbylene optionally contains 1 or 2 heteroatoms selected from N, O, and S, and each occurrence of R_{d} and Rₑ is independently selected from C₁₋₄ alkyl (*e.g.,* methyl, ethyl, propyl, isopropyl, butyl, isobutyl, or *tert*-butyl), C₁₋₄ haloalkyl (*e.g.,* trifluoromethyl or trifluoroethyl), C₁₋₄ alkoxy (*e.g.*, methoxy, ethoxy, propoxy, or butoxy), C₁₋₄ haloalkoxy, and the like.

In some specific embodiments, when present, L is selected from the group consisting of -CH₂-, -CH(CH₃)-, -CH₂CH₂-, and -NH-CH=CH-.

In some specific embodiments, when present, L is selected from the group consisting of -CH₂-, -CH(CH₃)-, -CH₂CH₂-, -NH-CH=CH-, and -N=CH₂-.

In some embodiments, the present disclosure provides a compound of Formula (II), a pharmaceutically acceptable salt thereof, a solvate thereof, a stereoisomer thereof, or an isotopic variant thereof:

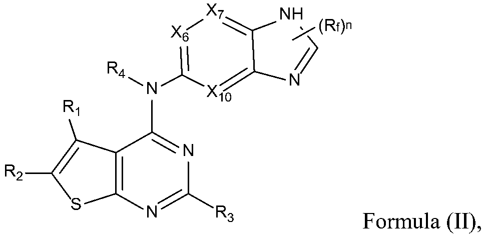

wherein R₁, R₂, R₃, R₄, X₆, X₇, X₁₀, and R_{f} are as defined above, and n is an integer of 1 to 3. The embodiments and specific embodiments described in detail above for Formula (I) are equally applicable to Formula (II).

In some embodiments, the compound of Formula (I) or Formula (II) is selected from the following compounds: 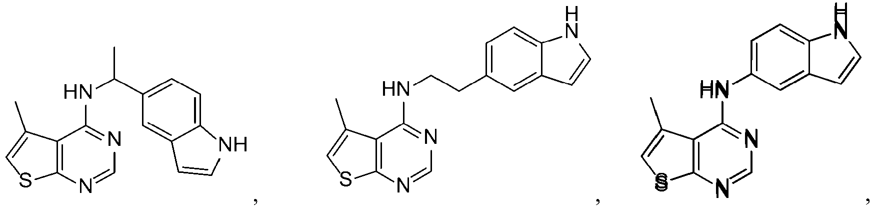 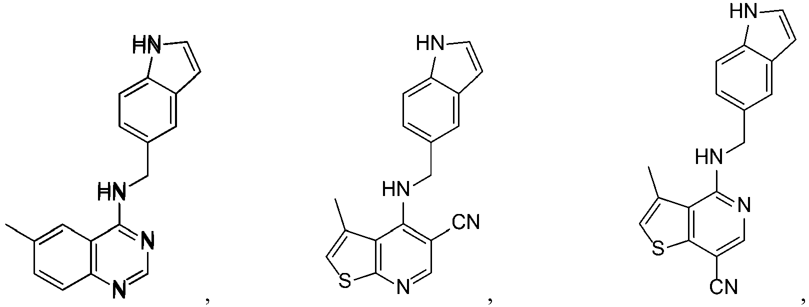 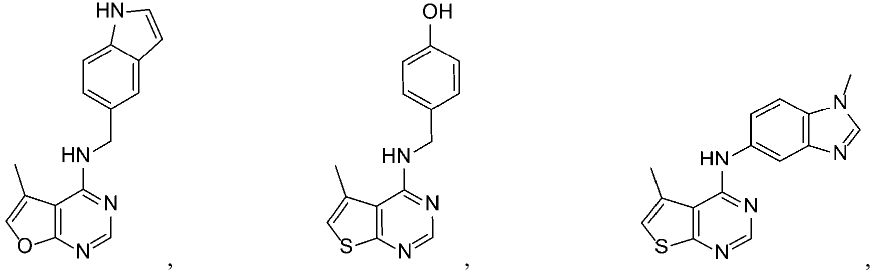 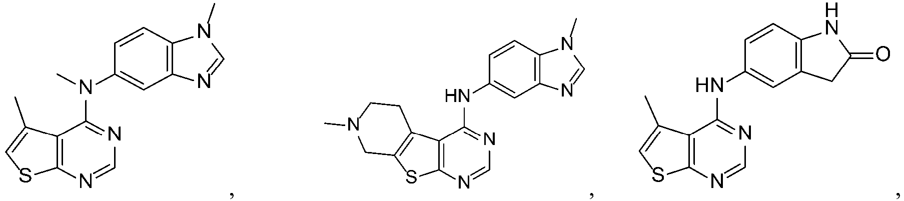 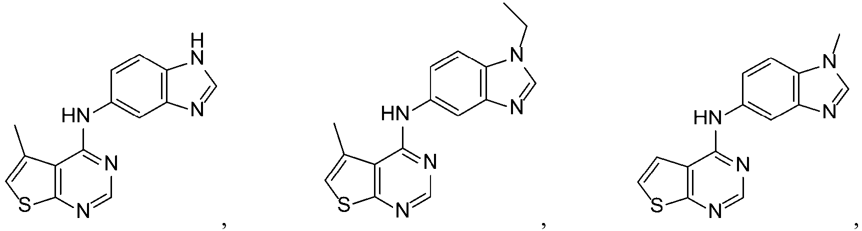 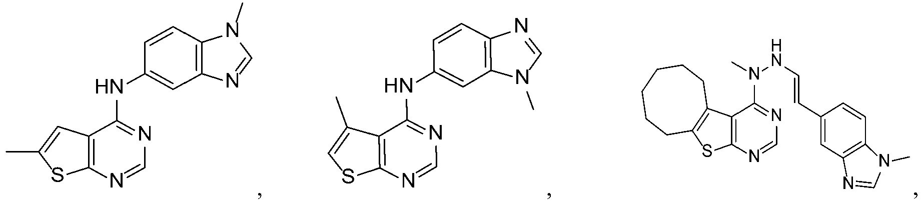 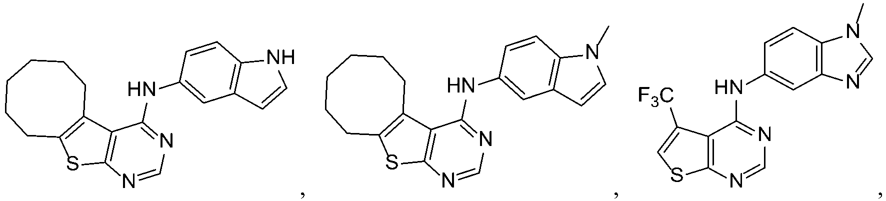 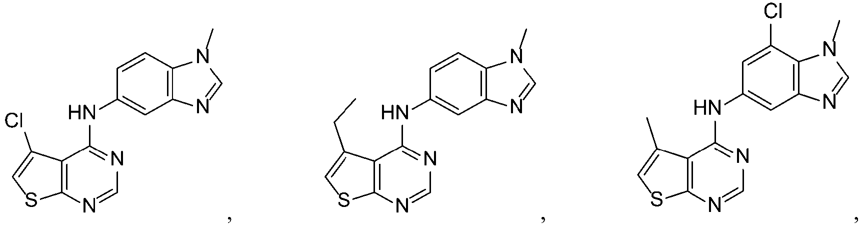 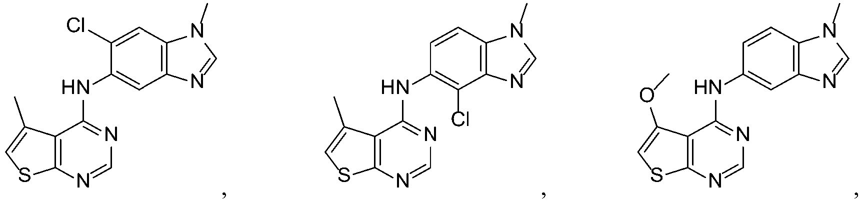 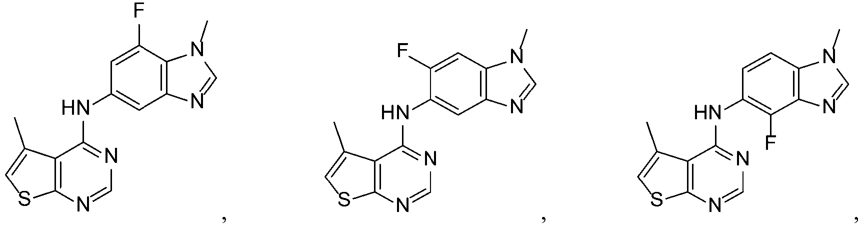 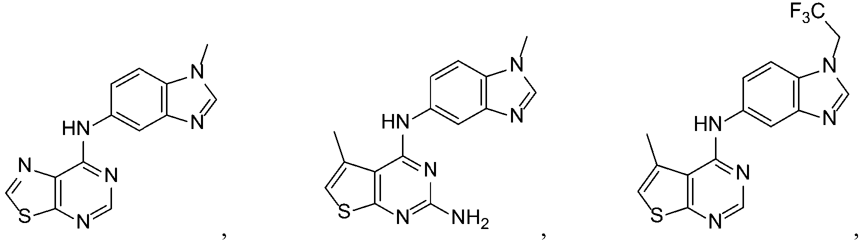 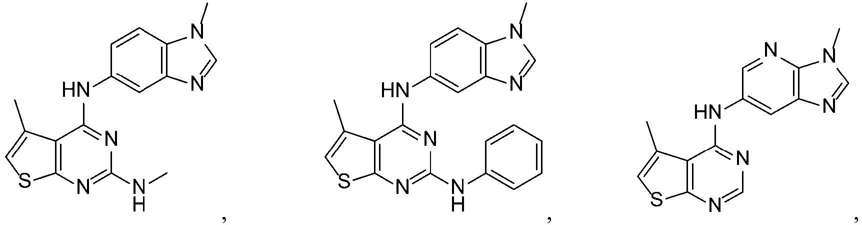 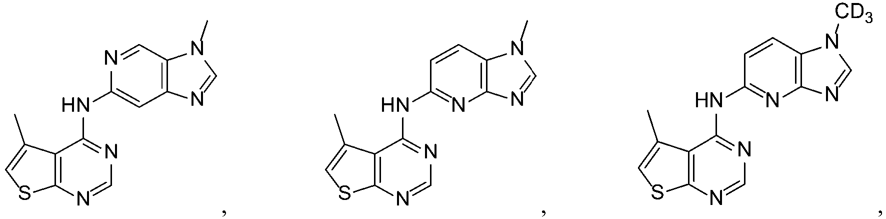 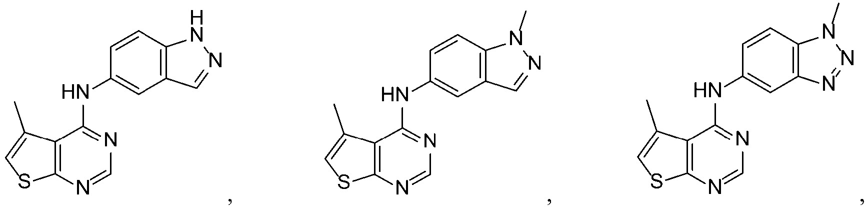 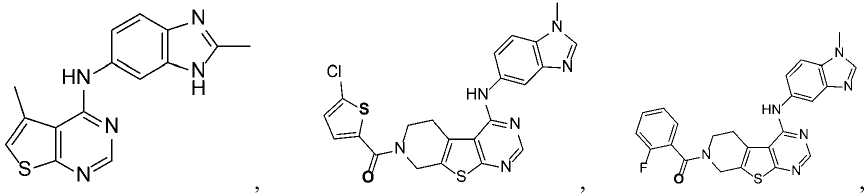 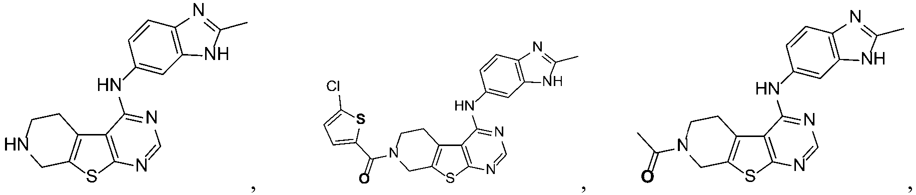 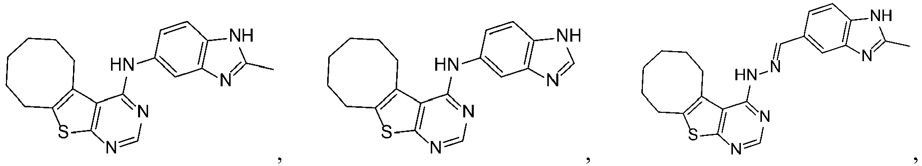 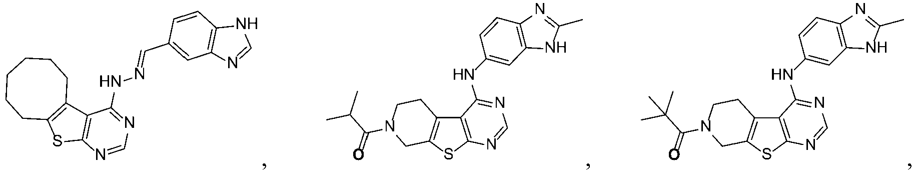 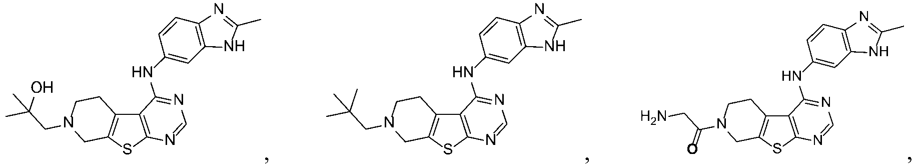 and

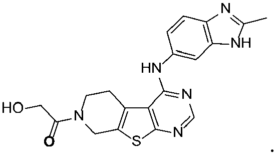

In one aspect, the present disclosure provides a preparation method for the compound of Formula (I), the stereoisomer thereof, the isotopic variant thereof, or the pharmaceutically acceptable salt or solvate of any one of the foregoing, wherein the preparation method comprises the following synthetic route: 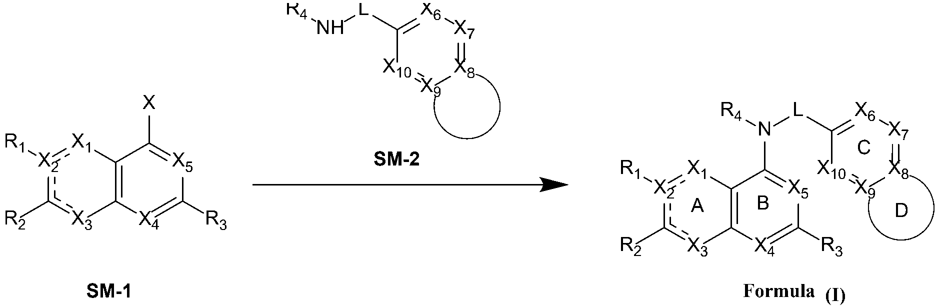 wherein X in SM-1 represents a leaving group selected from a halogen, such as F, Cl, Br, or I; an activated hydroxyl group, such as OTs, OMs, or OTf. SM-1 and SM-2 react under a suitable base such as TEA, DIPEA, pyridine or a suitable acid such as TFA or HCl. Under base-catalyzed conditions, the preferred reaction temperature is 20-120°C or 20-40°C, and under acid-catalyzed conditions, the preferred reaction temperature is 70-90°C.

In one aspect, the present disclosure provides a pharmaceutical composition comprising the compound of Formula (I), the stereoisomer thereof, the isotopic variant thereof, or the pharmaceutically acceptable salt or solvate of any one of the foregoing as described in the present disclosure, and a pharmaceutically acceptable carrier or excipient.

In one aspect, the present disclosure provides a method for inhibiting intracellular Arf1 pathway activity in a cell, comprising administering an effective amount of the compound of Formula (I), the stereoisomer thereof, the isotopic variant thereof, or the pharmaceutically acceptable salt or solvate of any one of the foregoing to the cell, such that the Arf1 pathway activity in the cell is reduced.

In some embodiments, the cell is a progenitor cell, a stem cell, a cancer stem cell, or a cancer cell.

In some embodiments, the method induces cell death at the cellular level.

In some embodiments, the method is performed *in vitro.*

In some embodiments, the method is performed *in vivo.*

In one aspect, the present disclosure provides a use of the compound of Formula (I), the stereoisomer thereof, the isotopic variant thereof, or the pharmaceutically acceptable salt or solvate of any one of the foregoing in the manufacture of an agent for inhibiting intracellular Arf1 pathway activity in a cell.

In some embodiments, the cell is a progenitor cell, a stem cell, a cancer stem cell, or a cancer cell.

In some embodiments, the agent induces cell death at the cellular level.

In some embodiments, the agent is applied to the cell *in vitro.*

In some embodiments, the agent is applied to the cell *in vivo.*

In one aspect, the present disclosure provides a method for treating or preventing a disease associated with Arf1 pathway activity in a subject, comprising administering an effective amount of the compound of Formula (I), the stereoisomer thereof, the isotopic variant thereof, or the pharmaceutically acceptable salt or solvate of any one of the foregoing as described in the present disclosure to the subject.

In some embodiments, the disease associated with Arf1 pathway activity is cancer or a tumor.

In some embodiments, the cancer or the tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

In some embodiments, the disease associated with Arf1 pathway activity is selected from the group consisting of autoimmune diseases, inflammatory diseases, inflammatory bowel disease, arthritis, autoimmune demyelinating disorders, Alzheimer's disease, amyotrophic lateral sclerosis, stroke, ischemia-reperfusion injury, multiple sclerosis, other neurodegenerative diseases, and the like.

In some embodiments, the subject is a mammal, such as a human.

In some embodiments, the method further comprises co-administering at least one immune checkpoint inhibitor, such as an anti-PD-1 antibody.

In some embodiments, the compound of Formula (I), the stereoisomer thereof, the isotopic variant thereof, or the pharmaceutically acceptable salt or solvate of any one of the foregoing as described in the present disclosure is administered simultaneously with or sequentially in any order with at least one immune checkpoint inhibitor, such as an anti-PD-1 antibody.

In one aspect, the present disclosure provides the compound of Formula (I), the stereoisomer thereof, the isotopic variant thereof, or the pharmaceutically acceptable salt or solvate of any one of the foregoing as described in the present disclosure for use in treating or preventing a disease associated with Arf1 pathway activity in a subject.

In some embodiments, the disease associated with Arf1 pathway activity is cancer or a tumor.

In some embodiments, the cancer or the tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

In some embodiments, the disease associated with Arf1 pathway activity is selected from the group consisting of autoimmune diseases, inflammatory diseases, inflammatory bowel disease, arthritis, autoimmune demyelinating disorders, Alzheimer's disease, amyotrophic lateral sclerosis, stroke, ischemia-reperfusion injury, multiple sclerosis, other neurodegenerative diseases, and the like.

In some embodiments, the subject is a mammal, such as a human.

In some embodiments, the compound of Formula (I), the stereoisomer thereof, the isotopic variant thereof, or the pharmaceutically acceptable salt or solvate of any one of the foregoing as described in the present disclosure is administered in combination with at least one immune checkpoint inhibitor, such as an anti-PD-1 antibody.

In some embodiments, the compound of Formula (I), the stereoisomer thereof, the isotopic variant thereof, or the pharmaceutically acceptable salt or solvate of any one of the foregoing as described in the present disclosure is administered simultaneously with or sequentially in any order with at least one immune checkpoint inhibitor, such as an anti-PD-1 antibody.

In one aspect, the present disclosure provides a use of the compound of Formula (I), the stereoisomer thereof, the isotopic variant thereof, or the pharmaceutically acceptable salt or solvate of any one of the foregoing as described in the present disclosure in the manufacture of a medicament for treating or preventing a disease associated with Arf1 pathway activity in a subject.

In some embodiments, the disease associated with Arf1 pathway activity is cancer or a tumor.

In some embodiments, the cancer or the tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

In some embodiments, the disease associated with Arf1 pathway activity is selected from the group consisting of autoimmune diseases, inflammatory diseases, inflammatory bowel disease, arthritis, autoimmune demyelinating disorders, Alzheimer's disease, amyotrophic lateral sclerosis, stroke, ischemia-reperfusion injury, multiple sclerosis, other neurodegenerative diseases, and the like.

In some embodiments, the subject is a mammal, such as a human.

In one aspect, the present disclosure provides a kit for use in treating or preventing a disease associated with Arf1 pathway activity in a subject, the kit comprising the compound of Formula (I), the stereoisomer thereof, the isotopic variant thereof, or the pharmaceutically acceptable salt or solvate of any one of the foregoing as described in the present disclosure, or the pharmaceutical composition as described in the present disclosure; a container; and optionally a package insert or label indicating the treatment.

In some embodiments, the disease associated with Arf1 pathway activity is cancer or a tumor.

In some embodiments, the cancer or the tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

In some embodiments, the disease associated with Arf1 pathway activity is selected from the group consisting of autoimmune diseases, inflammatory diseases, inflammatory bowel disease, arthritis, autoimmune demyelinating disorders, Alzheimer's disease, amyotrophic lateral sclerosis, stroke, ischemia-reperfusion injury, multiple sclerosis, other neurodegenerative diseases, and the like.

In some embodiments, the subject is a mammal, such as a human.

In some embodiments, the kit further comprises at least one immune checkpoint inhibitor, such as an anti-PD-1 antibody.

In one aspect, the present disclosure provides a kit for use in diagnosing a disease associated with Arf1 pathway activity in a subject, comprising at least one compound of Formula (I), the stereoisomer thereof, the isotopic variant thereof, or the pharmaceutically acceptable salt or solvate of any one of the foregoing as described in the present disclosure as a test agent.

In some embodiments, the test agent detects at least one indicative biomarker of the presence of a disease associated with Arf1 pathway activity.

In some embodiments, the test agent is capable of detecting Arf1 GTPase activity, serving as a reporter gene for detecting lipolytic activity, detecting lipid droplet formation, detecting autophagy activity, or acting as an upstream or downstream surrogate modulator of Arf1 activity or function.

In some embodiments, the disease associated with Arf1 pathway activity is cancer or a tumor.

In some embodiments, the cancer or the tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

In some embodiments, the disease associated with Arf1 pathway activity is selected from the group consisting of autoimmune diseases, inflammatory diseases, inflammatory bowel disease, arthritis, autoimmune demyelinating disorders, Alzheimer's disease, amyotrophic lateral sclerosis, stroke, ischemia-reperfusion injury, multiple sclerosis, other neurodegenerative diseases, and the like.

In some embodiments, the subject is a mammal, such as a human.

In one aspect, the present disclosure provides the compound of Formula (I), the stereoisomer thereof, the isotopic variant thereof, or the pharmaceutically acceptable salt or solvate of any one of the foregoing as described in the present disclosure for use in the manufacture of a therapeutic vaccine for blocking tumor development.

In some embodiments, the tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

In one aspect, the present disclosure provides a use of the compound of Formula (I), the stereoisomer thereof, the isotopic variant thereof, or the pharmaceutically acceptable salt or solvate of any one of the foregoing as described in the present disclosure in the manufacture of a therapeutic vaccine for blocking tumor development.

In some embodiments, the tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

In one aspect, the present disclosure provides a method for preparing a therapeutic vaccine for blocking tumor development, comprising contacting the compound of Formula (I), the stereoisomer thereof, the isotopic variant thereof, or the pharmaceutically acceptable salt or solvate of any one of the foregoing as described in the present disclosure with a cancer stem cell, thereby converting the cancer stem cell into a therapeutic vaccine.

In one aspect, the present disclosure provides a vaccination method, comprising administering the therapeutic vaccine prepared by the method as described in the present disclosure to a subject.

In some embodiments, the subject is a mammal, such as a human.

In one aspect, the present disclosure provides a method for killing cells and inducing an anti-tumor immune response, comprising inhibiting at least one Arf1 in cells, particularly the activity of the COPI/Arf1-lipolysis-β-oxidation pathway, via an Arf1 pathway inhibitor.

In some embodiments, the cell is a progenitor cell, a stem cell, a cancer stem cell, or a cancer cell.

In some embodiments, the Arf1 pathway inhibitor is selected from the group consisting of a small molecule Arf1 inhibitor, an RNAi agent targeting Arf1, an antisense agent targeting Arf1, a peptidomimetic Arf1 inhibitor, and a DNA aggregation deoxynucleotide inhibitor containing the target protein Arf1.

In some embodiments, the small molecule Arf1 inhibitor is the compound of Formula (I), the stereoisomer thereof, the isotopic variant thereof, or the pharmaceutically acceptable salt or solvate of any one of the foregoing as described in the present disclosure.

### BRIEF DESCRIPTION OF THE DRAWINGS

**FIG. 1** shows the workflow for the development and the functional characterization of the Arf1-specific inhibitors in the present disclosure.
**FIG. 2** shows the solubility of Compounds 3 and 9 of the present disclosure in DMSO and H₂O. Where A represents the solubility of Compounds 3 and 9 in DMSO; B represents the solubility of Compounds 3 and 9 in H₂O.
**FIG. 3** shows that Compounds 3 and 9 of the present disclosure can effectively inhibit the activation of Arf1. Where A is the Western blot result of Arf1 activation, specifically, DU101 refers to Compound DU101 disclosed in WO2022/028429A1, 3 refers to Compound DU101-3, 4 refers to Compound DU101-4, 6 refers to Compound DU101-6, and 8 refers to Compound DU101-8; B is the quantification of the inhibitory degree of Arf1 activation by Compound 3; C is the quantification of the inhibitory degree of Arf1 activation by Compound 9.
**FIG. 4** shows that Compounds 3 and 9 of the present disclosure induce Golgi apparatus defects in murine cells. Where A shows the effects of Compound 3 and GCA on Golgi morphology; B shows the effects of Compound 9 and GCA on Golgi morphology.
**FIG. 5** shows the promotion effects of Compounds 3 and 9 of the present disclosure on the expression level of the chemokine CCL5. Where A is the degree of CCL5 expression induced by Compound 3; B is the degree of CCL5 expression induced by Compound 9.
**FIG. 6** shows the promotion effects of Compounds 3 and 9 of the present disclosure on the activation of P65, a key protein in the NF-κB pathway.
**FIG. 7** shows the inhibitory effects of Compounds 3 and 9 of the present disclosure on the orthotopic tumor growth in Drosophila. Where A shows the degree of inhibition of Compound 3 on Drosophila orthotopic tumor growth; B shows the degree of inhibition of Compound 9 on Drosophila orthotopic tumors.
**FIG. 8** shows the inhibitory effect of Compound 9 of the present disclosure on B16-F10 melanoma in C57B/6 mice. Where A shows that Compounds 9 and Du102 significantly inhibited the size of murine melanoma tumors; B shows that Compound 9 and Du102 significantly reduced the weight of murine melanoma tumors.
**FIG. 9** shows the inhibitory effect of Compound 9 of the present disclosure on CT26 colon cancer in BALB/c mice. Where A is the size of murine colon cancer inhibited by Compound 9; B is the weight of murine colon cancer reduced significantly by Compound 9.
**FIG. 10** shows the inhibitory effects of Compounds 25, 26, and 27 of the present disclosure on CT26 colon cancer in BALB/c mice.
**FIG. 11** shows the inhibitory effects of Compounds 28, 30, 31, and 33 of the present disclosure on CT26 colon cancer in BALB/c mice.

### DETAILED DESCRIPTION OF THE PREFERRED EMBODIMENT

Certain embodiments will now be described in detail, examples of which are illustrated in the accompanying detailed description of the preferred embodiment. Although the listed embodiments will be described, it should be understood that they are not intended to limit the present disclosure to these embodiments. On the contrary, the present disclosure is intended to cover all alternatives, modifications, and equivalents, which may be included within the scope of the present disclosure as defined by the claims. Those skilled in the art will recognize many methods and materials similar or equivalent to those described herein, which can be used in the practice of the present disclosure. The present disclosure is in no way limited to the described methods and materials. If one or more incorporated documents and similar materials differ from or contradict the present disclosure, including but not limited to defined terms, term usage, described techniques, *etc.,* the present disclosure shall prevail.

It is to be understood that certain features of the present disclosure, which are described in the context of separate embodiments for clarity, may also be provided in combination in a single embodiment. Conversely, for brevity, various features of the present disclosure described in the context of a single embodiment may also be provided separately or in any suitable sub-combination.

### Definitions

Terms used but not defined herein have their ordinary meanings, and the meaning of such terms is independent in each occurrence. However, unless otherwise specified, the following definitions shall apply throughout the specification and claims.

As used herein, the terms "comprising" and "including" are intended to specify the presence of the stated features, integers, components, or steps, but they do not preclude the presence or addition of one or more other features, integers, components, steps, or groups thereof.

Definitions for specific functional groups and chemical terms are described in more detail below. For the purposes of the present disclosure, chemical elements are identified according to the Periodic Table of the Elements, CAS version, Handbook of Chemistry and Physics, 75th Edition, inside cover, and specific functional groups are generally as defined therein. Furthermore, general principles of organic chemistry, as well as specific functional moieties and reactivity, are described in Organic Chemistry, Thomas Sorrell, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; Carruthers, Some Modem Methods of Organic Synthesis, 3rd Edition, Cambridge University Press, Cambridge, 1987.

All ranges cited herein are inclusive unless expressly stated otherwise.

When a range of values is listed, it is intended to encompass each value and sub-range within that range. For example, "C₁₋₆" is intended to encompass C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆.

When any variable occurs more than once in any component or in Formula (I) or in any other formula depicting and describing the compounds of the present disclosure, its definition in each occurrence is independent of its definition in every other occurrence. Furthermore, combinations of substituents and/or variables are permissible only if such combinations result in stable compounds.

As used herein, the terms "cancer stem cell" and "CSC" are interchangeable. The CSC is of mammalian origin, and in some embodiments, the CSC is of human origin, but is not limited thereto. The definition and functional characteristics of cancer stem cells are: 1) a population of tumor cells with extensive proliferative capacity; 2) the ability to undergo asymmetric cell division, producing one or more differentiated progeny with reduced proliferative or developmental potential; 3) the ability to undergo self-renewal or self-maintenance through symmetric cell division. Other common methods for characterizing CSCs include examining morphology, cell surface markers, transcriptional profiles, and drug responses. In the research literature, CSCs are also referred to as tumor/cancer-initiating cells, cancer stem-like cells, stem-like cancer cells, highly tumorigenic cells, cancer stem cells, solid cancer stem cells, drug-surviving cells (DSCs), drug-resistant cells (DRCs), or super-malignant cells.

As used herein, the terms "cancer" and "cancerous" refer to or describe a physiological condition in mammals in which a population of cells is characterized by unregulated cell growth. As used herein, the terms "cancer cell" and "tumor cell" refer to the total cell population derived from a tumor, including tumorigenic stem cells (cancer stem cells) that constitute the majority of the tumor cell population and non-tumorigenic cells. Examples of cancers include, but are not limited to: carcinoma, lymphoma, blastoma, sarcoma, and leukemia. More specific examples of such cancers include squamous cell carcinoma, small cell lung cancer, non-small cell lung cancer, lung adenocarcinoma, squamous cell lung cancer, peritoneal cancer, hepatocellular carcinoma, gastrointestinal cancer, pancreatic cancer, glioblastoma, cervical cancer, ovarian cancer, liver cancer, bladder cancer, liver cancer, breast cancer, colon cancer, colorectal cancer, endometrial or uterine cancer, salivary gland cancer, kidney cancer, liver cancer, prostate cancer, vulvar cancer, thyroid cancer, liver cancer, and various types of head and neck cancer.

As used herein, the term "tumor" refers to any mass of tissue resulting from excessive cell growth or proliferation, whether benign (non-cancerous) or malignant (cancerous), including precancerous lesions.

As used herein, the term "metastasis" refers to the process by which cancer spreads or migrates from its site of origin to other regions of the body, resulting in similar cancerous lesions at new locations. "Metastatic" cells are those that lose adhesive contact with neighboring cells and migrate from the primary site of the disease to invade adjacent body structures through the blood or lymphatic system.

As used herein, the term "subject" refers to any animal (*e.g*., a mammal), including but not limited to humans, non-human primates, rodents, and the like, who is or will be the recipient of a particular treatment. Generally, the terms "subject" and "patient" are used interchangeably herein to refer to a human subject.

As used herein, the terms "treatment" or "alleviation" refer to: 1) curing, slowing, relieving symptoms and/or halting the progression of a diagnosed pathological condition or disorder; 2) a prophylactic or therapeutic method to prevent or slow the development of a targeted pathological condition or disorder. Thus, individuals in need of treatment include those already afflicted with the disease; those predisposed to the disease; and those requiring prevention. A subject is considered successfully "treated" according to the method of the present disclosure if the patient exhibits one or more of the following conditions: reduction or complete absence of cancer cells; decrease in tumor size; inhibition or absence of cancer cell infiltration into peripheral organs (these include the spread of cancer cells to soft tissues and bones); inhibition or absence of tumor metastasis; inhibition or absence of tumor growth; alleviation of one or more symptoms associated with the specific cancer; reduction in morbidity and mortality; and improvement in quality of life.

As used herein, the term "inhibition", when applied in the context of biological activity, refers to the downregulation of biological activity that may reduce or eliminate the target function, such as the production of proteins or the phosphorylation of molecules. In some embodiments, inhibition may refer to a reduction in target activity by approximately 20%, 30%, 40%, 50%, 60%, 70%, 80%, 90%, or 95%. When used in the context of a disorder or disease, the term may refer to the effect achieved in preventing the onset of symptoms, alleviating symptoms, or eliminating the disease, condition, or disorder.

As used herein, the term "lipolysis" refers to the breakdown of lipids and involves the hydrolysis of triglycerides into glycerol and free fatty acids. Lipolysis primarily occurs in adipose tissue to mobilize stored energy during fasting or exercise.

As used herein, the term "guanosine triphosphatase" refers to a hydrolase capable of binding and hydrolyzing guanosine triphosphate (GTP). Both GTP binding and hydrolysis occur in the highly conserved G domain common to all GTPases.

As used herein, the term "β-oxidation" refers to the catabolic process in which fatty acid molecules are broken down in the cytoplasm of prokaryotes and the mitochondria of eukaryotes to generate acetyl-CoA that enters the citric acid cycle, as well as NADH2 and FADH, which serve as coenzymes in the electron transport chain. β-Oxidation is so named because the β-carbon of the fatty acid is oxidized to a carbonyl group. β-Oxidation is primarily catalyzed by the mitochondrial trifunctional protein, a multi-enzyme complex associated with the inner mitochondrial membrane, although some fatty acids are oxidized in peroxisomes.

As used herein, the term "necrotic cell death" refers to when cells are exposed to non-physiological conditions (*e.g*., low temperature, hypoxia) or extreme events that may cause plasma membrane damage. Under physiological conditions, agents such as complement viruses and lytic viruses can induce direct damage to the plasma membrane. Necrosis is triggered by the inability of the cell to maintain homeostasis, leading to an influx of water and extracellular ions. Intracellular organelles, particularly mitochondria, and ultimately the entire cell swell and rupture (cytolysis). Due to the rupture of the plasma membrane, cellular contents, including lysosomal enzymes, are released into the extracellular space. Consequently, *in vivo,* necrotic cell death is typically accompanied by a robust inflammatory response and extensive tissue damage.

As used herein, the terms "immune condition" or "immune disorder" include, but are not limited to, pathological inflammation, inflammatory disorders, and autoimmune disorders or diseases. "Immune condition" also refers to infections, persistent infections, and proliferative conditions such as cancer, tumors, and angiogenesis, including infections, tumors, and cancers that resist eradication by the immune system. "Neoplastic diseases" include but are not limited to cancer, cancer cells, tumors, angiogenesis, as well as precancerous lesions such as dysplasia.

As used herein, the term "cytotoxic T cell" refers to a T lymphocyte (a type of white blood cell) that kills cancer cells, virus-infected cells, or damaged cells. It is known that most cytotoxic T cells express T cell receptors (TCRs) that recognize specific antigens. https://en.wikipedia.org/wiki/AntigenAn antigen is a molecule capable of stimulating an immune response, typically produced by cancer cells or viruses. Intracellular antigens bind to class I MHC molecules and are then transported to the cell surface, where they can be recognized by T cells. When the TCR encounters its specific antigen, it directly binds to the complex of the class I MHC molecule and the antigen, thereby enabling the T cell to destroy the target cell.

To interact with the class I MHC molecule, the TCR must be accompanied by the glycoprotein CD8, which binds to the anchoring portion of the class I MHC molecule. Thus, these T cells are referred to as CD8⁺ T cells.

Once cytotoxic CD8⁺ T cells (CTLs) recognize their target cells in the periphery, a highly specialized intercellular contact structure called the immunological synapse (IS) forms between the CTL and its target cell, leading to CTL activation. TCR activation triggers the engagement of src-family kinases Lck and Fyn, as well as the recruitment of the syk-family kinase ZAP-70 to the TCR, where these tyrosine kinases become activated on TCR. The activated ZAP70 subsequently phosphorylates the adaptor protein linker for activation of T cells (LAT), which orchestrates the assembly of a multiprotein signaling complex, including phospholipase c-γ1 (PLC-γ1) and SLP76 (SH2 domain-containing leukocyte protein of 76 kDa). PLCγ1 converts phosphatidylinositol-4,5-bisphosphate (PIP₂) into DAG (diacylglycerol) and IP (inositol₃-1,4,5-trisphosphate). The accumulation of DAG at the immune synapse leads to the recruitment of novel protein kinase Cs (PKCs) such as PKCθ. Subsequently, PKCθ promotes the polarization of the centrosome (microtubule organizing center, MTOC) through the mutual localization of dynein at the synapse. Dynein pulls the MTOC toward the synapse and non-muscle myosin II (NMII) toward the opposite side of the cell, where it pushes the MTOC toward the synapse. The docking of the MTOC beneath the IS ensures dynein- and calcium-dependent targeted delivery and exocytosis of granule contents directly into the synaptic cleft, leading to the lysis of target cells through the synergistic action of granzymes and perforin (de la Roche *et al.,* 2016).

As used herein, the term "deuterated" refers to the replacement of one or more hydrogen atoms on a compound or group with deuterium atoms. When a compound or group is deuterated, one, two, three, or even more hydrogen atoms on the compound or group may be replaced by deuterium atoms, until all hydrogen atoms on the compound or group are replaced by deuterium atoms, at which point it may be referred to as a "fully deuterated compound or group".

In some embodiments, the deuterium isotopic abundance at the deuterated position is greater than the natural deuterium isotopic abundance (0.015%), preferably greater than 50%, more preferably greater than 60%, greater than 70%, greater than 80%, greater than 90%, greater than 95%, greater than 96%, greater than 97%, greater than 98%, greater than 99%, greater than 99.5%, or 100%.

In some cases, for example, when "hydrogen" and "deuterium" appear as parallel optional options or when "hydrogen" is described as being replaced by "deuterium", the term "hydrogen" refers to the hydrogen isotope "¹hydrogen (¹H)", while the term "deuterium" refers to the hydrogen isotope "²hydrogen (²H)"; or it can be understood that at the position of the compound, the state where hydrogen exists with its natural isotopic abundance is replaced by a state where deuterium exists with an abundance greater than the natural deuterium isotopic abundance (*e.g*., deuterium abundance greater than 50%, greater than 60%, greater than 70%, greater than 80%, greater than 90%, greater than 95%, greater than 96%, greater than 97%, greater than 98%, greater than 99%, greater than 99.5%, or 100%).

As used herein, the term "hydrocarbyl" refers to a chemical group containing hydrogen and carbon. The hydrocarbyl group may be substituted or unsubstituted. The hydrocarbyl group may be unsaturated, saturated, branched, unbranched, cyclic, polycyclic, or heterocyclic, and includes alkyl, alkenyl, and alkynyl groups, and the like. The hydrocarbyl group may be fully saturated, monounsaturated, or polyunsaturated, and may include divalent and polyvalent groups, such as when it is a divalent group, it may be referred to as a "hydrocarbylene". The hydrocarbyl group has the specified number of carbon atoms (*i.e.,* C₁-C₁₀ means 1 to 10 carbon atoms, including 1, 2, 3, 4, 5, 6, 7, 8, 9, and 10 carbon atoms). In some embodiments, the hydrocarbon group contains one or more, such as 1, 2, 3, or 4 unsaturated carbon-carbon double bonds (-C=C-), carbon-carbon triple bonds (-C≡C-) groups, and/or any combination thereof. In some embodiments, the hydrocarbyl group may be deuterated, also referred to as a "deuterated hydrocarbyl group", meaning that one, two, three, or even more hydrogen atoms on the hydrocarbyl group may be replaced by deuterium atoms, until all hydrogen atoms on the hydrocarbyl group are replaced by deuterium atoms, at which point it may be termed a "fully deuterated hydrocarbyl group".

As used herein, the term "heterohydrocarbyl" refers to a hydrocarbyl group containing heteroatoms such as N, O, and S. The heterohydrocarbyl group may be substituted or unsubstituted. The heterohydrocarbyl group may be unsaturated, saturated, branched, unbranched, cyclic, polycyclic, or heterocyclic, and includes heteroalkyl, heteroalkenyl, heteroalkynyl, and the like. The heterohydrocarbyl group may be fully saturated, monounsaturated, or polyunsaturated, and may include divalent and polyvalent groups, such as when it is a divalent group, it may be referred to as a "heterohydrocarbylene group". The heterohydrocarbyl group has a specified number of carbon atoms (*i.e.,* C₁-C₁₀ means 1 to 10 carbon atoms, including 1, 2, 3, 4, 5, 6, 7, 8, 9, and 10 carbon atoms), or alternatively, the heterohydrocarbyl group has a specified number of backbone atoms, *i.e.,* the heterohydrocarbyl backbone has 1, 2, 3, 4, 5, 6, 7, 8, 9, and 10 atoms. In some embodiments, the heteroatoms present in the heterohydrocarbyl group may form the backbone of the heterohydrocarbyl group together with carbon atoms, such as, but not limited to, -C-N-C-, -C-O-C-, -C-O-O-C-, -C-S-C-, -C-S-S-C-, and other group structures or any combination thereof. In some embodiments, the heteroatoms contained in the heterohydrocarbyl group may be substituents attached to carbon atoms, such as, but not limited to, -C≡N, -C=N-, -C-N=, -C=O, -C-OH, -C=S, -C-SH, and other substituent structures. In some of these embodiments, the heteroatoms contained in the heterohydrocarbyl group may be any combination of the aforementioned group structures. In some embodiments, the heterohydrocarbyl group contains one or more, for example, 1, 2, 3, or 4 unsaturated carbon-carbon double bonds (-C=C-), carbon-carbon triple bonds (-C=C-), , -NH-, -O-, -C(O)-, -S-, -C(S)-, and/or any combination thereof. In some embodiments, the heterohydrocarbyl group may be deuterated, also referred to as a "deuterated heterohydrocarbyl group", meaning that one, two, three, or even more hydrogen atoms on the heterohydrocarbyl may be replaced by deuterium atoms, until all hydrogen atoms on the heterohydrocarbyl group are replaced by deuterium atoms, at which point it may be termed a "fully deuterated heterohydrocarbyl group".

As used herein, the term "alkyl" refers to a straight or branched saturated hydrocarbyl group. The term "Cᵢ₋ⱼ alkyl" refers to an alkyl group having i to j carbon atoms. Unless otherwise specified, the alkyl group may contain 1 to 10 carbon atoms. In certain embodiments, the alkyl group contains 1 to 6 carbon atoms, such as 1 to 5 carbon atoms, 1 to 4 carbon atoms, 1 to 3 carbon atoms, or 1 to 2 carbon atoms. Non-limiting examples of alkyl groups include methyl, ethyl, *n*-propyl, isopropyl, *n-*butyl, *sec*-butyl, isobutyl, *tert*-butyl, neopentyl, and the like. As used herein, the term "alkylene" refers to a divalent substituent, which is a monovalent alkyl group with one hydrogen atom substituted by a valence.

In some embodiments, the alkyl group may be deuterated, also referred to as a "deuterated alkyl group".

As used herein, the term "deuterated alkyl group" refers to a substituent obtained by replacing one or more hydrogen atoms on an alkyl group with deuterium atoms. When the alkyl group is deuterated, one, two, three, or even more hydrogen atoms on the alkyl group may be replaced by deuterium atoms, until all hydrogen atoms on the alkyl group are replaced by deuterium, at which point it may be referred to as a "fully deuterated alkyl group". In some embodiments, non-limiting examples of deuterated alkyl groups include deuterated methyl, such as monodeuterated methyl, dideuterated methyl, trideuterated methyl (fully deuterated methyl), monodeuterated ethyl, dideuterated ethyl, trideuterated ethyl, tetradeuterated ethyl, pentadeuterated ethyl (fully deuterated ethyl), and the like.

Those skilled in the art will appreciate that, in some embodiments, other types of groups mentioned herein, as long as the group contains hydrogen atoms, can be deuterated. When the group is deuterated, one, two, three, or even more hydrogen atoms on it can be replaced by deuterium atoms until all hydrogen atoms on the group are replaced by deuterium atoms, at which point it may be referred to as a "fully deuterated group".

As used herein, the term "alkenyl" refers to a straight or branched hydrocarbon group having at least one carbon-carbon double bond, and includes groups with *"cis"* and *"trans"* orientations, or alternatively, "*E*" and "*Z*" orientations. Unless otherwise specified, the alkenyl group may contain 2 to 10 carbon atoms. In certain embodiments, the alkenyl group may contain 2 to 6 carbon atoms, such as 2 to 5 carbon atoms, 2 to 4 carbon atoms, or 2 to 3 carbon atoms. In certain embodiments, the alkenyl group contains 2 carbon atoms. Non-limiting examples of alkenyl groups include ethenyl (vinyl), propenyl, butenyl, pentenyl, 1-methyl-2-buten-1-yl, 5-hexenyl, and the like.

As used herein, the term "alkynyl" refers to a straight or branched hydrocarbon group having at least one carbon-carbon triple bond. Unless otherwise specified, the alkynyl group may contain 2 to 10 carbon atoms. In certain embodiments, the alkynyl group contains 2 to 8 carbon atoms, 2 to 6 carbon atoms, 2 to 4 carbon atoms, or 2 to 3 carbon atoms. In certain embodiments, the alkynyl group contains 2 carbon atoms. Non-limiting examples of alkynyl groups include ethynyl, 1-propynyl, 2-propynyl, and the like.

As used herein, the term "alkoxy" refers to a group -O-alkyl, wherein the alkyl has the meaning as defined herein.

As used herein, the term "cycloalkyl" refers to a non-aromatic, saturated monocyclic or polycyclic ring system wherein all ring-forming atoms are carbon atoms. Unless otherwise specified, the cycloalkyl group may contain 3 to 10 ring-forming carbon atoms (*i.e.,* C₃₋₁₀ cycloalkyl). In certain embodiments, the cycloalkyl group may contain 3 to 9, 3 to 8, 3 to 7, 3 to 6, 4 to 10, 4 to 9, 4 to 8, 4 to 7, 4 to 6, 4 to 5, 5 to 10, 5 to 9, 5 to 8, 5 to 7, or 5 to 6 ring-forming carbon atoms, and the like. In particular, the cycloalkyl group may be monocyclic or bicyclic. Optionally, the bicyclic cycloalkyl group may include fused, spiro, and bridged cycloalkyl structures.

In another aspect, also included are cycloalkyl rings wherein 1, 2, or 3 heteroatoms replace the ring-forming carbon atoms. Such groups are referred to as "heterocyclyl" or "heterocycle", which means a cycloalkyl group as defined above but containing at least one heteroatom selected from N, O, and S as a ring-forming atom. Unless otherwise specified, the heterocyclyl group may contain 3 to 10 ring-forming atoms (*i.e.,* 3- to 10-membered heterocyclyl). In certain embodiments, the heterocyclyl group may contain 3 to 9, 3 to 8, 3 to 7, 3 to 6, 4 to 10, 4 to 9, 4 to 8, 4 to 7, 4 to 6, 4 to 5, 5 to 10, 5 to 9, 5 to 8, 5 to 7, or 5 to 6 ring-forming atoms, and the like. In particular, the heterocyclyl group may be monocyclic or bicyclic. Optionally, the bicyclic heterocyclyl group may include fused, spiro, and bridged heterocyclyl structures. Non-limiting examples of heterocyclyl groups include oxiranyl, pyrrolidinyl, piperidinyl, tetrahydropyranyl, piperazinyl, pyrrolidinyl, and morpholinyl. Heterocyclyl groups may also be described by the number of carbon atoms. For example, C₃₋₆ heterocyclyl refers to a heterocyclyl group containing three to six ring-forming carbon atoms, which may further contain at least one heteroatom, such as 1, 2, or 3 heteroatoms as ring-forming atoms. In certain embodiments, the heterocyclyl group or heterocycle contains 1 or 2 heteroatoms as ring-forming atoms. In some embodiments, the heterocyclyl group may be monocyclic or bicyclic, such as fused bicyclic and spiro bicyclic. In the context of the present disclosure, the terms "heterocyclyl" and "heterocycle" may be used interchangeably.

As used herein, the term "aryl" or "aromatic ring" refers to a monocyclic, bicyclic, or polycyclic carbocyclic system having at least one aromatic ring. Unless otherwise specified, the aryl group may be 6- to 10-membered. In certain embodiments, the aryl group may contain 6 ring-forming carbon atoms. All atoms within the carbocyclic aryl group are carbon atoms. Non-limiting examples of aryl groups include phenyl, naphthyl, 1,2-dihydronaphthyl, 1,2,3,4-tetrahydronaphthyl, fluorenyl, indanyl, indenyl, and the like. In the context of the present disclosure, the terms "aryl" and "aromatic ring" may be used interchangeably.

As used herein, the term "heteroaryl" or "heteroaromatic ring" refers to a monocyclic system, or a fused or bridged bicyclic system, wherein the ring system contains one, two, three, or four heteroatoms independently selected from the group consisting of nitrogen, oxygen, and sulfur; and at least one ring is an aromatic ring. Unless otherwise specified, the heteroaryl group may be 5- to 10-membered. In certain embodiments, the heteroaryl group may be 5-membered or 6-membered. In certain embodiments, the heteroaryl group may contain one, two, or three heteroatoms. In certain embodiments, the heteroaryl group may contain one or two heteroatoms. Non-limiting examples of heteroaryl groups include benzimidazolyl, benzofuranyl, benzothiazolyl, benzothiophenyl, benzoxazolyl, furanyl, imidazolyl, indolyl, isoindazolyl, isoquinolinyl, isothiazolyl, isothiazolyl, isoxazolyl, oxadiazolyl, oxazolyl, purinyl, pyrrolyl, pyridinyl, pyrazinyl, pyrimidinyl, quinolinyl, quinolinyl, thiadiazolyl, thiazolyl, thiophenyl, triazolyl, tetrazolyl, dihydroindolyl, tetrahydroquinolinyl, tetrahydroisoquinolinyl, and the like. Heteroaryl groups include at least one ring having at least one heteroatom as described above and at least one aromatic ring. For example, the ring having at least one heteroatom may be fused to one, two, or three carbocyclic rings, such as an aryl ring, a cyclohexane ring, a cyclohexene ring, a cyclopentane ring, a cyclopentene ring, or another monocyclic heterocycle. Non-limiting examples of fused heteroaryl groups include 1,2,3,5,8,8a-hexahydroindolizine, 2,3-dihydrobenzofuran, 2,3-dihydroindole, 2,3-dihydrobenzothiophene, and the like. In the context of the present disclosure, the terms "heteroaryl" and "heteroaromatic ring" may be used interchangeably.

As used herein, the term "oxo" refers to a divalent oxygen atom, and the structure of oxo may be represented as =O.

As used herein, the term "halo" or "halogen" refers to fluoride, chloride, bromide, and iodide. In certain embodiments, non-limiting examples of halo include fluoride, chloride, and bromide, more particularly fluoride and chloride.

As used herein, the term "hydroxy" refers to the substitution of one or more hydrogen atoms on a chemical group with a hydroxyl substituent (-OH). For example, the term "hydroxyalkyl" refers to a structure formed by replacing one or more hydrogen atoms on an alkyl group with a hydroxyl group, such as hydroxymethyl (-CH₂OH), hydroxyethyl (-CH₂CH₂OH), and the like.

As used herein, the term "amino" refers to the substitution of one or more hydrogen atoms on a chemical group with an amino substituent (-NH₂). For example, the term "aminoalkyl" refers to a structure formed by replacing one or more hydrogen atoms on an alkyl group with an amino group, such as methylamino (-CH₂NH₂), ethylamino (-CH₂CH₂NH₂), and the like.

As used herein, the term "heteroatom" refers to nitrogen (N), oxygen (O), and sulfur (S), and may include any oxidized form of nitrogen and sulfur, as well as any quaternized form of basic nitrogen, unless otherwise specified.

As used herein, the term "substituted", when referring to a chemical group, means that the chemical group has one or more hydrogen atoms removed and replaced by substituents. As used herein, the term "substituent" has its ordinary meaning as known in the art and refers to a chemical moiety covalently attached to a parent group or, if appropriate, fused to the parent group. It is to be understood that the substitution of a given atom is limited by its valence. It is to be understood that substituents may be further substituted.

When a moiety in Formula (I) or any of its embodiments is indicated to be "optionally" substituted, this means that Formula (I) or its embodiments encompass both compounds wherein the moiety is substituted with the indicated substituent and compounds wherein the moiety does not contain the indicated substituent (*i.e.,* wherein the moiety is unsubstituted).

The compounds provided herein are described with reference to general formulas and specific compounds. In addition, the compounds of the present disclosure may exist in various different forms or derivatives, all of which are within the scope of the present disclosure. These include, for example, pharmaceutically acceptable salts, tautomers, stereoisomers, racemic mixtures, positional isomers, prodrugs, solvated forms, different crystal forms or polymorphs, and active metabolites.

As used herein, unless otherwise specified, the term "pharmaceutically acceptable salt" includes salts that retain the biological effectiveness of the free acid/base form of the specific compound and are not biologically or otherwise undesirable. Pharmaceutically acceptable salts may include those formed with inorganic bases or acids and organic bases or acids. In the case where the compounds of the present disclosure contain one or more acidic or basic groups, the present disclosure also encompasses their corresponding pharmaceutically acceptable salts. Thus, compounds of the present disclosure containing acidic groups (*e.g*., carboxyl groups) may exist in salt form and can be used according to the present disclosure, for example, as alkali metal salts, alkaline earth metal salts, aluminum salts, or ammonium salts. Further non-limiting examples of such salts include lithium salts, sodium salts, potassium salts, calcium salts, magnesium salts, barium salts, or salts with ammonia or organic amines (*e.g*., ethylamine, ethanolamine, diethanolamine, triethanolamine, piperidine, *N*-methylglutamine, or amino acids). For example, these salts are readily obtainable by reacting compounds with acidic groups with suitable bases (*e.g*., lithium hydroxide, sodium hydroxide, sodium propoxide, potassium hydroxide, potassium ethoxide, magnesium hydroxide, calcium hydroxide, or barium hydroxide). Other basic salts of the compounds of the present disclosure include, but are not limited to, copper(I), copper(II), iron(II), iron(III), manganese(II), and zinc salts. The compounds of the present disclosure contain one or more basic groups, such as protonatable groups, and may exist in the form of salts. They can be used in the form of their addition salts with inorganic or organic acids according to the present disclosure. Examples of suitable acids include hydrochloric acid, hydrobromic acid, hydroiodic acid, phosphoric acid, sulfuric acid, nitric acid, methanesulfonic acid, *p*-toluenesulfonic acid, naphthalenedisulfonic acid, sulfoacetic acid, trifluoroacetic acid, oxalic acid, acetic acid, tartaric acid, lactic acid, salicylic acid, benzoic acid, carbonic acid, formic acid, propionic acid, pivalic acid, diethylacetic acid, malonic acid, succinic acid, pimelic acid, fumaric acid, malonic acid, maleic acid, malic acid, pamoic acid, mandelic acid, sulfamic acid, phenylpropionic acid, gluconic acid, ascorbic acid, isonicotinic acid, citric acid, adipic acid, taurocholic acid, glutaric acid, stearic acid, glutamic acid or aspartic acid, as well as other acids known to those skilled in the art. The salts formed are particularly hydrochlorides, chlorides, hydrobromides, bromides, iodides, sulfates, phosphates, methanesulfonates (mesylates), tosylates, carbonates, bicarbonates, formates, acetates, sulfoacetates, triflates, oxalates, malonates, maleates, succinates, tartrates, malates, pamoates, mandelates, fumarates, lactates, citrates, glutarates, stearates, aspartates, and glutamates. Furthermore, the stoichiometry of the salts formed from the compounds of the present disclosure may be an integer or non-integer multiple of 1.

The compounds of the present disclosure containing basic nitrogen-containing groups can be quaternized using reagents such as C₁₋₄ haloalkanes, for example, methyl, ethyl, isopropyl, and *tert-*butyl chlorides, bromides, and iodides; di-C₁₋₄ alkyl sulfates, such as dimethyl, diethyl, and dipentyl sulfates; C₁₀₋₁₈ alkyl halides, such as decyl, dodecyl, lauryl, myristyl, and stearyl chlorides, bromides, and iodides; and aryl-C₁₋₄ alkyl halides, such as benzyl chloride and phenethyl bromide.

If the compounds of the present disclosure contain both acidic and basic groups in the molecule, in addition to the salt forms mentioned above, the present disclosure also includes inner salts or betaines (zwitterions). The corresponding salts can be obtained by conventional methods known to those skilled in the art, such as by contacting them with organic or inorganic acids or bases in a solvent or dispersant, or through anion exchange or cation exchange with other salts. The present disclosure also encompasses all salts of the compounds of the present disclosure, which, due to low physiological compatibility, are not directly suitable for pharmaceuticals but can be used, for example, as intermediates in chemical reactions or for the preparation of pharmaceutically acceptable salts. For a review of more suitable salts, refer to Stahl and Wermuth, Handbook of Pharmaceutical Salts: Properties, Selection, and Use (Wiley-VCH, 2002).

The compound of Formula (I) and the pharmaceutically acceptable salt thereof may exist in both unsolvated and solvated forms. As used herein, the term "solvate" refers to a molecular complex comprising the compound of Formula (I), the stereoisomer thereof, or the pharmaceutically acceptable salt thereof, and one or more pharmaceutically acceptable solvent molecules. For example, the term "hydrate" is used when the solvent is water.

The compound of Formula (I) may have one or more chiral (asymmetric) centers. The present disclosure encompasses all stereoisomeric forms of the compound of Formula (I). The asymmetric centers present in the compound of Formula (I) may independently have either the (*R*) or (*S*) configuration. When the bonds to chiral carbons are depicted as straight lines in the structural formulas of the present disclosure, or when a compound name is described without the (*R*) or (*S*) chiral designation for a chiral carbon, it is to be understood that both the (*R*) and (*S*) configurations of each such chiral carbon, and hence each enantiomer or diastereomer and mixtures thereof, are encompassed by such formula or name. The production of specific stereoisomers or mixtures thereof may be identified in examples where such stereoisomers or mixtures are obtained, but this in no way limits the inclusion of all stereoisomers and their mixtures within the scope of the present disclosure. When the bonds of a chiral carbon are depicted as solid or dashed wedges in the structural formulas of the present disclosure, or when the compound name is described with the chiral designation (*R*) or (*S*) at a chiral carbon, it shall be understood that the compound represented by such structural formula or name possesses a defined stereochemical configuration at that chiral carbon position, thereby distinguishing it from other stereoisomers or enantiomers or diastereomers or mixtures thereof.

The present disclosure encompasses all possible enantiomers and diastereomers, as well as mixtures of two or more stereoisomers, such as mixtures of enantiomers and/or diastereomers in any ratio. Accordingly, the enantiomers are the enantiomerically pure forms (as levo and dextro enantiomers), the racemate form, and mixtures of the two enantiomers in all proportions of the subject matter of the present disclosure. In the case of *cis*/*trans* isomers, the present disclosure includes the *cis* form and the *trans* form as well as mixtures of these forms in all proportions. If desired, individual stereoisomers may be prepared by separating the mixtures using conventional methods (*e.g*., through chromatography or crystallization, by using stereochemically homogeneous starting materials for synthesis, or via stereoselective synthesis). Optionally, derivatization may be performed prior to the separation of stereoisomers. The separation of the stereoisomeric mixture may be carried out at an intermediate step during the synthesis of the compound of Formula (I), or may be performed on the final racemic product. The absolute stereochemistry can be determined by X-ray crystallography of the crystalline product or crystalline intermediate, if necessary, by derivatizing these crystalline products or intermediates with reagents containing stereocenters of known configuration. Alternatively, the absolute stereochemistry can be determined by vibrational circular dichroism (VCD) spectroscopy.

Unless otherwise indicated, the structures described herein are also intended to include compounds that differ only in the presence of one or more isotopically enriched atoms, *i.e.,* where one or more atoms are replaced by atoms having the same atomic number but an atomic mass or mass number different from the atomic mass or mass number predominantly found in nature. Such compounds are referred to as "isotopic variants". The present disclosure is intended to encompass all pharmaceutically acceptable isotopic variants of the compound of Formula (I). Examples of isotopes suitable for inclusion in the compounds of the present disclosure include, but are not limited to, isotopes of hydrogen, such as ²H (*i.e.,* D, deuterium) and ³H (*i.e.,* tritium); carbon, such as ¹¹C, ¹³C, and ¹⁴C; chlorine, such as ³⁶Cl; fluorine, such as ¹⁸F; iodine, such as ¹²³I and ¹²⁵I; nitrogen, such as ¹³N and ¹⁵N; oxygen, such as ¹⁵O, ¹⁷O, and ¹⁸O; phosphorus, such as ³²P; and sulfur, such as ³⁵S. Certain isotopic variants of the compound of Formula (I), such as those incorporating radioactive isotopes, may be useful in drug and/or substrate tissue distribution studies. In particular, compounds having the depicted structures differing only in the replacement of heavier isotopes (*e.g*., replacement of hydrogen with deuterium (²H or D)) may provide certain therapeutic advantages, such as due to greater metabolic stability, increased *in vivo* half-life, or reduced dosage requirements, and thus may be utilized in some specific circumstances. The isotopic variants of the compounds of Formula (I) can generally be prepared by conventional techniques known to those skilled in the art or by methods analogous to those described in the accompanying examples, using appropriately isotopically labeled reagents in place of the non-labeled reagents previously employed.

When describing the structure of a compound herein, if the hydrogen atoms in the depicted structural formula are not indicated as D (*i.e.,* ²H), it should generally be understood that the hydrogen at this position exists in the form of the hydrogen isotope "¹hydrogen (¹H)" or in the form of natural isotopic abundance as found in the natural state. When the depicted structure indicates that a hydrogen atom is D (*i.e.,* ²H, deuterium), it should be understood that the hydrogen at this position exists in the form of the hydrogen isotope "²H (²H, D, deuterium)" or with a deuterium isotopic abundance greater than the natural level (*e.g*., deuterium abundance greater than 50%, greater than 60%, greater than 70%, greater than 80%, greater than 90%, greater than 95%, greater than 96%, greater than 97%, greater than 98%, greater than 99%, greater than 99.5%, or 100%).

The pharmaceutically acceptable solvates according to the present disclosure may include those in which the crystallization solvent can be isotopically substituted, such as D₂O, *d*₆-acetone, *d*₆-DMSO.

One way to implement the present disclosure is to administer the compound of Formula (I) in the form of a prodrug. Thus, certain derivatives of the compound of Formula (I) may possess little or no pharmacological activity themselves but are converted into the compound of Formula (I) with the desired activity when administered into or onto the body, for example, by hydrolytic cleavage, particularly hydrolysis facilitated by esterases or peptidases. Such derivatives are referred to as "prodrugs". Further information on the use of prodrugs can be found in, for example, T. Higuchi and W. Stella, "Pro-drugs as Novel Delivery Systems", Vol. 14, ACS Symposium Series, and E. B. Roche (Ed.), "Bioreversible Carriers in Drug Design", Pergamon Press, 1987, American Pharmaceutical Association. See also Nature Reviews/Drug Discovery, 2008, 7, 355, and Current Opinion in Drug Discovery and Development, 2007, 10, 550.

The prodrugs according to the present disclosure can be prepared, for example, by replacing appropriate functional groups present in the compound of Formula (I) with certain moieties known to those skilled in the art, such as the "promoieties" described in H. Bundgaard, "Design of Prodrugs", Elsevier, 1985, and Y. M. Choi-Sledeski and C. G. Wermuth, "Designing Prodrugs and Bioprecursors", Practice of Medicinal Chemistry, 4th Edition, Chapter 28, 657-696, Elsevier, 2015. Thus, the prodrugs according to the present disclosure may include, but are not limited to, (a) ester or amide derivatives of carboxylic acids in the compound of Formula (I), if present; (b) amide, imine, carbamate, or amine derivatives of amino groups in the compound of Formula (I); (c) oxime or imine derivatives of carbonyl groups in the compound of Formula (I), if present; or (d) methyl, primary alcohol, or aldehyde groups in the compound of Formula (I) that can be metabolically oxidized to carboxylic acids, if present.

The mention of the compound of Formula (I) includes the compound itself and its prodrug. The present disclosure includes such compounds of Formula (I), as well as pharmaceutically acceptable salts of such compounds and pharmaceutically acceptable solvates of the compounds and salts.

### Use and Administration

The compounds of the present disclosure (the compound of Formula (I), the stereoisomer thereof, or the isotopic variant thereof) - or the pharmaceutically acceptable salt thereof, or the solvate thereof, including mixtures thereof in all proportions - may be used as medicaments. It has been discovered that they exhibit pharmacological activity in inhibiting the COPI/Arf1-lipolysis-β-oxidation pathway. Through this activity, the compounds of the present disclosure can kill progenitor cells, stem cells, cancer stem cells, or cancer cells and induce an anti-tumor immune response, and can be used to treat conditions or diseases associated with Arf1 pathway activity; to treat refractory, recurrent, or metastatic cancers; and to selectively kill cancer cells through specific administration regimens.

Therefore, the compounds of the present disclosure, as Arf1 inhibitors, are particularly suitable for treating diseases and conditions related to the Arf1 pathway, especially the COPI/Arf1-lipolysis-β-oxidation pathway, such as cancers, including but not limited to the following: breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

The compounds of the present disclosure may be administered in an amount effective to treat the diseases or conditions described herein. The compounds of the present disclosure may be administered as the compounds themselves or, alternatively, as pharmaceutically acceptable salts. For the purposes of administration and dosing, the compounds themselves of the present disclosure (the compound of Formula (I), the stereoisomer thereof, or the isotopic variant thereof) - or the pharmaceutically acceptable salt thereof, or the solvate thereof, will be collectively referred to as the compounds of the present disclosure.

The compounds of the present disclosure are administered by any suitable route in the form of pharmaceutical compositions adapted to such route and at doses effective for the intended therapy. The compounds of the present disclosure may be administered orally, rectally, vaginally, parenterally, or topically.

As used herein, the term "administration" refers to the absorption, ingestion, injection, inhalation, implantation, or other introduction of the compounds of the present disclosure or pharmaceutical compositions thereof. The term "treatment" refers to reversing, alleviating, delaying the onset of, or inhibiting the progression of a "pathological condition" (*e.g*., a disease, disorder, or condition, or one or more signs or symptoms thereof) as described herein. In certain embodiments, treatment may be administered after one or more signs or symptoms of the disease or condition have developed or have been observed. In other embodiments, the treatment may be administered in the absence of signs or symptoms of the disease or condition. For example, susceptible individuals may be treated prior to the onset of symptoms (*e.g*., based on symptom history and/or genetic or other susceptibility factors). Treatment may also be continued after symptoms subside, such as to delay or prevent recurrence. As used herein, the terms "disease", "disorder", "condition", and "pathological condition" are used interchangeably.

A person skilled in the art can determine the dosage level for administration through routine experimentation. The dosage regimen of the compounds of the present disclosure and/or compositions containing said compounds is based on various factors, including the type, age, weight, sex, and medical condition of the patient; the severity of the condition; the route of administration; and the activity of the specific compound used. Therefore, the dosage regimen may vary widely. For example, the dosage level of the compounds of the present disclosure may range from about 0.001 to about 100 mg/kg (*i.e.,* mg/kg body weight) per day. In certain embodiments, the total daily dosage of the compound of the present disclosure administered in a single or divided dose may be about 0.001 to about 10 mg/kg. It is not uncommon for the administration of the compound of the present disclosure to be repeated multiple times within a day.

In some embodiments, the compounds of the present disclosure may be used in combination with one or more other therapeutic agents. In some embodiments, non-limiting examples of the other therapeutic agents include immune checkpoint inhibitors, such as anti-PD-1 antibodies. These therapeutic agents may be administered before, after, or simultaneously with the compound of the present disclosure.

As used herein, the term "immune checkpoint inhibitor" refers to a molecule that fully or partially reduces, inhibits, interferes with, or modulates one or more immune checkpoint proteins.

As used herein, the term "immune checkpoint protein" has its ordinary meaning in the art and refers to a molecule expressed by T cells that either turns on a signal (stimulatory checkpoint molecule) or dampens a signal (inhibitory checkpoint molecule). Immune checkpoint molecules are well known in the art and constitute immune checkpoint pathways analogous to CTLA-4 and PD-1-dependent pathways (see for example, Pardoll, 2012. Nature Rev Cancer 12:252-264; Mellman et al., 2011. Nature 480:480-489). Examples of stimulatory checkpoints include CD27, CD28, CD40, CD122, CD137, OX40, GITR, and ICOS. Examples of inhibitory checkpoint molecules include A2AR, B7-H3, B7-H4, BTLA, CTLA-4, CD277, IDO, KIR, PD-1, LAG-3, TIM-3, and VISTA. The PD-1 receptor has two ligands, PD-L1 and PD-L2. One advantage of targeting PD-1 is its ability to restore immune function in the tumor microenvironment. Tumor cells often exploit these checkpoints to evade detection by the immune system. Therefore, inhibiting the checkpoint proteins in immune system can enhance anti-tumor T cell responses.

In some embodiments, an immune checkpoint inhibitor refers to any compound that inhibits the function of an immune checkpoint protein. Inhibition includes reducing the function and completely blocking. In some embodiments, the immune checkpoint inhibitor may be an antibody, synthetic or natural sequence peptide, small molecule, or aptamer that binds to immune checkpoint proteins and their ligands.

In some specific embodiments, the immune checkpoint inhibitor is an antibody. Typically, the antibody targets A2AR, B7-H3, B7-H4, BTLA, CTLA-4, CD277, IDO, KIR, PD-1, LAG-3, TIM-3, or VISTA.

In some embodiments, the immune checkpoint inhibitor is an anti-PD-1 antibody. Examples of commercially available anti-PD-1 antibodies include nivolumab (BMS), pembrolizumab (also known as Lambrolizumab, or MK-3475, MERCK).

### Pharmaceutical Composition

In some aspects, the present disclosure relates to a pharmaceutical composition comprising the compound of Formula (I), the stereoisomer thereof, the isotopic variant thereof, or the pharmaceutically acceptable salt or solvate of any one of the foregoing as provided herein, and at least one pharmaceutically acceptable carrier or excipient.

As used herein, the term "pharmaceutically acceptable carrier and/or excipient" refers to a carrier or excipient that can be used in the manufacture of a pharmaceutical composition, which is generally safe, non-toxic, and neither biologically nor otherwise undesirable, and includes carriers or excipients acceptable for veterinary use as well as human pharmaceutical use. The pharmaceutically acceptable carrier or excipient, as used herein, includes one and more than one such carrier or excipient. The specific carrier or excipient used will depend on the manner and purpose of applying the compounds of the present disclosure. Suitable carriers and excipients are well known to those skilled in the art and are detailed in, for example, Ansel, Howard C et al., Ansel's Pharmaceutical Dosage Forms and Drug Delivery Systems. Philadelphia: Lippincott, Williams & Wilkins, 2004; Gennaro, Alfonso R. et al., Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. It may also include one or more of buffers, stabilizers, surfactants, wetting agents, lubricants, emulsifiers, suspending agents, preservatives, antioxidants, opacifiers, glidants, processing aids, colorants, sweeteners, flavoring agents, taste-masking agents, diluents, and other known additives to provide refined performance of the medicament (*i.e.,* the compounds or pharmaceutical compositions provided herein) or to facilitate the production of pharmaceutical products (*i.e.,* medicaments).

The compositions of the present disclosure can be formulated into various forms. These include, for example, liquid, semi-solid, and solid dosage forms, such as liquid solutions (*e.g*., injectable and infusible solutions), dispersions or suspensions, tablets, pills, powders, liposomes, suppositories, and the like. The form depends on the intended mode of administration and therapeutic application.

The pharmaceutical composition of the present disclosure can be prepared by any well-known pharmaceutical techniques (*e.g*., effective formulation and administration procedures). The aforementioned considerations regarding effective formulation and administration procedures are well-known in the art and are described in standard textbooks. For example, the formulation of pharmaceutical products is discussed in Hoover, John E., Remington's Pharmaceutical Sciences, Mack Publishing Co., Easton, Pennsylvania, 1975; Liberman et al., eds., Pharmaceutical Dosage Forms, Marcel Decker, New York, N.Y., 1980; and Kibbe et al., eds., Handbook of Pharmaceutical Excipients, 3rd edition, American Pharmaceutical Association, Washington, 1999.

In yet another aspect, the present disclosure relates to kits for use in treating diseases and conditions associated with Arf1 pathway activity, such as cancer, comprising the compound of Formula (I), the stereoisomer thereof, the isotopic variant thereof, or the pharmaceutically acceptable salt or solvate of any one of the foregoing as provided herein, or the pharmaceutical composition comprising the compound of Formula (I), the stereoisomer thereof, the isotopic variant thereof, or the pharmaceutically acceptable salt or solvate of any one of the foregoing as provided herein, a container, and optionally a package insert or label indicating the treatment.

### Treatment Methods

In yet another aspect, the present disclosure relates to a method for treating a disease or condition associated with Arf1 pathway activity, such as cancer, in a subject in need thereof, due to the Arf1 inhibitory activity of the compounds of the present disclosure, the method comprising administering to the subject a therapeutically effective amount of the compound of Formula (I), the stereoisomer thereof, the isotopic variant thereof, or the pharmaceutically acceptable salt or solvate of any one of the foregoing as provided herein.

As used herein, the term "subject in need thereof" refers to a subject suffering from a condition associated with Arf1 pathway activity, such as cancer, or a subject who has an increased risk of developing a disease or condition associated with Arf1 pathway activity relative to the general population. In some embodiments, the subject is a warm-blooded animal. In some embodiments, the warm-blooded animal is a mammal. In some embodiments, the warm-blooded animal is a human.

As used herein, the term "disease and condition associated with Arf1 pathway activity" refers to any pathophysiological condition where inhibition of Arf1 would be beneficial. In certain embodiments, the disease and condition associated with Arf1 pathway activity is cancer. In some embodiments, the disease or condition associated with Arf1 pathway activity is cancer or a tumor selected from the group consisting of: breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like. In some embodiments, the disease associated with Arf1 pathway activity is selected from the group consisting of autoimmune diseases, inflammatory diseases, inflammatory bowel disease, arthritis, autoimmune demyelinating disorders, Alzheimer's disease, amyotrophic lateral sclerosis, stroke, ischemia-reperfusion injury, multiple sclerosis, other neurodegenerative diseases, and the like.

In yet another aspect, the present disclosure relates to the compound of Formula (I), the stereoisomer thereof, the isotopic variant thereof, or the pharmaceutically acceptable salt or solvate of any one of the foregoing as provided herein, for use in treating a disease and condition associated with Arf1 pathway activity, such as cancer or a tumor.

In yet another aspect, the present disclosure relates to a use of the compound of Formula (I), the stereoisomer thereof, the isotopic variant thereof, or the pharmaceutically acceptable salt or solvate of any one of the foregoing as provided herein, in the manufacture of a medicament for treating a disease and condition associated with Arf1 pathway activity, such as cancer or a tumor.

The compound of Formula (I), the stereoisomer thereof, the isotopic variant thereof, or the pharmaceutically acceptable salt or solvate of any one of the foregoing as provided herein increases the expression of MHC-I and MHC-II; or compared to DMSO, the compound of Formula (I), the stereoisomer thereof, the isotopic variant thereof, or the pharmaceutically acceptable salt or solvate of any one of the foregoing as provided herein enhances the infiltration and activation of T cells in tumors; or the compound of Formula (I), the stereoisomer thereof, the isotopic variant thereof, or the pharmaceutically acceptable salt or solvate of any one of the foregoing as provided herein increases the expression of T cell activation markers, such as GzmA, GzmB, and Perforin.

The compound of Formula (I), the stereoisomer thereof, the isotopic variant thereof, or the pharmaceutically acceptable salt or solvate of any one of the foregoing as provided herein increases the expression of at least one inflammatory cytokine or chemokine selected from IFNγ, IL-1β, CCL5, CXCL10, CXCL11, and CCL22.

The compound of Formula (I), the stereoisomer thereof, the isotopic variant thereof, or the pharmaceutically acceptable salt or solvate of any one of the foregoing as provided herein increases the expression of at least one inflammatory cytokine or chemokine selected from IFNγ, IL-1β, CCL5, CXCL10, CXCL11, and CCL22.

### Detection and Diagnosis

In yet another aspect, the present disclosure relates to the compound of Formula (I), the stereoisomer thereof, the isotopic variant thereof, or the pharmaceutically acceptable salt or solvate of any one of the foregoing as provided herein, for use as a test agent for diagnosing a disease associated with Arf1 pathway activity in a subject.

In yet another aspect, the present disclosure relates to kits for use in diagnosing a disease associated with Arf1 pathway activity in a subject, comprising at least one compound of Formula (I), the stereoisomer thereof, the isotopic variant thereof, or the pharmaceutically acceptable salt or solvate of any one of the foregoing as provided herein as a test agent.

In some embodiments, the test agent detects at least one indicative biomarker of the presence of a disease associated with Arf1 pathway activity.

In some embodiments, the test agent is capable of detecting Arf1 GTPase activity, serving as a reporter gene for detecting lipolytic activity, detecting lipid droplet formation, detecting autophagy activity, or acting as an upstream or downstream surrogate modulator of Arf1 activity or function.

### Vaccines and Immunization

In yet another aspect, the present disclosure relates to a use of the compound of Formula (I), the stereoisomer thereof, the isotopic variant thereof, or the pharmaceutically acceptable salt or solvate of any one of the foregoing as provided herein, in the manufacture of a therapeutic vaccine for blocking tumor development.

In yet another aspect, the present disclosure relates to a use of the compound of Formula (I), the stereoisomer thereof, the isotopic variant thereof, or the pharmaceutically acceptable salt or solvate of any one of the foregoing as provided herein, in the manufacture of a therapeutic vaccine for blocking tumor development.

The tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

In yet another aspect, the present disclosure relates to a method for preparing a therapeutic vaccine for blocking tumor development, comprising contacting the compound of Formula (I), the stereoisomer thereof, the isotopic variant thereof, or the pharmaceutically acceptable salt or solvate of any one of the foregoing as provided herein with a cancer stem cell, thereby converting the cancer stem cell into a therapeutic vaccine.

In yet another aspect, the present disclosure relates to a vaccination method, comprising administering to a subject the therapeutic vaccine prepared according to the method provided herein. The subject is a mammal, such as a human.

In yet another aspect, the present disclosure relates to a method for killing cells and inducing an anti-tumor immune response, comprising inhibiting at least one Arf1 in cells, particularly the activity of the COPI/Arf1-lipolysis-β-oxidation pathway, via an Arf1 pathway inhibitor.

In some embodiments, the cell is a progenitor cell, a stem cell, a cancer stem cell, or a cancer cell.

In some embodiments, the Arf1 pathway inhibitor is selected from the group consisting of a small molecule Arf1 inhibitor, an RNAi agent targeting Arf1, an antisense agent targeting Arf1, a peptidomimetic Arf1 inhibitor, and a DNA aggregation deoxynucleotide inhibitor containing a target protein Arf1.

In some embodiments, the small molecule Arf1 inhibitor is the compound of Formula (I), the stereoisomer thereof, the isotopic variant thereof, or the pharmaceutically acceptable salt or solvate of any one of the foregoing as provided herein.

### Synthesis

The compounds of the present disclosure can be prepared by the general and specific methods described below, using techniques well-known to those skilled in the art of synthetic organic chemistry. Such general knowledge can be found in standard reference works, such as Barton and Ollis (eds.), Comprehensive Organic Chemistry, Elsevier; Richard Larock, Comprehensive Organic Transformations: A Guide to Functional Group Preparations, John Wiley and Sons; and Compendium of Organic Synthetic Methods, Volumes I-XII, Wiley-Interscience.

The schemes described below are intended to provide a general description of methods for preparing the compounds of the present disclosure. Some compounds of the present disclosure may contain one or multiple chiral centers with stereochemical designations (R) or (S). It will be apparent to those skilled in the art that all synthetic transformations can be carried out in a similar manner, whether the material is enantiomerically enriched or racemic. Furthermore, resolution of the desired optically active material can be performed at any desired point in the procedure using known methods, such as those described herein and in the chemical literature.

### Example

To describe the present disclosure in more detail, the following examples are provided. The examples described herein are intended to illustrate the compounds, methods, and compositions provided herein and should not be construed in any way as limiting their scope.

During the synthesis process, it may be necessary and/or desirable to protect any sensitive or reactive groups on the relevant molecules. This can be achieved using conventional protecting groups, such as those described in T.W. Greene and P.G.M. Wutts, Protective Groups in Organic Synthesis, 4th Edition, John Wiley and Sons. The protecting groups are optionally removed at a convenient subsequent stage using methods well known in the art.

The compounds of the present disclosure can be readily prepared using readily available starting materials, reagents, and conventional synthetic procedures according to the following reaction schemes and examples or modifications thereof. Variants known to those skilled in the art but not mentioned in more detail may also be used in these reactions. Furthermore, other methods for preparing the compounds of the present disclosure will be apparent to those skilled in the art based on the reaction schemes and examples described herein. Unless otherwise specified, all variables are as defined above. In general, in chemical procedures, all reagents and starting materials are commercially available or can be readily prepared by those skilled in the art.

### Example 1

### Synthesis of Compound 1

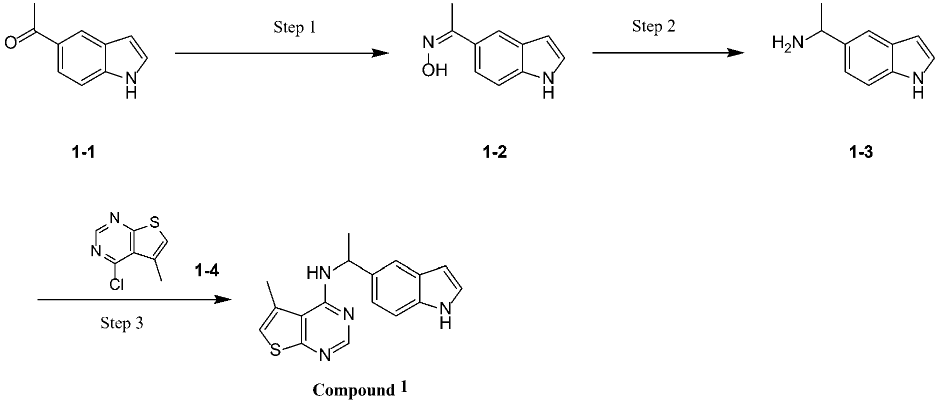

### Step 1: Preparation of Compound 1-2

Compound 1-1 (1.00 g, 6.28 mmol, 1 *eq*) was dissolved in EtOH (5.00 mL). Hydroxylamine hydrochloride (654 mg, 9.42 mmol, 1.5 *eq*) was added portionwise over 0.5 hours at 20°C, followed by the addition of NaOAc (773 mg, 9.42 mmol, 1.5 *eq*) at 20°C. The above reaction mixture was heated to 65°C and stirred for 16.5 hours. LCMS detection found that Compound 1-1 was nearly completely converted, with the main peak corresponding to the molecular weight of the target product. The reaction mixture was filtered, and the filtrate was concentrated to obtain a crude product, which was directly used in the next step. MS(ESI) m/z = 175.1 [M+H]⁺.

### Step 2: Preparation of Compound 1-3

Compound 1-2 (1.00 g, 5.74 mmol, 1 *eq*) and Pd/C (3.00 g, 2.83 mmol, 10.0% content, 1 *eq*) were mixed in EtOH (5.00 mL). The reaction mixture was purged three times each with nitrogen and hydrogen, and then stirred at 20°C under a hydrogen atmosphere (15 Psi) for 8 hours. LCMS detection found that Compound 1-2 was nearly completely converted, with the main peak corresponding to the molecular weight of the target product. The reaction mixture was filtered, and the filtrate was concentrated to obtain a crude product. The crude product was purified by preparative high-performance liquid chromatography (chromatographic column: Phenomenex C18 75*30 mm*3 µm; mobile phase: [water (TFA)-acetonitrile]; B%: 0%-24%, 36 minutes) to obtain Compound 1-3 (600 mg, 3.74 mmol, yield: 65.2%) as a yellow solid. MS(ESI) m/z = 144.0 [M+H]⁺.

**¹H NMR:** 400 MHz, DMSO-*d*₆ δ (ppm) = 11.21 (br s, 1H), 7.64 (s, 1H), 7.43 (d, *J =* 8.4 Hz, 1H), 7.39 (t, *J =* 2.8 Hz, 1H), 7.20 (dd, *J =* 8.4, 1.6 Hz, 1H), 6.45 (td, *J =* 2.0, 1.2 Hz, 1H), 4.05 (br s, 2H), 4.02 (br s, 1H), 1.55 (d, *J =* 6.8 Hz, 2H).

### Step 3: Preparation of Compound 1

Compound 1-3 (600 mg, 3.74 mmol, 1 *eq*) was dissolved in THF (3.00 mL). DIEA (968 mg, 7.49 mmol, 1.30 mL, 2 *eq)* and Compound 1-4 (100 mg, 541 µmol, 1 *eq*) were added. The above reaction mixture was heated to 50°C and stirred for 16 hours. LCMS detection found that Compound 1-3 was nearly completely converted, with the main peak corresponding to the molecular weight of the target product. The reaction mixture was cooled to 0°C, quenched with 5.00 mL of water, and extracted with EtOAc (10.0 mL). The phases were separated. The organic phase was washed with saturated brine (5.0 mL), dried over anhydrous sodium sulfate, and filtered. The filtrate was concentrated to obtain a crude product. The crude product was purified by column chromatography (silica gel, petroleum ether/ethyl acetate = 100/1 to 1/1) to obtain Compound 1 (30.0 mg, 94.1 µmol, yield: 2.51%) as a yellow solid. MS(ESI) m/z = 309.0 [M+H]⁺.

**¹H NMR:** (400 MHz, DMSO-*d*₆) δ (ppm) = 11.04 (br s, 1H), 8.45 (s, 1H), 7.62 (s, 1H), 7.35 (d, *J =* 8.4 Hz, 1H), 7.32 (t, *J* = 2.8 Hz, 1H), 7.28 - 7.26 (m, 1H), 7.24 - 7.15 (m, 1H), 7.04 - 6.95 (m, 1H), 6.38 (br s, 1H), 5.65 - 5.56 (m, 1H), 2.66 (s, 3H), 1.65 (d, *J =* 6.8 Hz, 3H).

### Example 2

### Synthesis of Compound 2

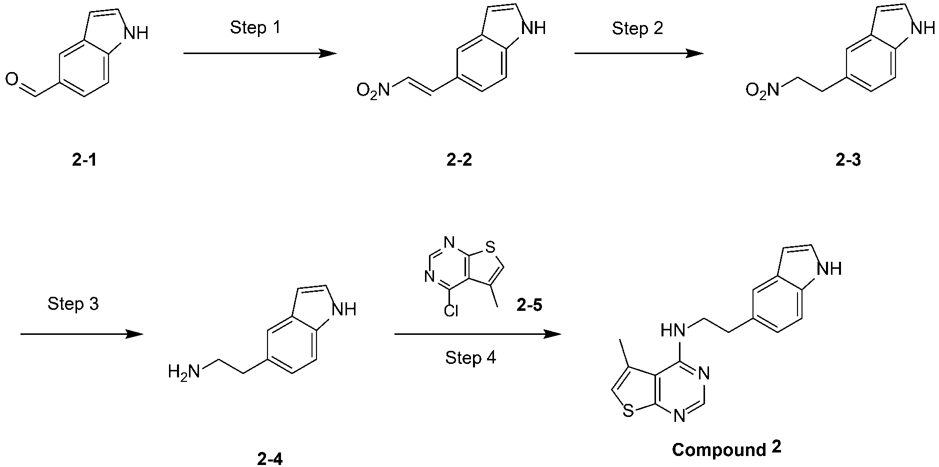

### Step 1: Preparation of Compound 2-2

Compound 2-1 (5.00 g, 34.5 mmol, 1 *eq*) was dissolved in CH₃NO₂ (40.1 g, 657 mmol, 35.5 mL, 19.1 *eq*). NH₄OAc (3.45 g, 44.8 mmol, 1.30 *eq*) was added, and the reaction mixture was heated to 110°C and stirred for 3 hours. LCMS detection found that Compound 2-1 was nearly completely converted, with the main peak corresponding to the molecular weight of the target product. The reaction mixture was concentrated to obtain a residue, which was purified by column chromatography (silica gel, petroleum ether/ethyl acetate = 99/1 to 3/1) to obtain Compound 2-2 (3.57 g, 19.0 mmol, yield: 55.1%) as an orange-yellow solid. MS(ESI) m/z = 189.1 [M+H]⁺.

**¹H NMR:** (400 MHz, DMSO-*d*₆) δ (ppm) = 11.51 (br s, 1H), 8.26 - 8.21 (m, 1H), 8.17 - 8.12 (m, 1H), 8.08 (s, 1H), 7.61 (dd, *J =* 8.6, 1.6 Hz, 1H), 7.49 - 7.44 (m, 2H), 6.54 (d, *J =* 2.1 Hz, 1H).

### Step 2: Preparation of Compound 2-3

Compound 2-2 (2.47 g, 13.1 mmol, 1 *eq*) was dissolved in MeOH (17.0 mL), and NaBH₄ (1.78 g, 47.1 mmol, 3.58 *eq*) was added. The reaction mixture was stirred at 25°C for 0.6 hours. TLC (petroleum ether/ethyl acetate = 3/1) detection showed that Compound 2-2 was nearly completely converted. The reaction was quenched with 20.0 mL of water at 0-10°C, and then the pH was adjusted to approximately 7.0 with acetic acid. The mixture was extracted twice with EtOAc (20 mL). The organic phases were combined, washed twice with saturated brine (10.0 mL), dried over anhydrous sodium sulfate, and filtered. The filtrate was concentrated to obtain a crude product, which was purified by column chromatography (silica gel, petroleum ether/ethyl acetate = 99/1 to 4/1) to obtain Compound 2-3 (1.04 g, 5.47 mmol, yield: 41.7%) as a yellow oil.

**¹H NMR:** (400 MHz, DMSO-*d*₆) δ (ppm) = 11.04 (br s, 1H), 7.39 (s, 1H), 7.34 - 7.28 (m, 2H), 6.98 (dd, *J* = 8.3, 1.6 Hz, 1H), 6.36 (t, *J =* 2.1 Hz, 1H), 4.82 (t, *J =* 7.0 Hz, 2H), 3.26 (t, *J =* 7.0 Hz, 2H).

### Step 3: Preparation of Compound 2-4

Compound 2-3 (1.04 g, 5.47 mmol, 1 *eq*) was dissolved in MeOH (2.2 mL), and Pd/C (100 mg, 547 µmol, 10% content, 0.1 *eq*) was added. The reaction mixture was purged three times each with argon and hydrogen. The reaction mixture was stirred at 25°C under a hydrogen atmosphere (15 Psi) for 12 hours. LCMS detection found that Compound 2-3 was nearly completely converted, with the main peak corresponding to the molecular weight of the target product. The reaction mixture was filtered, and the filtrate was concentrated to obtain the crude product of Compound 2-4 (730 mg) as a white oil. MS(ESI) m/z = 161.1 [M+H]⁺.

**¹HNMR:** (400 MHz, DMSO-*d*₆) δ (ppm) = 10.95 (br s, 1H), 7.34 - 7.23 (m, 3H), 6.91 (d, *J =* 8.3 Hz, 1H), 6.33 (br s, 1H), 2.79 - 2.73 (m, 2H), 2.70 - 2.64 (m, 2H).

### Step 4: Preparation of Compound 2

Compound 2-4 (150 mg, 936 µmol, 1 *eq*) was dissolved in THF (3.00 mL), followed by the addition of DIEA (363 mg, 2.81 mmol, 489 µL, 3 *eq*) and Compound 2-5 (173 mg, 936 µmol, 1 *eq*). The above reaction mixture was stirred at 25°C for 12 hours. LCMS detection found that Compound 2-4 was nearly completely converted, with the main peak corresponding to the molecular weight of the target product. The reaction mixture was concentrated to obtain a crude product. The crude product was suspended in EtOH (3.0 mL) and stirred at 25°C for 3 hours. The mixture was filtered, and the filter cake was dried to obtain Compound 2 (44.0 mg, 142 µmol, yield: 15.2%) as a white solid. MS(ESI) m/z = 309.1 [M+H]⁺.

**¹HNMR:** (400 MHz, DMSO-*d*₆) δ (ppm) = 10.99 (br s, 1H), 8.35 (s, 1H), 7.42 (s, 1H), 7.35 - 7.25 (m, 2H), 7.11 (s, 1H), 7.00 (d, *J =* 8.0 Hz, 1H), 6.70 (br t, *J =* 5.3 Hz, 1H), 6.36 (br s, 1H), 3.79 - 3.72 (m, 2H), 2.98 (br t, *J =* 7.3 Hz, 2H), 2.47 (s, 3H).

### Example 3

### Synthesis of Compound 3

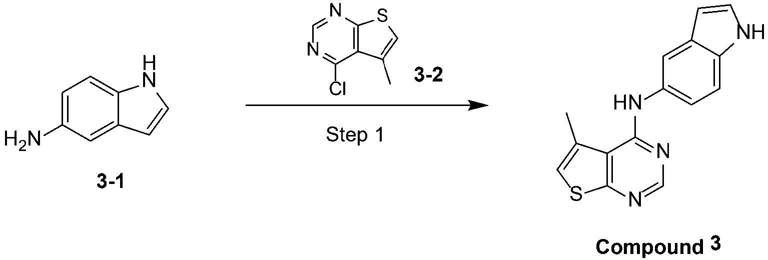

### Step 1: Preparation of Compound 3-2

Compound 3-2 (143 mg, 1.08 mmol, 1 *eq*), Compound 3-1 (143 mg, 1.08 mmol, 1 *eq*), and TFA (12.3 mg, 108 µmol, 8.02 µL, 0.1 *eq*) were mixed in isopropanol (1.0 mL). The reaction mixture was purged three times with nitrogen, heated to 85°C, and stirred under a nitrogen atmosphere for 12 hours. LCMS detection found that Compound 3-1 was nearly completely converted, with the main peak corresponding to the molecular weight of the target product. The reaction mixture was concentrated to obtain a residue. The residue was purified by preparative high-performance liquid chromatography (chromatographic column: Welch Xtimate C18 150*30 mm*5 µm; mobile phase: [water (NH₄HCO₃)-acetonitrile]; B%: 18%-58%, 36 minutes) to obtain Compound 3 (51.9 mg, 181 µmol, yield: 16.8%) as an off-white solid. MS(ESI) m/z = 281.0 [M+H]⁺.

**¹H NMR:** (400 MHz, DMSO-*d*₆) δ (ppm) = 11.07 (br s, 1H), 8.31 (s, 1H), 8.21 (s, 1H), 7.79 (d, *J =* 1.6 Hz, 1H), 7.38 (d, *J =* 8.6 Hz, 1H), 7.35 (t, *J =* 2.7 Hz, 1H), 7.28 - 7.24 (m, 2H), 6.42 (t, *J =* 2.0 Hz, 1H), 2.74 (d, *J =* 0.6 Hz, 3H).

### Example 4

### Synthesis of Compound 4

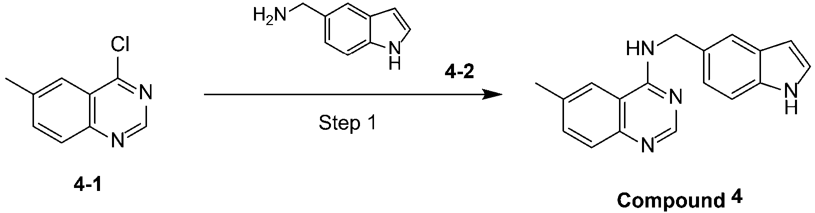

### Step 1: Preparation of Compound 4

Compound 4-1 (200 mg, 1.12 mmol, 1 *eq*) and Compound 4-2 (212 mg, 1.46 mmol, 1.3 *eq*) were dissolved in THF (2.0 mL), and DIEA (217 mg, 1.68 mmol, 292 µL, 1.5 *eq*) was added. The above reaction mixture was stirred at 20°C for 12 hours. LCMS detection found that Compound 4-1 was nearly completely converted, with the main peak corresponding to the molecular weight of the target product. The reaction was quenched with 20.0 mL of water and extracted twice with EtOAc (20.0 mL). The organic phases were combined, washed with saturated brine (20.0 mL), dried over anhydrous sodium sulfate, and filtered. The filtrate was concentrated to obtain a residue, which was purified by column chromatography (silica gel, petroleum ether/ethyl acetate = 99/1 to 0/1) to obtain a crude product. The crude product was suspended in EtOH and stirred at 20°C for 12 hours. The mixture was filtered, and the filter cake was dried to obtain Compound 4 (100 mg, 346 µmol, yield: 25.8%) as a white solid. MS(ESI) m/z = 289.0 [M+H]⁺.

**¹H NMR:** (400 MHz, DMSO-*d*₆) δ (ppm) = 10.99 (s, 1H), 8.65 (t, *J =* 6.0 Hz, 1H), 8.39 (s, 1H), 8.11 (s, 1H), 7.59 (s, 2H), 7.51 (s, 1H), 7.35 - 7.31 (m, 1H), 7.29 (t, *J =* 2.8 Hz, 1H), 7.15 - 7.10 (m, 1H), 6.38 - 6.34 (m, 1H), 4.83 (d, *J =* 6.0 Hz, 2H), 2.45 (s, 3H).

### Example 5

### Synthesis of Compound 5

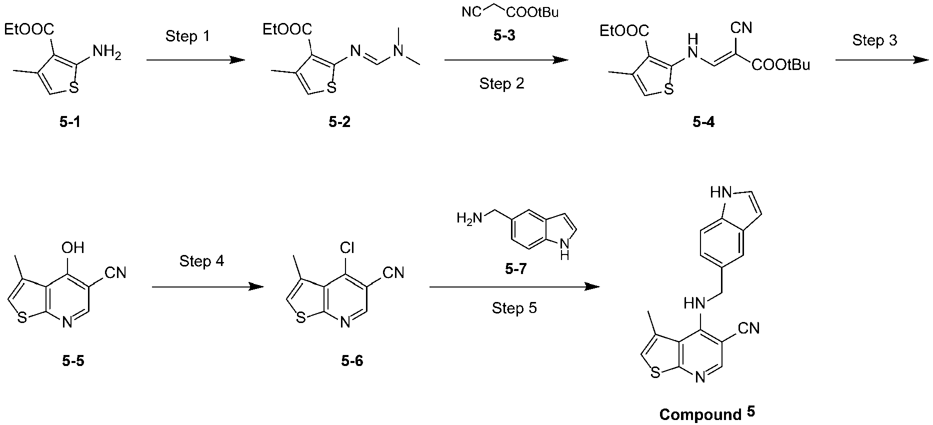

### Step 1: Preparation of Compound 5-2

Compound 5-1 (20.0 g, 108 mmol, 1 eq) was dissolved in DMF-DMA (35.9 g, 301 mmol, 40.0 mL, 2.79 eq). The reaction mixture was heated to 100°C and stirred for 12 hours. LCMS detection found that Compound 5-1 was nearly completely converted, with the main peak corresponding to the molecular weight of the target product. The reaction mixture was concentrated to obtain a crude product. The crude product was suspended in H₂O and stirred at 20°C for 10 minutes. The mixture was filtered, and the filter cake was dried under reduced pressure to obtain Compound 5-2 (24.0 g, 99.8 mmol, yield: 92.5%) as a yellow solid. MS(ESI) m/z = 240.9 [M+H]⁺.

**HNMR:** (400 MHz, CDCl₃) δ (ppm) = 7.63 (s, 1H), 6.22 (d, *J =* 0.8 Hz, 1H), 4.35 - 4.17 (m, 2H), 3.07 (br d, *J =* 8.0 Hz, 6H), 2.31 (d, *J =* 0.8 Hz, 3H), 1.34 (t, *J* = 7.2 Hz, 3H).

### Step 2: Preparation of Compound 5-4

Compound 5-2 (24.0 g, 99.8 mmol, 1 *eq*) was dissolved in *tert*-butanol (50.0 mL), and Compound 5-3 (56.4 g, 399 mmol, 57.1 mL, 4 *eq*) was added. The reaction mixture was stirred at 20°C for 18 hours. LCMS detection found that Compound 5-2 was nearly completely converted, with the main peak corresponding to the molecular weight of the target product. The reaction mixture was filtered, and the filter cake was dried to obtain the crude product of Compound 5-4 (27.3 g) as a yellow solid. MS(ESI) m/z = 337.0 [M+H]⁺.

**¹H NMR:** (400 MHz, CDCl₃) δ (ppm) = 12.52 (br d, *J =* 13.2 Hz, 1H), 7.55 (d, *J =* 13.2 Hz, 1H), 6.37 (s, 1H), 4.48 - 4.41 (m, 2H), 2.37 (d, *J =* 0.8 Hz, 3H), 1.57 (s, 9H), 1.42 (t, *J=* 7.2 Hz, 3H).

### Step 3: Preparation of Compound 5-5

Compound 5-4 (27.0 g, 80.2 mmol, 1 *eq*) was dissolved in Ph₂O (150.0 mL), and the reaction mixture was stirred at 255°C for 2 hours. LCMS detection found that Compound 5-4 was nearly completely converted, and the molecular weight corresponding to the target product was detected. The reaction mixture was cooled to 20°C, and n-hexane (300.0 mL) was added. After shaking, the mixture was filtered. The filter cake was dried to obtain the crude product of Compound 5-5 (11.5 g) as a brown solid. MS(ESI) m/z = 190.9 [M+H]⁺.

**¹H NMR:** (400 MHz, DMSO-*d*₆) δ (ppm) = 13.36 (br s, 1H), 8.56 (s, 1H), 7.00 - 6.98 (m, 1H), 2.47 (d, *J* = 0.8 Hz, 3H).

### Step 4: Preparation of Compound 5-6

Compound 5-5 (500 mg, 2.63 mmol, 1 *eq)* was dissolved in toluene (2.5 mL), and POCl₃ (2.02 g, 13.1 mmol, 1.22 mL, 5 *eq*) was added. The reaction mixture was stirred at 100°C for 4 hours. LCMS detection found that Compound 5-5 was nearly completely converted, and the molecular weight corresponding to the target product was detected. The reaction mixture was cooled to 25°C and added to water. The pH was adjusted to approximately 10 with sodium bicarbonate. The phases were separated, and the organic phase was concentrated under reduced pressure to obtain the crude product of Compound 5-6 (300 mg) as a yellow solid. MS(ESI) m/z = 208.8 [M+H]⁺.

**¹H NMR:** (400 MHz, DMSO-*d*₆) δ (ppm) = 8.94 (s, 1H), 7.85 (d, *J* = 0.8 Hz, 1H), 2.65 (d, *J=* 1.2 Hz, 3H) ppm.

### Step 5: Preparation of Compound 5

Compound 5-6 (100 mg, 479 µmol, 1 *eq),* Compound 5-7 (77.0 mg, 527 µmol, 1.1 *eq),* and DIEA (124 mg, 958 µmol, 167 µL, 2 *eq)* were mixed in THF (1.0 mL), and the reaction mixture was stirred at 20°C for 12 hours. LCMS detection found that Compound 5-6 was nearly completely converted, and the molecular weight corresponding to the target product was detected. The reaction was quenched with 30.0 mL of water and extracted twice with EtOAc (20.0 mL). The organic phases were combined, washed with saturated brine (20.0 mL), dried over anhydrous sodium sulfate, and filtered. The filtrate was concentrated to obtain a residue, which was purified by preparative high-performance liquid chromatography (chromatographic column: Welch Xtimate C18 150*30 mm*5 µm; mobile phase: [water (NH₃H₂O+NH₄HCO₃)-acetonitrile]; B%: 28%-68%, 36 minutes) to obtain Compound 5 (30.0 mg, 92.9 µmol, yield: 19.3%) as a pale yellow solid. MS(ESI) m/z = 319.1 [M+H]⁺.

**¹H NMR:** (400 MHz, DMSO-*d*₆) δ (ppm) = 11.10 (br s, 1H), 8.28 (s, 1H), 7.56 (s, 1H), 7.38 (d, *J* = 8.2Hz, 1H), 7.34 - 7.30 (m, 2H), 7.13 (d, *J=* 8.2 Hz, 1H), 6.93 - 6.86 (m, 1H), 6.39 (br s, 1H), 5.06 (d, *J* = 6.4 Hz, 2H), 2.63 (s, 3H).

### Example 6

### Synthesis of Compound 6

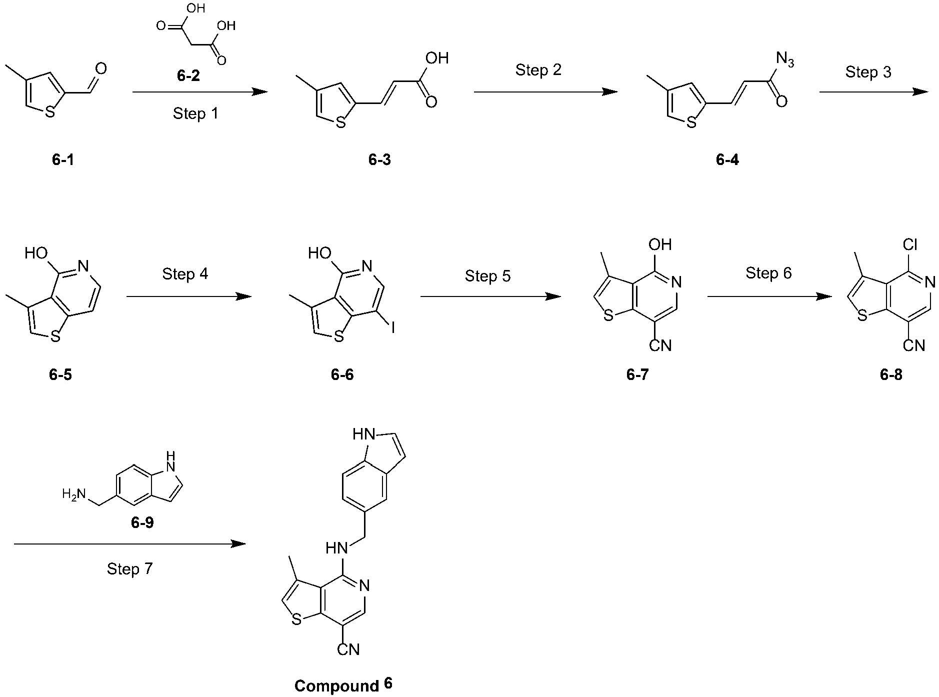

### Step 1: Preparation of Compound 6-3

Compound 6-1 (18.0 g, 142 mmol, 1 *eq*) and Compound 6-2 (59.4 g, 570 mmol, 59.3 mL, 4 *eq*) were dissolved in pyridine (120.0 mL), and piperidine (1.21 g, 14.2 mmol, 1.41 mL, 0.1 *eq*) was added. The reaction mixture was heated to 120°C and stirred for 12 hours. LCMS detection found that Compound 6-1 was nearly completely converted, and the molecular weight corresponding to the target product was detected. The reaction mixture was cooled to 25°C and concentrated under reduced pressure to obtain a residue. The residue was added to water, and the pH was adjusted to 4.0 with 1 N dilute hydrochloric acid. The mixture was extracted twice with EtOAc (200 mL). The organic phases were combined, washed with saturated brine (50.0 mL), dried over anhydrous sodium sulfate, and filtered. The filtrate was concentrated to obtain the crude product of Compound 6-3 (20.0 g) as a yellow solid. MS(ESI) m/z = 169.0 [M+H]⁺.

**¹H NMR:** (400 MHz, CDCl₃) δ (ppm) = 9.78 (br s, 1H), 7.82 (d, *J* = 15.6 Hz, 1H), 7.11 (s, 1H), 7.02 (s, 1H), 6.20 (d, *J* = 15.6 Hz, 1H), 2.27 (s, 3H).

### Step 2: Preparation of Compound 6-4

Compound 6-3 (20.0 g, 118 mmol, 1 *eq)* and TEA (8.0 g, 178 mmol, 24.8 mL, 1.5 *eq)* were dissolved in acetone (150 mL). Ethyl chloroacetate (17.8 g, 130 mmol, 17.1 mL, 1.1 *eq*) was added at 0°C, and the mixture was stirred at 20°C for 20 minutes. Sodium azide (13.7 g, 211 mmol, 1.78 *eq*) was then added, and the final mixture was stirred at 20°C for an additional 6 hours. LCMS detection found that Compound 6-3 was nearly completely converted, and the molecular weight corresponding to the target product was detected. The reaction mixture was added to water, and the pH was adjusted to 9.0 with sodium carbonate. The mixture was extracted twice with EtOAc (150 mL). The organic phases were combined, washed sequentially with water (150 mL) and saturated brine (20.0 mL), dried over anhydrous sodium sulfate, filtered, and the filtrate was concentrated to obtain the crude product of Compound 6-4 (20.0 g) as a yellow solid. MS(ESI) m/z = 166.0 [M+H-N₂]⁺.

**¹H NMR:** (400 MHz, DMSO-*d*₆) δ (ppm) = 7.85 (d, *J* = 15.6 Hz, 1H), 7.48 (s, 1H), 7.43 (s, 1H), 6.25 (d, *J* = 15.6 Hz, 1H), 2.21 (s, 3H).

### Step 3: Preparation of Compound 6-5

Compound 6-4 (20.0 g, 103 mmol, 1 *eq*) was dissolved in xylene (130 mL), and I₂ (5.25 g, 20.7 mmol, 4.17 mL, 0.2 *eq*) was added. The reaction mixture was heated to 145°C and stirred for 6 hours. LCMS detection found that Compound 6-4 was nearly completely converted, and the molecular weight corresponding to the target product was detected. The reaction mixture was cooled and filtered. The filter cake was dried to obtain a crude product. The crude product was suspended in MTBE/THF (10/1, 200 mL) and stirred at 20°C for 12 hours. The mixture was filtered, and the filter cake was dried to obtain Compound 6-5 (4.50 g, 27.2 mmol, yield: 26.3%) as a black solid. MS(ESI) m/z = 166.0 [M+H]⁺.

**¹H NMR:** (400 MHz, DMSO-*d*₆) δ (ppm) = 11.24 (br s, 1H), 7.18 - 7.14 (m, 1H), 7.13 (s, 1H), 6.75 (d, *J* = 7.2 Hz, 1H), 2.50 (br s, 3H).

### Step 4: Preparation of Compound 6-6

Compound 6-5 (1.00 g, 6.05 mmol, 1 *eq*) was dissolved in THF (10.0 mL), and NIS (1.91 g, 8.47 mmol, 1.4 *eq*) was added at -78°C. The reaction mixture was slowly warmed to 20°C and stirred for 12 hours. LCMS detection found that Compound 6-5 was nearly completely converted, and the molecular weight corresponding to the target product was detected. The reaction mixture was filtered, and the filter cake was dried to obtain the crude product of Compound 6-6 (600 mg) as a yellow solid. MS(ESI) m/z = 291.8 [M+H]⁺.

**¹H NMR:** (400 MHz, DMSO-*d*₆) δ (ppm) = 11.47 (br s, 1H), 7.43 (s, 1H), 7.25 (s, 1H), 2.47 (s, 3H).

### Step 5: Preparation of Compound 6-7

Compound 6-6 (550 mg, 1.89 mmol, 1 *eq*) was dissolved in DMF (0.5 mL), and CuCN (338 mg, 3.78 mmol, 825 µL, 2 *eq*) was added. The reaction mixture was stirred at 145°C for 12 hours. LCMS detection found that Compound 6-6 was nearly completely converted, and the molecular weight corresponding to the target product was detected. The reaction was quenched with 30.0 mL of water and extracted twice with EtOAc (20.0 mL). The organic phases were combined, washed with saturated brine (20.0 mL), dried over anhydrous sodium sulfate, and filtered. The filtrate was concentrated to obtain a residue, which was purified by column chromatography (silica gel, petroleum ether/ethyl acetate = 99/1 to 3/1) to obtain Compound 6-7 (165 mg, 867 µmol, 45.9%) as a yellow solid. MS(ESI) m/z = 190.9 [M+H]⁺.

**¹H NMR:** (400 MHz, DMSO-*d*₆) δ (ppm) = 12.20 (br s, 1H), 8.19 (br s, 1H), 7.52 - 7.24 (m, 1H), 2.50 (s, 3H).

### Step 6: Preparation of Compound 6-8

Phosphoryl chloride (1.65 g, 10.7 mmol, 1 mL, 12.4) was added to Compound 6-7 (165 mg, 867 µmol, 1 *eq*)*.* The reaction mixture was heated to 100°C and stirred for 6 hours. LCMS detection found that Compound 6-7 was nearly completely converted, and the molecular weight corresponding to the target product was detected. The reaction mixture was cooled to 25°C and slowly added to water. The pH was adjusted to 10.0 with sodium bicarbonate. The mixture was filtered, and the filter cake was dried to obtain the crude product of Compound 6-8 (160 mg) as a yellow solid. MS(ESI) m/z = 208.9 [M+H]⁺.

**¹H NMR:** (400 MHz, DMSO-*d*₆) δ (ppm) = 8.79 (s, 1H), 7.72 (s, 1H), 2.66 (s, 3H).

### Step 7: Preparation of Compound 6

Compound 6-8 (150 mg, 718 µmol, 1 *eq*) and Compound 6-9 (105 mg, 718 µmol, 1 *eq*) were dissolved in THF (1.0 mL). DIEA (186 mg, 1.44 mmol, 250 µL, 2 *eq*) was added, and the reaction mixture was stirred at 25°C for 12 hours. LCMS detection found that Compound 6-8 was nearly completely converted, and the molecular weight corresponding to the target product was detected. The reaction was quenched with 30.0 mL of water and extracted twice with EtOAc (20.0 mL). The organic phases were combined, washed with saturated brine (20.0 mL), dried over anhydrous sodium sulfate, and filtered. The filtrate was concentrated to obtain a residue, which was purified by preparative high-performance liquid chromatography (chromatographic column: Welch Ultimate XB-CN 250*50*10 µm; mobile phase: [Heptane-EtOH (0.1% NH₃H₂O)]; B%: 15%-45%, 15 minutes) to obtain Compound 6 (30.0 mg, 91.1 µmol, yield: 12.6%) as a yellow solid. MS(ESI) m/z = 318.9 [M+H]⁺.

**¹H NMR:** (400 MHz, DMSO-*d*₆) δ (ppm) = 11.00 (br s, 1H), 8.34 (s, 1H), 7.51 (s, 1H), 7.41 (s, 1H), 7.37 - 7.31 (m, 2H), 7.29 (t, *J* = 2.8 Hz, 1H), 7.15 - 7.11 (m, 1H), 6.36 (br s, 1H), 4.84 (d, *J* = 6.0 Hz, 2H), 2.68 (s, 3H).

### Example 7

### Synthesis of Compound 7

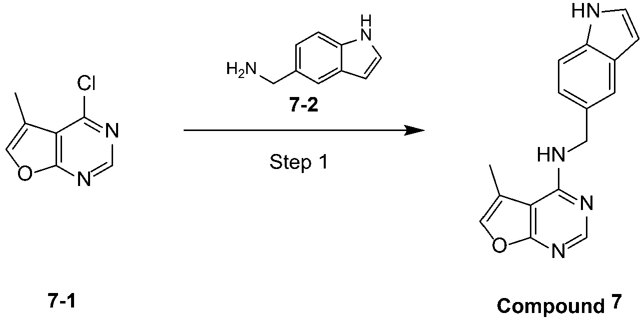

### Step 1: Preparation of Compound 7

Compound 7-1 (78.1 mg, 534 µmol, 1 *eq*) was dissolved in THF (2.0 mL). Compound 7-2 (90.0 mg, 534 µmol, 1 *eq*) and DIEA (69.0 mg, 534 µmol, 93.0 µL, 1 *eq*) were added. The reaction mixture was stirred at 25°C for 12 hours. LCMS detection showed 17.2% of Compound 7-2 remained, with the target product content at 68.6%. The reaction mixture was quenched with 3.0 mL of water and extracted twice with EtOAc (5.0 mL). The organic phases were combined, washed twice with saturated brine (5.0 mL), dried over anhydrous sodium sulfate, and filtered. The filtrate was concentrated to obtain a residue, which was purified by column chromatography (silica gel, petroleum ether/ethyl acetate = 2/1) to obtain Compound 7 (43.0 mg, 155 µmol, yield: 28.9%) as a white solid. MS(ESI) m/z = 279.0 [M+H]⁺.

**¹H NMR:** (400 MHz, DMSO-*d*₆) δ (ppm) = 10.99 (br s, 1H), 8.19 (s, 1H), 7.56 (d, *J* = 1.3 Hz, 1H), 7.50 (s, 1H), 7.45 (br t, *J* = 6.2 Hz, 1H), 7.32 (d, *J* = 8.3 Hz, 1H), 7.29 (t, *J* = 2.7 Hz, 1H), 7.12 (dd, *J* = 1.3, 8.3 Hz, 1H), 6.44 - 6.32 (m, 1H), 4.79 (d, *J* = 6.1 Hz, 2H), 2.36 - 2.32 (m, 3H).

### Example 8

### Synthesis of Compound 8

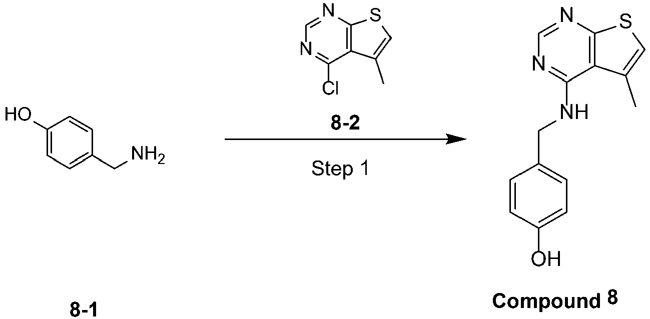

### Step 1: Preparation of Compound 8

Compound 8-1 (100 mg, 812 µmol, 1 *eq*), Compound 8-2 (179 mg, 974 µmol, 1.2 *eq),* and TEA (164 mg, 1.62 mmol, 226 µL, 2 *eq)* were mixed in THF (0.5 mL). The reaction mixture was purged three times with nitrogen, and then the reaction mixture was heated to 50°C and stirred under a nitrogen atmosphere for 16 hours. LCMS detection found that Compound 8-1 was nearly completely converted, with the main peak corresponding to the molecular weight of the target product. The reaction mixture was cooled to room temperature, quenched with 5.0 mL of water, and extracted with EtOAc (5.0 mL). The organic phase was washed with saturated brine (5.0 mL), dried over anhydrous sodium sulfate, and filtered. The filtrate was concentrated to obtain a residue, which was purified by preparative high-performance liquid chromatography (chromatographic column: Welch Xtimate C18 150*30 mm*5 µm; mobile phase: [water (NH₄HCO₃)-acetonitrile]; B%: 10%-50%, 36 minutes) to obtain Compound 8 (35.0 mg, 122 µmol, yield: 15.0%) as a white solid. MS(ESI) m/z = 271.9 [M+H]⁺.

**¹H NMR:** (400 MHz, DMSO-*d*₆) δ (ppm) = 9.25 (s, 1H), 8.45 (s, 1H), 8.25 - 8.19 (m, 1H), 7.71 (d, *J* = 1.3 Hz, 1H), 7.14 (d, *J* = 8.5 Hz, 2H), 6.72 - 6.64 (m, 2H), 4.60 (d, *J =* 6.0 Hz, 2H), 2.33 - 2.31 (m, 3H).

### Example 9

### Synthesis of Compound 9 and Compound 10

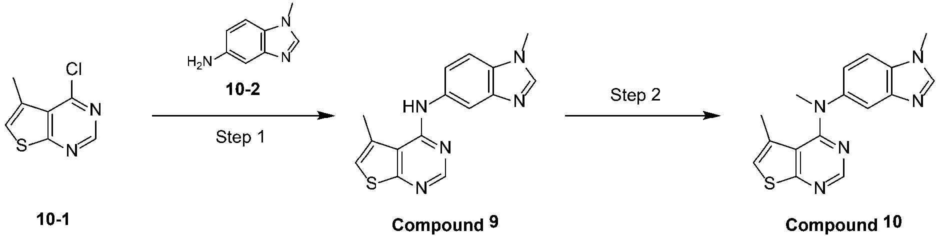

### Step 1: Preparation of Compound 9

Compound 10-1 (500 mg, 2.71 mmol, 1 *eq*), Compound 10-2 (398 mg, 2.71 mmol, 1 *eq*), and TFA (30.9 mg, 271 µmol, 20.1 µL, 0.1 *eq*) were mixed in an isopropanol (3.0 mL) solution. The reaction mixture was purged three times with nitrogen, heated to 85°C, and stirred under a nitrogen atmosphere for 12 hours. LCMS detection showed that the starting material 10-1 was nearly completely converted. The reaction mixture was cooled to room temperature and filtered. The filter cake was suspended in acetonitrile (2.0 mL) and stirred at 25°C for 3 hours. After filtration, the filter cake was dried to obtain Compound 9 (623 mg, 2.06 mmol, yield: 76.2%) as a white solid. MS(ESI) m/z = 296.0 [M+H]⁺.

**¹H NMR:** (400 MHz, DMSO-*d*₆) δ (ppm) = 9.51 (s, 1H), 8.72 (br s, 1H), 8.48 (s, 1H), 8.34 (d, *J* = 1.6 Hz, 1H), 7.94 (d, *J* = 9.0 Hz, 1H), 7.85 - 7.80 (m, 1H), 7.38 (d, *J =* 1.0 Hz, 1H), 4.07 (s, 3H), 2.78 (d, *J* = 0.9 Hz, 3H).

### Step 2: Preparation of Compound 10

Compound 9 (400 mg, 1.35 mmol, 1 *eq*) was dissolved in DMF (4.0 mL), and MeI (346 mg, 2.44 mmol, 152 µL, 1.8 *eq)* and cesium carbonate (1.10 g, 3.39 mmol, 2.5 *eq*) were added. The reaction mixture was stirred at 25°C for 4 hours. TLC (dichloromethane/methanol = 10/1) detection showed that Compound 9 was nearly completely converted. The reaction mixture was added to H₂O (3.0 mL) and extracted twice with EtOAc (3.0 mL). The organic phases were combined, washed with saturated brine (4.0 mL), dried over anhydrous sodium sulfate, and filtered. The filtrate was concentrated to obtain a residue, which was purified by preparative high-performance liquid chromatography (neutral conditions, chromatographic column Xtimate C18 150*40 mm*10 µm; mobile phase: [water (NH₃H₂O+NH₄HCO₃)-acetonitrile]; gradient: 12%-52% B, 36 minutes) to obtain Compound 10 (50.0 mg, 162 µmol, yield: 12.5%) as a yellow solid. MS(ESI) m/z = 310.1 [M+H]⁺.

**¹H NMR:** (400 MHz, DMSO-*d*₆) δ (ppm) = 8.66 (s, 1H), 8.16 (s, 1H), 7.50 (d, *J* = 8.6 Hz, 1H), 7.24 (d, *J* = 1.9 Hz, 1H), 7.09 (d, *J* = 0.9 Hz, 1H), 7.05 - 6.98 (m, 1H), 3.81 (s, 3H), 3.55 (s, 3H), 1.43 (s, 3H).

### Example 10

### Synthesis of Compound 11

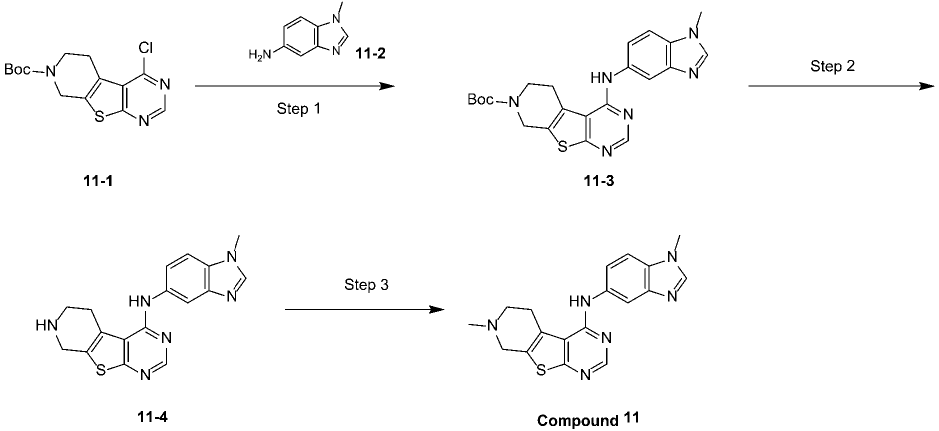

### Step 1: Preparation of Compound 11-3

Compound 11-1 (200 mg, 613 µmol, *1 eq)* was dissolved in isopropanol (2.0 mL). Compound 11-2 (90.1 mg, 613 µmol, 1 *eq*) and TFA (7.00 mg, 61.3 µmol, 4.56 µL, 0.1 *eq*) were added. The reaction mixture was heated to 80°C and stirred for 12 hours. LCMS detection showed that Compound 11-1 was nearly completely converted, with the main peak corresponding to the molecular weight of the target product. The reaction mixture was cooled and concentrated under reduced pressure to obtain the crude product of Compound 11-3 (267 mg) as a brown solid. MS(ESI) m/z = 437.1 [M+H]⁺.

### Step 2: Preparation of Compound 11-4

Compound 11-3 (250 mg, 572 µmol, 1 *eq*) was dissolved in MeOH (2.0 mL). A solution of HCl (gas) in 1,4-dioxane (4 M, 143 µL, 1 *eq*) was slowly added dropwise at 25°C. After the dropwise addition was completed, the reaction mixture was stirred at 25°C for 2 hours. LCMS detection showed that Compound 11-3 was nearly completely converted, with the main peak corresponding to the molecular weight of the target product. The reaction mixture was concentrated to obtain the crude product of Compound 11-4 (148 mg) as a white solid. MS(ESI) m/z = 337.0 [M+H]⁺.

### Step 3: Preparation of Compound 11

Compound 11-4 (148 mg, 441 µmol, 1 *eq*) was dissolved in EtOH (5.0 mL). Aqueous formaldehyde solution (184 mg, 2.21 mmol, 169 µL, 36.0% purity, 5 *eq*) was added dropwise at 20°C. After the addition was completed, the reaction mixture was stirred for 1 hour. Then, NaBH₃CN (55.5 mg, 883 µmol, 2 *eq*) was added, and the reaction mixture was maintained at 20°C with stirring for 12 hours. LCMS detection showed that Compound 11-4 was nearly completely converted, with the main peak corresponding to the molecular weight of the target product. The mixture was filtered, and the filtrate was concentrated to obtain a residue. The residue was purified by preparative high-performance liquid chromatography (chromatographic column: Xtimate C18 150*40 mm*10 µm; mobile phase: [water (NH₃H₂O+NH₄HCO₃)-acetonitrile]; gradient: 44%-84% B, 36 minutes) to obtain Compound 11 (35.0 mg, 99.8 µmol, yield: 22.6%) as a white solid. MS(ESI) m/z = 351.2 [M+H]⁺.

**¹H NMR:** (400 MHz, DMSO-*d*₆) δ (ppm) = 8.33 (s, 1H), 8.22 - 8.13 (m, 2H), 7.95 (s, 1H), 7.56 - 7.51 (m, 1H), 7.48 - 7.43 (m, 1H), 3.84 (s, 3H), 3.64 (s, 2H), 3.27 - 3.23 (m, 2H), 2.75 (br t, *J* = 5.4 Hz, 2H), 2.41 (s, 3H).

### Example 11

### Synthesis of Compound 12

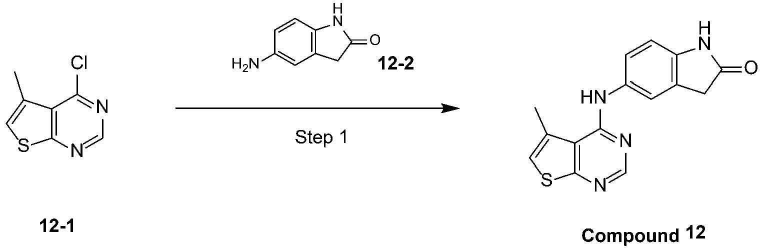

### Step 1: Preparation of Compound 12

Compound 12-1 (150 mg, 812 µmol, 1 *eq*)*,* Compound 12-2 (120 mg, 812 µmol, 1 *eq*)*,* and TFA (9.26 mg, 81.2 µmol, 6.03 µL, 0.1 *eq*) were mixed in an isopropanol (1.0 mL) solution. The reaction mixture was purged three times with nitrogen, heated to 85°C, and stirred under a nitrogen atmosphere for 12 hours. LCMS detection showed that 12-1 was nearly completely converted. The reaction mixture was cooled to room temperature and filtered. The filter cake was suspended in acetonitrile (2.0 mL) and stirred at 25°C for 3 hours. After filtration, the filter cake was dried to obtain Compound 12 (156 mg, 516 µmol, yield: 63.5%) as a white solid. MS(ESI) m/z = 297.1 [M+H]⁺.

**¹H NMR:** (400 MHz, DMSO-*d*₆) δ (ppm) = 10.59 - 10.39 (m, 1H), 8.64 - 8.31 (m, 2H), 7.48 (s, 1H), 7.39 - 7.14 (m, 2H), 6.94 - 6.76 (m, 1H), 3.56 - 3.50 (m, 2H), 2.72 (s, 3H).

### Example 12

### Synthesis of Compound 13

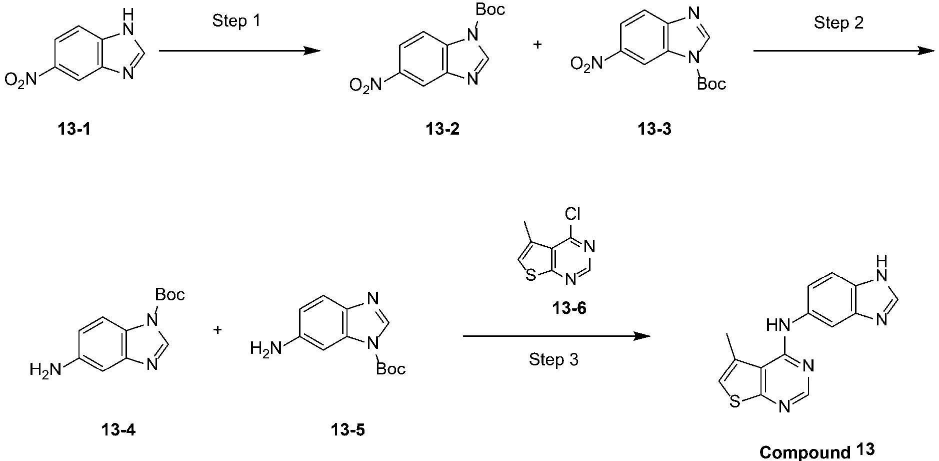

### Step 1: Preparation of Compounds 13-2 & 13-3

Compound 13-1 (5.00, 30.6 mmol, 1 *eq*)*,* triethylamine (9.30 g, 91.9 mmol, 12.80 mL, 3 *eq*), and Boc₂O (10.0 g, 45.9 mmol, 10.56 mL, 1.5 *eq*) were mixed in a DCM (50 mL) solution. The reaction mixture was purged three times with nitrogen and stirred at 20°C under a nitrogen atmosphere for 12 hours. LCMS detection showed that Compound 13-1 was nearly completely converted. The reaction mixture was poured into water and extracted twice with EtOAc (300 mL). The organic phases were combined, washed twice with saturated brine (300 mL), and filtered. The filtrate was concentrated under reduced pressure to obtain a residue. The residue was purified by column chromatography (silica gel) to obtain a mixture of Compound 13-2 and Compound 13-3 (7.20 g, 27.3 mmol, yield: 89.2%) as a white solid. MS(ESI) m/z = 208.0 [M+H]⁺.

**¹H NMR:** (400 MHz, DMSO-*d*₆) δ (ppm) = 9.01 (s, 1H), 8.93 (s, 1H), 8.77 (d, *J* = 2.3 Hz, 1H), 8.62 (d, *J* = 2.3 Hz, 1H), 8.34 (dd, *J* = 2.3, 9.0 Hz, 1H), 8.27 (dd, *J* = 2.4, 8.9 Hz, 1H), 8.14 (d, *J* = 9.0 Hz, 1H), 7.99 (d, *J* = 8.9 Hz, 1H), 1.68 (d, *J* = 4.9 Hz, 9H).

### Step 2: Preparation of Compounds 13-4 & 13-5

A mixture of Compound 13-2 and Compound 13-3 (5.00, 30.6 mmol, *1 eq),* Pd/C (4.04 g, 3.79 mmol, 10% purity, 9.99e-2 *eq),* and triethylamine (3.84 g, 37.9 mmol, 5.29 mL, 1 *eq)* were mixed in a mixed solvent of ethanol (100 mL) and methanol (50 mL). The reaction mixture was purged three times each with nitrogen and hydrogen. The reaction mixture was then heated to 50°C and stirred under a hydrogen atmosphere (50 Psi) for 12 hours. LCMS detection showed that Compounds 13-2 & 13-3 were nearly completely converted. The reaction mixture was filtered under reduced pressure, and the filtrate was concentrated to obtain a residue. The residue was purified by preparative high-performance liquid chromatography (basic conditions, chromatographic column: Phenomenex luna C18 250*50 mm*10 µm; mobile phase: [water (NH₃H₂O+NH₄HCO₃)-acetonitrile]; gradient: 0%-30% B, 20 minutes) to obtain a mixture of Compound 13-4 and Compound 13-5 (1.24 g, 5.32 mmol, yield: 14.0%) as a white solid. MS(ESI) m/z = 178.0 [M+H-56]⁺.

**¹H NMR:** (400 MHz, DMSO-*d*₆) δ (ppm) = 8.25 (s, 1H), 7.46 - 7.27 (m, 1H), 7.21 - 7.06 (m, 1H), 7.14 (d, *J* = 1.9 Hz, 1H), 6.69 - 6.45 (m, 2H), 1.63 (s, 9H).

### Step 3: Preparation of Compound 13

A mixture of Compound 13-4 and Compound 13-5 (5.00, 30.6 mmol, *1 eq*), Compound 13-6 (340 mg, 1.84 mmol, 1 *eq*), and TFA (210 mg, 1.84 mmol, 136 µL, 1 *eq*) were mixed in isopropanol (8.6 mL). The reaction mixture was purged three times with nitrogen, heated to 80°C, and stirred under a nitrogen atmosphere for 12 hours. LCMS detection showed that Compounds 13-4 & 13-5 were nearly completely converted. The reaction mixture was filtered under reduced pressure, and the filtrate was concentrated to obtain a residue. The residue was purified by preparative high-performance liquid chromatography (chromatographic column: Xtimate C18 150*40 mm*10 µm; mobile phase: [water (NH₃H₂O+NH₄HCO₃)-acetonitrile]; gradient: 4%-44% B, 36 minutes) to obtain Compound 13 (314 mg, 1.12 mmol, yield: 60.7%) as a white solid. MS(ESI) m/z = 282.1 [M+H]⁺.

**¹H NMR:** (400 MHz, DMSO-*d*₆) δ (ppm) = 12.40 (br s, 1H), 8.45 - 8.26 (m, 2H), 8.23 - 8.14 (m, 1H), 7.98 (br d, *J* = 18.9 Hz, 1H), 7.65 - 7.49 (m, 1H), 7.45 - 7.24 (m, 2H), 2.76 (s, 3H).

### Example 13

### Synthesis of Compound 14

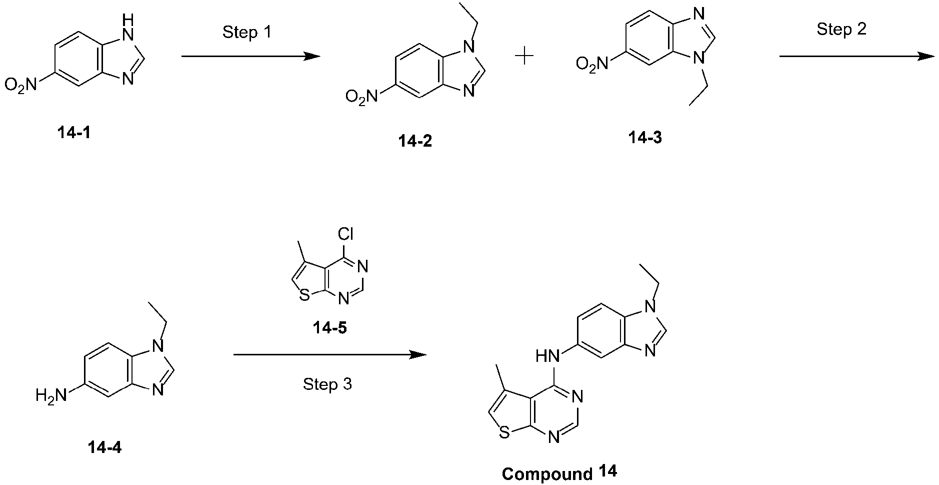

### Step 1: Preparation of Compounds 14-2 & 14-3

Compound 14-1 (5.00 g, 30.6 mmol, 1 *eq)* and iodoethane (5.74 g, 36.8 mmol, 1.2 *eq)* were dissolved in DMF (50 mL), and potassium hydroxide (2.58 g, 46.0 mmol, 1.5 *eq*) was added. The reaction mixture was stirred at 20°C for 12 hours. LCMS detection showed that Compound 14-1 was nearly completely converted. The reaction mixture was filtered, and the filtrate was concentrated under reduced pressure to obtain a residue. The residue was purified by preparative high-performance liquid chromatography (basic conditions, chromatographic column: Welch Xtimate C18 250*50 mm*10 µm; mobile phase: [water (NH₃H₂O+NH₄HCO₃)-acetonitrile]; gradient: 0%-30% B, 25 minutes) to obtain a mixture of Compound 14-2 and Compound 14-3 (5.0 g) as a yellow solid. MS(ESI) m/z = 192.0 [M+H]⁺.

### Step 2: Preparation of Compound 14-4

Compounds 14-2 & 14-3 (3.79 g, 19.8 mmol, 1 *eq*)*,* Pd/C (1.60 g, 1.50 mmol, 10% purity, 7.58e-2 *eq*), and triethylamine (2.01 g, 19.8 mmol, 2.76 mL, 1 *eq*) were dissolved in ethanol (75 mL). The reaction mixture was purged three times each with nitrogen and hydrogen. The reaction mixture was then heated to 50°C and stirred under a hydrogen atmosphere (50 Psi) for 12 hours. LCMS detection showed that Compounds 14-2 & 14-3 were nearly completely converted. The reaction mixture was filtered under reduced pressure, and the filtrate was concentrated to obtain a residue. The residue was purified by supercritical fluid chromatography SFC (neutral conditions, chromatographic column: DAICEL CHIRALPAK AD (250 mm*50 mm, 10 µm); mobile phase: [CO₂-EtOH(0.1% NH₃H₂O)]; B%: 45%, isocratic elution) to obtain Compound 14-4 (1.02 g, 6.30 mmol, yield: 31.8%) as a white solid. MS(ESI) m/z = 162.0 [M+H]⁺.

**¹H NMR:** (400 MHz, DMSO-*d*₆) δ (ppm) = 7.86 (s, 1H), 7.28 (d, *J* = 8.5 Hz, 1H), 6.60 (d, *J* = 2.0 Hz, 1H), 6.52 (dd, *J* = 2.1, 8.6 Hz, 1H), 4.98 (br d, *J* = 4.6 Hz, 2H), 4.08 (q, *J* = 7.3 Hz, 2H), 1.35 (t, *J* = 7.3 Hz, 3H).

### Step 3: Preparation of Compound 14

Compound 14-4 (500 mg, 3.10 mmol, 1 *eq),* Compound 14-5 (572.71 mg, 3.10 mmol, *1 eq),* and TFA (35.4 mg, 310 µmol, 23.0 µL, 0.1 *eq)* were mixed in isopropanol (10 mL). The reaction mixture was purged three times with nitrogen, heated to 80°C, and stirred under a nitrogen atmosphere for 12 hours. LCMS detection showed that Compound 14-4 was nearly completely converted. The reaction mixture was filtered under reduced pressure. The filter cake was suspended in ethanol and stirred for 60 minutes, then filtered under reduced pressure. The filter cake was dried to obtain Compound 14 (239 mg, 772 µmol, yield: 24.9%) as a white solid. MS(ESI) m/z = 310.1 [M+H]⁺.

**¹H NMR:** (400 MHz, DMSO-*d*₆) δ (ppm) = 9.57 (s, 1H), 8.84 - 8.67 (m, 1H), 8.46 (s, 1H), 8.33 (s, 1H), 7.85 (s, 2H), 7.38 (d, *J* = 0.6 Hz, 1H), 4.46 (q, *J* = 7.3 Hz, 2H), 2.78 (s, 3H), 1.55 (t, *J* = 7.3 Hz, 3H).

### Example 14

### Synthesis of Compound 15

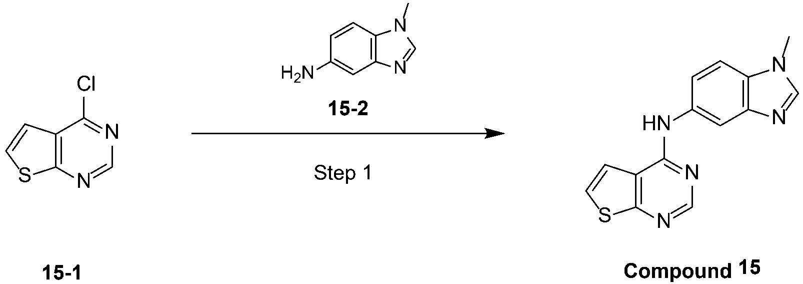

### Step 1: Preparation of Compound 15

Compound 15-1 (200 mg, 1.17 mmol, 1 *eq),* Compound 15-2 (173 mg, 1.17 mmol, 1 *eq),* and TFA (13.4 mg, 117 µmol, 8.71 µL, 0.1 *eq)* were mixed in isopropanol (1 mL). The reaction mixture was purged three times with nitrogen, heated to 85°C, and stirred under a nitrogen atmosphere for 12 hours. LCMS detection showed that Compound 15-1 was nearly completely converted. The reaction mixture was filtered under reduced pressure, and the filtrate was concentrated under reduced pressure to obtain a residue. The residue was purified by preparative high-performance liquid chromatography (neutral conditions, chromatographic column: Xtimate C₁₈ 150*40 mm*10 µm; mobile phase: [water (NH₃H₂O+NH₄HCO₃) - acetonitrile]; gradient: 4% - 44% B, 36 minutes) to obtain Compound 15 (190 mg, 675 µmol, yield: 57.6%) as a white solid. MS(ESI) m/z = 282.1 [M+H]⁺.

**¹H NMR:** (400 MHz, DMSO-*d*₆) δ (ppm) = 10.59 - 10.39 (m, 1H), 8.64 - 8.31 (m, 2H), 7.48 (s, 1H), 7.39 - 7.14 (m, 2H), 6.94 - 6.76 (m, 1H), 3.56 - 3.50 (m, 2H), 2.72 (s, 3H).

### Example 15

### Synthesis of Compound 16

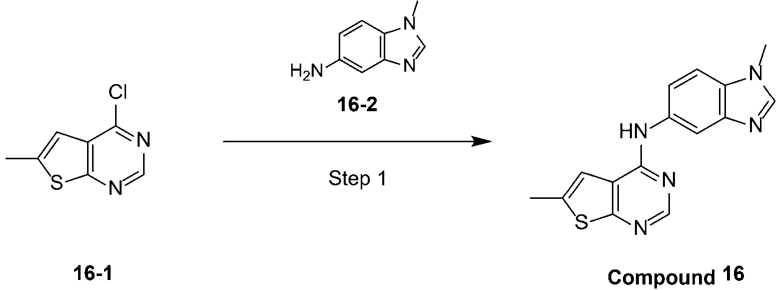

### Step 1: Preparation of Compound 16

Compound 16-1 (200 mg, 1.08 mmol, 1 *eq),* Compound 16-2 (159 mg, 1.08 mmol, 1 *eq),* and TFA (12.4 mg, 108 µmol, 8.05 µL, 0.1 *eq*) were mixed in isopropanol (2 mL). The reaction mixture was purged three times with nitrogen, heated to 85°C, and stirred under a nitrogen atmosphere for 12 hours. LCMS detection showed that Compound 16-1 was nearly completely converted. The reaction mixture was filtered under reduced pressure, and the filtrate was concentrated under reduced pressure to obtain a residue. The residue was purified by preparative high-performance liquid chromatography (neutral conditions, chromatographic column: Xtimate C₁₈ 150*40 mm*10 µm; mobile phase: [water (NH₃H₂O+NH₄HCO₃) - acetonitrile]; gradient: 6% - 46% B, 36 minutes) to obtain Compound 16 (150 mg, 508 µmol, yield: 46.9%) as a white solid. MS(ESI) m/z = 296.0 [M+H]⁺.

**¹H NMR:** (400 MHz, DMSO-*d*₆) δ (ppm) = 9.48 (s, 1H), 8.39 (s, 1H), 8.19 - 8.11 (m, 2H), 7.59 - 7.51 (m, 3H), 3.84 (s, 3H), 2.59 (s, 3H).

### Example 16

### Synthesis of Compound 17

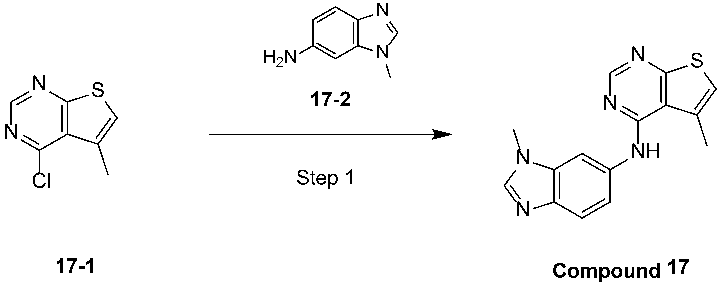

### Step 1: Preparation of Compound 17

Compound 17-1 (289 mg, 1.57 mmol, 1 *eq)* was dissolved in isopropanol (3 mL). Compound 17-2 (231 mg, 1.57 mmol, 1 *eq)* and TFA (17.9 mg, 157 µmol, 11.7 µL, 0.1 *eq)* were added. The reaction mixture was purged three times with nitrogen, heated to 80°C, and stirred under a nitrogen atmosphere for 6 hours. LCMS detection showed that Compound 17-1 was nearly completely converted. The reaction mixture was cooled and quenched with ammonia water, adjusting the pH to 7-8. Solids precipitated out and were collected by filtration under reduced pressure. The filter cake was suspended in acetonitrile (1.0 mL) and stirred at 20°C for 10 minutes. After filtration under reduced pressure, the filter cake was rinsed with water and collected. The filter cake was lyophilized to obtain Compound 17 (100 mg, 338 µmol, yield: 21.5%) as a white solid. MS(ESI) m/z = 296.0 [M+H]⁺.

**¹H NMR:** (400 MHz, DMSO-*d*₆) δ (ppm) = 8.40 (s, 2H), 8.22 (s, 1H), 7.94 (d, *J* = 1.4 Hz, 1H), 7.64 (d, *J* = 8.6 Hz, 1H), 7.43 (dd, *J* = 1.6, 8.6 Hz, 1H), 7.31 (s, 1H), 3.84 (s, 3H), 2.76 (s, 3H).

### Example 17

### Synthesis of Compound 18

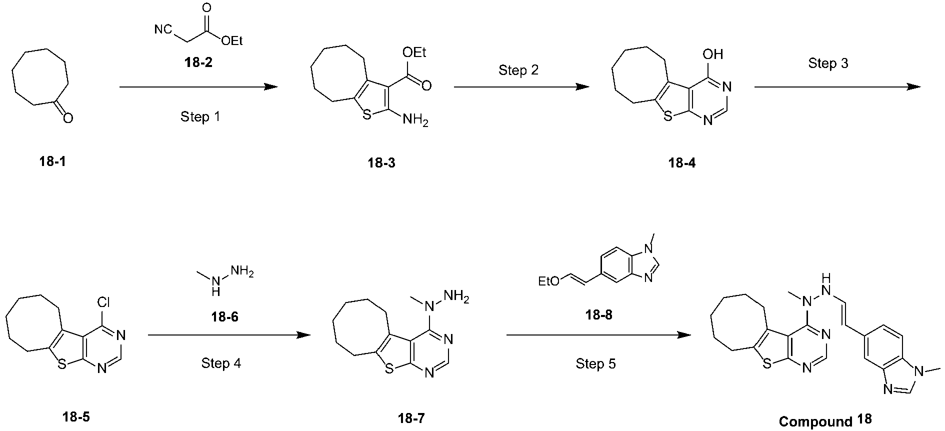

### Step 1: Preparation of Compound 18-3

Compound 18-1 (3.50 g, 16.6 mmol, 1 *eq*), Compound 18-2 (17.9 g, 158 mmol, 16.9 mL, *1 eq*), elemental sulfur (4.92 g, 153 mmol, 9.68e-1 *eq*), and morpholine (55.2 g, 634 mmol, 55.8 mL, 4 *eq*) were mixed in an EtOH (160.0 mL) solution. The reaction mixture was purged three times with nitrogen, heated to 60°C, and stirred under a nitrogen atmosphere for 5 hours. LCMS showed that the starting material 18-1 was nearly completely converted, with the main peak corresponding to the molecular weight of the target product. The reaction was quenched with water (300 mL) and extracted with EtOAc (500 mL). The organic phase was washed with saturated brine (200 mL), dried over anhydrous sodium sulfate, filtered, and concentrated to obtain a residue. The residue was purified by column chromatography (silica gel, petroleum ether/ethyl acetate = 15/1 to 10/1) to obtain Compound 18-3 (27.8 g, 110 mmol, yield: 69.2%) as a yellow oil. MS (ESI) m/z = 254.1 [M+H]⁺.

**¹H NMR:** (400 MHz, DMSO-*d*₆) δ (ppm) = 7.18 (s, 2H), 4.15 (q, *J* = 7.0 Hz, 2H), 2.78 - 2.70 (m, 2H), 2.57 - 2.51 (m, 2H), 2.33 - 2.29 (m, 1H), 1.78 - 1.73 (m, 1H), 1.53 (br d, *J* = 6.0 Hz, 2H), 1.49 - 1.46 (m, 2H), 1.39 (br d, *J* = 4.9 Hz, 2H), 1.25 - 1.22 (m, 3H).

### Step 2: Preparation of Compound 18-4

Compound 18-3 (20.0 g, 78.9 mmol, 1 *eq)* and ammonium formate (200 mL) were mixed. The reaction mixture was purged three times with nitrogen, heated to 135°C, and stirred under a nitrogen atmosphere for 48 hours. LCMS showed that the starting material 18-3 was nearly completely converted, with the main peak corresponding to the molecular weight of the target product. The reaction mixture was filtered, and the filter cake was purified by column chromatography (silica gel, petroleum ether/THF = 5/1 to 2/1) to obtain Compound 18-4 (13.7 g, 58.5 mmol, yield: 74.0%) as a white solid. MS(ESI) m/z = 234.9 [M+H]⁺.

**¹H NMR:** (400 MHz, DMSO-*d*₆) δ (ppm) = 12.31 (br s, 1H), 7.99 (s, 1H), 3.07 - 3.01 (m, 2H), 2.88 - 2.81 (m, 2H), 1.63 - 1.55 (m, 4H), 1.43 - 1.36 (m, 2H), 1.28 - 1.20 (m, 2H).

### Step 3: Preparation of Compound 18-5

POCl₃ (28.6 g, 187 mmol, 17.4 mL, 5 *eq*) was added dropwise to Compound 18-4 (8.74 g, 37.3 mmol, 1 *eq)* at 20°C over a period of 10 minutes or more. After the addition was completed, the reaction mixture was heated to 110°C and stirred for 12 hours. LCMS detection showed that some Compound 18-4 remained, but the main peak corresponded to the target product. The reaction mixture was cooled to 0°C, quenched with water (250 mL), and extracted with EtOAc (250 mL). The organic phase was washed with saturated brine (150 mL), dried over anhydrous sodium sulfate, and filtered. The filtrate was concentrated to obtain a residue, which was purified by column chromatography (silica gel, petroleum ether/ethyl acetate = 10/1 to 5/1) to obtain Compound 18-5 (4.50 g, 17.5 mmol, yield: 46.7%) as a yellow solid. MS(ESI) m/z = 252.8 [M+H]⁺.

**¹H NMR:** (400 MHz, DMSO-*d*₆) δ (ppm) = 8.79 (s, 1H), 3.14 - 3.05 (m, 2H), 3.02 - 2.95 (m, 2H), 1.73 - 1.58 (m, 4H), 1.48 - 1.38 (m, 2H), 1.24 - 1.14 (m, 2H).

### Step 4: Preparation of Compound 18-7

Compound 18-5 (4.30 g, 17.0 mmol, 1 *eq*) was dissolved in EtOH (64.5 mL), and Compound 18-6 (4.67 g, 101 mmol, 5.34 mL, 5.96 *eq*) was added. The reaction mixture was heated to 85°C and stirred for 2 hours. LCMS detection showed that Compound 18-5 was nearly completely converted. The reaction mixture was concentrated to obtain a residue, which was purified by column chromatography (silica gel, dichloromethane/methanol = 20/1 to 10/1) to obtain Compound 18-7 (2.90 g, 11.1 mmol, yield: 64.9%) as an off-white solid. MS(ESI) m/z = 262.9 [M+H]⁺.

**¹H NMR:** (400 MHz, DMSO-*d*₆) δ (ppm) = 8.37 (s, 1H), 4.88 (s, 2H), 3.22 - 3.17 (m, 2H), 3.13 (s, 3H), 2.93 - 2.88 (m, 2H), 1.68 - 1.58 (m, 4H), 1.45 - 1.37 (m, 2H), 1.20 - 1.12 (m, 2H).

### Step 5: Preparation of Compound 18

20 batches of parallel reactions with the same scale: Compound 18-7 (50.0 mg, 190 µmol, 1 *eq*) was dissolved in EtOH (0.5 mL), and Compound 18-8 (57.8 mg, 286 µmol, 1.5 *eq*) and boron trifluoride diethyl etherate (67.6 mg, 476 µmol, 58.6 µL, 2.5 *eq*) were added. The reaction mixture was heated to 85°C and stirred for 3 hours. LCMS detection showed that Compound 18-7 was nearly completely converted. The reaction was cooled to room temperature and quenched with sodium bicarbonate, adjusting the pH to 7-8. The mixture was extracted twice with EtOAc (35 mL), and the organic phases were combined. The organic phase was washed with saturated brine (10 mL), dried over anhydrous sodium sulfate, and filtered. The filtrate was concentrated to obtain a residue, which was purified by preparative high-performance liquid chromatography (chromatographic column: Xtimate C18 150*40 mm*10 µm; mobile phase: [water (NH₄HCO₃)-acetonitrile]; gradient: 36%-76% B, 36 minutes) to obtain Compound 18 (80.0 mg, 183 µmol, yield: 4.79%) as a white solid. MS(ESI) m/z = 419.0 [M+H]⁺.

**¹H NMR:** (400 MHz, DMSO-*d*₆) δ (ppm) = 8.55 (s, 1H), 8.15 (s, 1H), 7.52 (d, *J* = 8.8 Hz, 2H), 7.35 (t, *J* = 5.7 Hz, 1H), 7.16 (dd, *J* = 1.2, 8.3 Hz, 1H), 3.82 (s, 3H), 3.73 (d, *J* = 5.6 Hz, 2H), 3.40 (s, 3H), 3.19 - 3.13 (m, 2H), 2.98 - 2.91 (m, 2H), 1.71 - 1.63 (m, 2H), 1.51 (br s, 2H), 1.43 (br d, *J* = 4.1 Hz, 2H), 1.10 (br d, *J* = 3.6 Hz, 2H).

### Example 18

### Synthesis of Compound 19

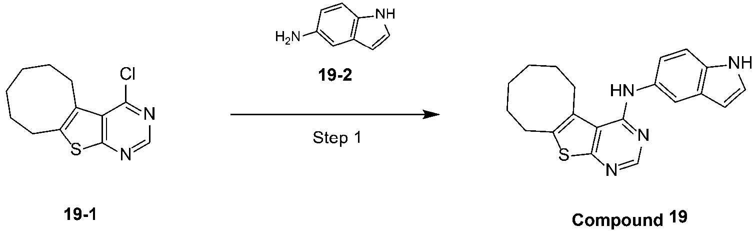

### Step 1: Preparation of Compound 19

Compound 19-1 (100 mg, 395 µmol, 1 *eq),* Compound 19-2 (52.3 mg, 396 µmol, 1 *eq),* and TFA (4.51 mg, 39.6 µmol, 2.94 µL, 0.1 *eq*) were mixed in isopropanol (1.0 mL). The reaction mixture was purged three times with nitrogen, heated to 85°C, and stirred under a nitrogen atmosphere for 12 hours. LCMS detection found that Compound 19-1 was nearly completely converted, with the main peak corresponding to the molecular weight of the target product. The reaction mixture was concentrated to obtain a residue, which was purified by preparative high-performance liquid chromatography (chromatographic column: Xtimate C18 150*40 mm*10 µm; mobile phase: [water (NH₃H₂O+NH₄HCO₃)-acetonitrile]; gradient: 38.0%-78.0% B, 36 minutes) to obtain Compound 19 (50.0 mg, 142 µmol, yield: 35.9%) as a white solid. MS(ESI) m/z = 348.9 [M+H]⁺.

**¹H NMR:** (400 MHz, DMSO-*d*₆) δ (ppm) = 11.07 (br s, 1H), 8.25 (s, 1H), 8.16 (s, 1H), 7.70 (d, *J* = 1.8 Hz, 1H), 7.41 - 7.31 (m, 2H), 7.20 (dd, *J* = 2.0, 8.6 Hz, 1H), 6.42 (d, *J* = 2.0 Hz, 1H), 3.23 - 3.16 (m, 2H), 2.97 - 2.90 (m, 2H), 1.77 - 1.69 (m, 2H), 1.64 (br d, *J* = 3.4 Hz, 2H), 1.50 (br s, 2H), 1.21 (br d, *J* = 4.3 Hz, 2H).

### Example 19

### Synthesis of Compound 20

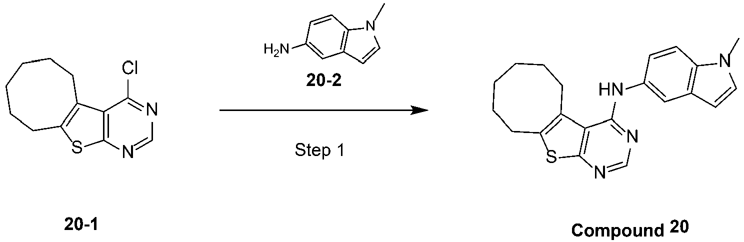

### Step 1: Preparation of Compound 20

Compound 20-1 (100 mg, 395 µmol, 1 *eq),* Compound 20-2 (58.2 mg, 395 µmol, 1 *eq),* and TFA were mixed in an isopropanol (1.0 mL) solution. The reaction mixture was purged three times with nitrogen, heated to 85°C, and stirred under a nitrogen atmosphere for 12 hours. LCMS detection showed that Compound 20-1 was nearly completely converted, with the main peak corresponding to the molecular weight of the target product. The reaction mixture was concentrated under reduced pressure to obtain a residue, which was purified by preparative high-performance liquid chromatography (chromatographic column: Xtimate C18 150*40 mm*10 µm; mobile phase: [water (NH₃H₂O+NH₄HCO₃)-acetonitrile]; gradient: 24.0%-64.0% B, 36 minutes) to obtain Compound 20 (30.0 mg, 82.5 µmol, yield: 20.8%) as a white solid. MS(ESI) m/z = 364.1 [M+H]⁺.

**¹H NMR:** (400 MHz, DMSO-*d*₆) δ (ppm) = 8.35 - 8.23 (m, 2H), 8.17 (s, 1H), 7.88 (d, *J* = 1.1 Hz, 1H), 7.54 (d, *J* = 8.6 Hz, 1H), 7.41 (dd, *J* = 1.6, 8.6 Hz, 1H), 3.85 (s, 3H), 3.22 (br s, 2H), 2.95 (br s, 2H), 1.76 - 1.61 (m, 4H), 1.50 (br s, 2H), 1.19 (br s, 2H).

### Example 20

### Synthesis of Compound 21

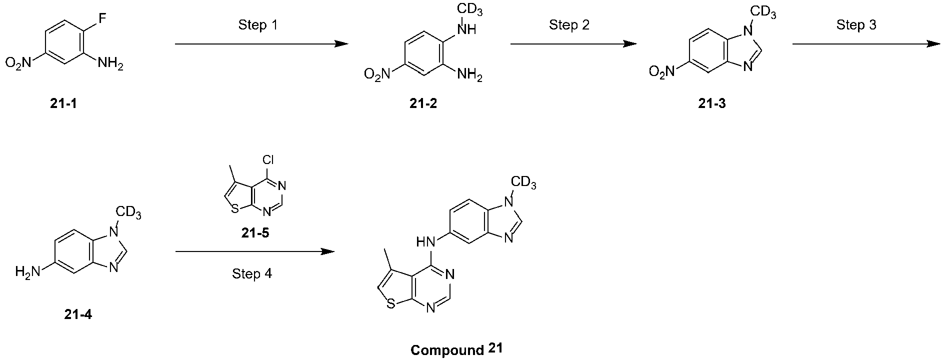

### Step 1: Preparation of Compound 21-2

Compound 21-1 (1.00 g, 6.41 mmol, 1 *eq*), methyl-*d*₃-amine hydrochloride (649 mg, 9.61 mmol, 1.5 *eq),* and diisopropylethylamine (2.48 g, 19.2 mmol, 3.35 mL, 3 *eq)* were mixed in *n*-butanol (10 mL). The reaction mixture was purged three times with nitrogen, heated to 120°C, and stirred under a nitrogen atmosphere for 12 hours. LCMS detection showed that Compound 21-1 was nearly completely converted, with the main peak corresponding to the molecular weight of the target product. The reaction mixture was cooled, and the solvent was removed by concentration under reduced pressure to obtain the crude product of Compound 21-2 as a red solid. The crude product was used directly in the next step without further purification. MS(ESI) m/z = 170.9 [M+H]⁺.

**¹H NMR:** (400 MHz, CDCl₃) δ (ppm) = 7.58 - 7.52 (m, 1H), 7.40 (d, *J* = 2.6 Hz, 1H), 6.45 - 6.39 (m, 1H), 6.08 (s, 1H), 5.10 - 5.02 (m, 2H).

### Step 2: Preparation of Compound 21-3

Compound 21-2 (0.5 g, 2.94 mmol, 1 *eq*), trimethyl orthoformate (1.94 g, 18.2 mmol, 2 mL, 6.21 *eq),* and trifluoroacetic acid (268 mg, 2.35 mmol, 175 µL, 0.8 *eq*) were mixed in ethanol (3 mL). The reaction mixture was purged three times with nitrogen and then stirred at 25°C under a nitrogen atmosphere for 1 hour. LCMS detection showed that Compound 21-2 was nearly completely converted, with the main peak corresponding to the molecular weight of the target product. The reaction mixture was concentrated under reduced pressure to remove the solvent to obtain the crude product of Compound 24-3. The above crude product was suspended in methyl *tert*-butyl ether (30 mL) and stirred at 25°C for 1 hour. The mixture was filtered under reduced pressure, and the filter cake was collected to obtain Compound 21-3 (0.4 g, 2.22 mmol, yield: 75.6%) as a brown solid. MS(ESI) m/z = 170.9 [M+H]⁺.

**¹H NMR:** (400 MHz, DMSO-*d*₆) δ (ppm) = 8.60 - 8.48 (m, 2H), 8.21 (dd, *J* = 8.9, 2.2 Hz, 1H), 7.82 (d, *J* = 8.9 Hz, 1H).

### Step 2: Preparation of Compound 21-4

Compound 21-3 (0.4 g, 2.22 mmol, 1 *eq*) was dissolved in methanol (10 mL). Under a nitrogen atmosphere, wet palladium on carbon (0.2 g, 188 µmol, 10% purity, 0.08 *eq*) and triethylamine (225 mg, 2.22 mmol, 309 µL, 1 *eq*) were added. The above suspension mixture was purged three times with hydrogen. The reaction mixture was then placed under a hydrogen atmosphere (1 MPa), heated to 80°C, and stirred for 12 hours. LCMS detection showed that Compound 21-3 was nearly completely converted, with the main peak corresponding to the molecular weight of the target product. The reaction mixture was filtered under reduced pressure, and the filtrate was collected and concentrated under reduced pressure to obtain the crude product of Compound 21-4 (0.3 g, 1.96 mmol, yield: 88.2%) as a red solid, which was directly used in the next step. MS(ESI) m/z = 150.9 [M+H]⁺.

**¹H NMR:** (400 MHz, DMSO-*d*₆) δ (ppm) = 7.90 (s, 1H), 7.20 (d, *J=* 8.5 Hz, 1H), 6.77 (d, *J=* 1.9 Hz, 1H), 6.61 (dd, *J=* 1.9, 8.5 Hz, 1H), 5.14 - 4.07 (m, 2H).

### Step 2: Preparation of Compound 21

Compound 21-4 (0.2 g, 1.33 mmol, 1 *eq),* Compound 21-5 (246 mg, 1.33 mmol, *1 eq),* and TFA (152 mg, 1.33 mmol, 98.9 µL, 1 *eq)* were mixed in an isopropanol (1.0 mL) solution. The reaction mixture was purged three times with nitrogen, heated to 85°C, and stirred under a nitrogen atmosphere for 8 hours. TLC (petroleum ether/ethyl acetate = 1/1, UV) detection showed that Compound 21-4 was nearly completely converted. The reaction mixture was filtered under reduced pressure, and the collected filter cake was dried to obtain the crude product of Compound 21. The above crude product was suspended in acetonitrile (20 mL) and stirred at 25°C for 8 hours. The mixture was filtered under reduced pressure, and the filter cake was collected and dried under vacuum to obtain Compound 21 (190 mg, 618 µmol, yield: 30.9%) as a white solid. MS(ESI) m/z = 304.9 [M+H]⁺.

**¹H NMR:** (400 MHz, DMSO-*d*₆) δ (ppm) = 9.57 (s, 1H),8.79 (br s, 1H), 8.50 (s, 1H), 8.35 (d, *J=* 1.6 Hz, 1H), 7.96 (d, *J* = 9.0 Hz, 1H), 7.84 (dd, *J* = 9.0, 1.9 Hz, 1H), 7.39 *(d, J=* 1.0 Hz, 1H), 2.78 (d, *J=* 0.9 Hz, 3H).

### Example 21

### Synthesis of Compound 22

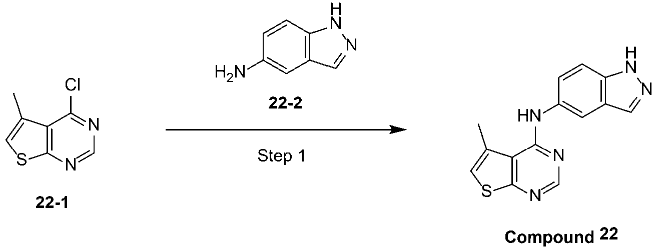

### Step 1: Preparation of Compound 22

Compound 22-1 (277 mg, 1.50 mmol, 1 *eq*)*,* 22-2 (200 mg, 1.50 mmol, *1 eq*), and a solution of HCl (gas) in ethyl acetate (27.4 mg, 751 µmol, 0.50 *eq*) were dissolved in isopropanol (5 mL). The reaction mixture was purged three times with nitrogen, heated to 90°C, and stirred under a nitrogen atmosphere for 12 hours. LCMS monitoring of the reaction showed that Compound 22-2 was nearly completely converted. The reaction mixture was cooled to room temperature and filtered under reduced pressure. The filter cake was collected and suspended in acetonitrile (4 mL), then stirred at room temperature for 1 hour. The mixture was filtered under reduced pressure. The filter cake was purified by column chromatography (silica gel, dichloromethane/methanol = 50/1 to 10/1) to obtain a crude product. This crude product was suspended in acetonitrile (5 mL) and stirred at room temperature for 1 hour. After filtration under reduced pressure, the filter cake was collected and dried under vacuum to obtain Compound 22 (0.125 g, 0.444 mmol, purity: 97.82%, yield: 29.6%) as a brown solid. MS(ESI) m/z = 282.0 [M+H]⁺.

**¹H NMR:** (400 MHz, DMSO-*d*₆) δ (ppm) = 8.76 (brs, 1H), 8.42 (s, 1H), 8.10 (s, 1H), 8.01 (d, *J=* 1.2 Hz, 1H), 7.58 (dd, *J* = 8.8, 1.6 Hz, 1H), 7.51 (dd, *J* = 8.8, 2.0 Hz, 1H), 7.37 (d, *J* = 0.8 Hz, 1H), 2.76 (s, 3H).

### Example 22

### Synthesis of Compound 23

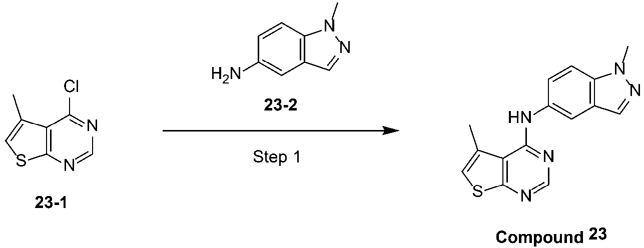

### Step 1: Preparation of Compound 23

Compound 23-1 (251 mg, 1.36 mmol, 1 *eq*), Compound 23-2 (200 mg, 1.36 mmol, 1 *eq),* and a solution of HCl (gas) in ethyl acetate (4 M, 339.73 µL, 1 *eq*) were dissolved in isopropanol (5 mL). The reaction mixture was purged three times with nitrogen, heated to 90°C, and stirred under a nitrogen atmosphere for 12 hours. LCMS monitoring of the reaction showed that the content of the target product was approximately 96%, and TLC (petroleum ether/ethyl acetate = 3/1) monitoring of the reaction showed that Compound 23-1 was nearly completely converted. The reaction mixture was cooled to room temperature, filtered under reduced pressure, and the filter cake was collected and dried under vacuum to obtain Compound 23 (350 mg, 1.15 mmol, purity: 97%, yield: 84.6%) as a gray solid. MS(ESI) m/z = 296.0 [M+H]⁺.

**¹H NMR:** (400 MHz, DMSO-*d*₆) δ (ppm) = 8.69 (brs, 1H), 8.41 (s, 1H), 8.09 - 7.99 (m, 2H), 7.67 (d, *J* = 8.8 Hz, 1H), 7.56 (dd, *J* = 8.8, 2.0 Hz, 1H), 7.36 (d, *J* = 1.0 Hz, 1H), 4.06 (s, 3H), 2.76 (d, *J* = 0.9 Hz, 3H).

### Example 23

### Synthesis of Compound 24

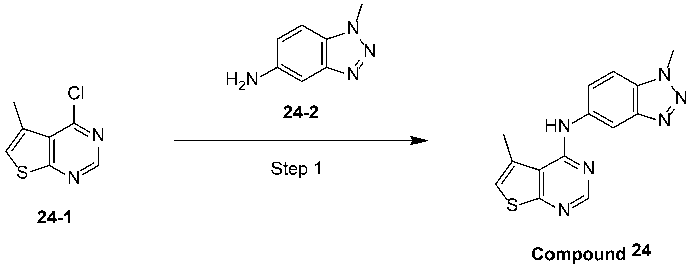

### Step 1: Preparation of Compound 24

Compound 24-1 (251 mg, 1.36 mmol, 1 *eq*), Compound 24-2 (200 mg, 1.36 mmol, 1 *eq*), and a solution of HCl (gas) in ethyl acetate (24.80 mg, 679.45 µmol, 0.5 *eq*) were dissolved in isopropanol (5 mL). The reaction mixture was purged three times with nitrogen, heated to 90°C, and stirred under a nitrogen atmosphere for 12 hours. LCMS monitoring of the reaction showed that Compound 24-2 was nearly completely converted. The reaction mixture was cooled to room temperature and filtered under reduced pressure. The filter cake was collected, suspended in acetonitrile (4 mL), and stirred at room temperature for 1 hour. After filtration under reduced pressure, the filter cake was purified by preparative high-performance liquid chromatography (chromatographic column: Welch Xtimate C18 40*200 mm 7 µm; mobile phase: [water (formic acid)-acetonitrile]; gradient: 2%-42% B, 25 minutes) to obtain Compound 24 (109 mg, 367.8 µmol, purity: 100%, yield: 27.0%) as a brown solid. MS(ESI) m/z = 294.9 [M-H]⁺, 297.0 [M+H]⁺.

**¹H NMR:** (400 MHz, DMSO-*d*₆) δ (ppm) = 9.14 (brs, 1H), 8.57 (s, 1H), 8.34 (d, *J* = 1.2 Hz, 1H), 7.89 (d, *J* = 8.8 Hz, 1H), 7.75 (dd, *J* = 8.8, 1.6 Hz, 1H), 7.44 (s, 1H), 4.33 (s, 3H), 2.79 (s, 3H).

### Example 24

### Synthesis of Compound 25

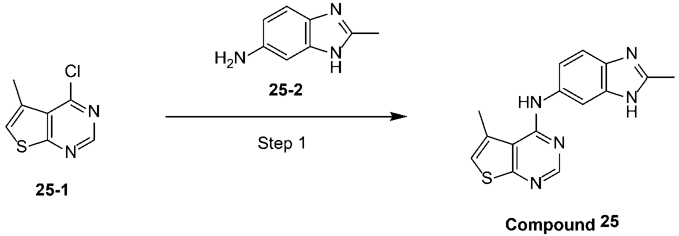

### Step 1: Preparation of Compound 25

Compound 25-2 (200 mg, 1.35 mmol, 1 *eq*), Compound 25-1 (249 mg, 1.35 mmol, 1 *eq),* and a solution of HCl (gas) in ethyl acetate (2 M, 33.8 µL, 0.05 *eq*) were dissolved in isopropanol (5 mL). The reaction mixture was purged three times with nitrogen, heated to 90°C, and stirred under a nitrogen atmosphere for 12 hours. LCMS monitoring of the reaction showed that Compound 25-2 was nearly completely converted. The reaction mixture was cooled to room temperature and filtered under reduced pressure. The filter cake was collected, suspended in acetonitrile (4 mL), and stirred at room temperature for 1 hour. After filtration under reduced pressure, the filter cake was collected and dried under vacuum to obtain Compound 25 (72.4 mg, 0.245 mmol, purity: 98.64%, yield: 18.9%) as an off-white solid. MS(ESI) m/z = 294.0 [M-H]⁺.

**¹H NMR:** (400 MHz, DMSO-*d*₆) δ (ppm) = 8.62 (s, 1H), 8.45 (s, 1H), 8.19 (s, 1H), 7.70 (s, 2H), 7.35 (s, 1H), 2.78 (s, 3H), 2.77 (s, 3H).

### Example 25

### Synthesis of Compound 26

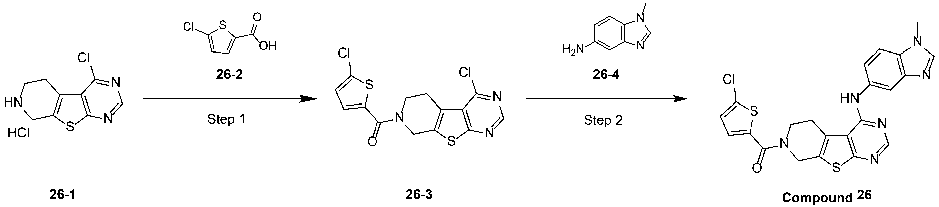

### Step 1: Preparation of Compound 26-3

Compound 26-1 (500 mg, 1.91 mmol, 1 *eq*), HATU (870 mg, 2.29 mmol, 1.2 *eq*), and diisopropylethylamine (740 mg, 5.72 mmol, 997 µL, 3 *eq*) were dissolved in DMF (3 mL). The reaction mixture was stirred at 20°C for 30 minutes. Compound 26-2 (326 mg, 2.00 mmol, 1.05 eq) was added to the reaction mixture. The final reaction mixture was stirred at 25°C for an additional 3.5 hours. LCMS monitoring of the reaction showed that Compound 26-1 was nearly completely converted. Water (20 mL) was added to the reaction mixture, and stirring was continued for 20 minutes. The mixture was filtered under reduced pressure, and the filter cake was collected and dried under vacuum to obtain the crude product of Compound 26-3. This crude product was suspended in a mixed solvent of water/ethyl acetate (50 mL, 10/1) and stirred at 20°C for 12 hours. After filtration under reduced pressure, the filter cake was collected and dried under vacuum to obtain Compound 26-3 (492 mg, 1.33 mmol, yield: 69.7%) as an off-white solid.

**¹H NMR:** (400 MHz, DMSO-*d*₆) δ (ppm) = 8.58 - 8.53 (m, 1H), 7.54 (d, *J* = 4.0 Hz, 1H), 7.24 - 7.19 (m, 1H), 5.17 - 5.08 (m, 2H), 4.13 - 4.05 (m, 2H), 3.43 - 3.36 (m, 2H).

### Step 2: Preparation of Compound 26

Compound 26-3 (250 mg, 675 µmol, 1 *eq*), Compound 26-4 (109 mg, 743 µmol, 1.1 *eq*), and diisopropylethylamine (262 mg, 2.03 mmol, 353 µL, 3 *eq*) were dissolved in DMSO (3 mL). The reaction mixture was purged three times with nitrogen, heated to 120°C, and stirred under a nitrogen atmosphere for 12 hours. LCMS monitoring of the reaction showed that Compound 26-3 was nearly completely converted. The reaction mixture was cooled to room temperature, and water (10 mL) was added to the reaction mixture. The mixture was filtered under reduced pressure, and the filter cake was collected. The filter cake was then dissolved in tetrahydrofuran (10 mL), and the mixture was concentrated under reduced pressure to obtain a residue. The residue was purified by preparative high-performance liquid chromatography (chromatographic column: F-Prepulite XP tC 18 40*200 mm*7 µm; mobile phase: [water (FA)-acetonitrile]; gradient: 2%-42% B, 20.5 minutes) to obtain Compound 26 (102 mg, 209 µmol, yield: 31%) as an off-white solid. MS(ESI) m/z = 481.1 [M-H]⁺.

**¹H NMR:** (400 MHz, DMSO-*d*₆) δ (ppm) = 8.36 (s, 1H), 8.26 (s, 1H), 8.17 (s, 1H), 7.95 (d, *J* = 1.2 Hz, 1H), 7.55 (d, *J* = 8.8 Hz, 1H), 7.50 (d, *J* = 4.0 Hz, 1H), 7.46 (dd, *J* = 8.8, 4.0 Hz, 1H), 7.25 (d, *J* = 4.0 Hz, 1H), 5.01 (brs, 2H), 4.03 - 3.96 (m, 2H), 3.85 (s, 3H), 3.40 (br s, 2H).

### Example 26

### Synthesis of Compound 27

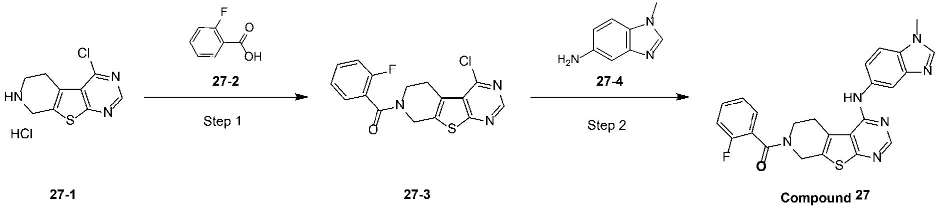

### Step 1: Preparation of Compound 27-3

Compound 27-1 (2.00 g, 7.63 mmol, 1 *eq*), HATU (3.48 g, 9.15 mmol, 1.2 *eq*), and diisopropylethylamine (2.96 g, 22.9 mmol, 3.99 mL, 3 *eq*) were dissolved in DMF (20 mL). The reaction mixture was stirred at 20°C for 30 minutes. Compound 27-2 (1.12 g, 8.01 mmol, 1.05 eq) was added to the reaction mixture. The final reaction mixture was stirred at 20°C for an additional 3.5 hours. LCMS monitoring of the reaction showed that Compound 27-1 was nearly completely converted. Water (20 mL) was added to the reaction mixture, and stirring was continued for 20 minutes. The mixture was filtered under reduced pressure, and the filter cake was collected and dried under vacuum to obtain the crude product of Compound 26-3. This crude product was suspended in a mixed solvent of water/ethyl acetate (100 mL, 10/1) and stirred at 20°C for 12 hours. After filtration under reduced pressure, the filter cake was collected and dried under vacuum to obtain Compound 27-3 (2.26 g, 6.50 mmol, yield: 85.2%) as an off-white solid.

**¹H NMR:** (400 MHz, DMSO-*d*₆) δ (ppm) = 7.82 - 7.74 (m, 2H), 7.67 - 7.59 (m, 2H), 7.58 - 7.47 (m, 3H), 7.46 - 7.31 (m, 2H), 6.91 (br d, *J* = 4.4 Hz, 1H), 3.53 - 3.36 (m, 2H), 3.25 - 3.03 (m, 2H), 2.81 - 2.70 (m, 1H), 2.02 - 1.65 (m, 2H).

### Step 2: Preparation of Compound 27

Compound 27-3 (200 mg, 575 µmol, 1 *eq*)*,* Compound 27-4 (93.1 mg, 633 µmol, 1.1 *eq*), and diisopropylethylamine (223 mg, 1.73 mmol, 301 µL, 3 *eq)* were dissolved in DMSO (2 mL). The reaction mixture was purged three times with nitrogen, heated to 120°C, and stirred under a nitrogen atmosphere for 10 hours. LCMS monitoring of the reaction showed that Compound 27-3 was nearly completely converted. The reaction mixture was cooled to room temperature, and water (10 mL) was added. The mixture was filtered under reduced pressure, and the filter cake was collected. The filter cake was dissolved in tetrahydrofuran (10 mL), dried over anhydrous sodium sulfate, and concentrated under reduced pressure to obtain a residue. The residue was purified by preparative high-performance liquid chromatography (chromatographic column: Welch Xtimate C18 40*200 mm 7 µm; mobile phase: [water (FA)-ACN]; gradient: 0%-38% B, 20 minutes) to obtain Compound 27 (54.9 mg, 120 µmol, yield: 20.8%) as a white solid. MS(ESI) m/z = 459.0 [M-H]⁺.

**¹H NMR:** (400 MHz, DMSO-*d*₆) δ (ppm) = 8.27 (s, 1H), 8.23 - 8.19 (m, 1H), 8.38 - 8.19 (m, 1H), 7.98 - 7.90 (m, 1H), 7.61 - 7.48 (m, 3H), 7.47 - 7.28 (m, 3H), 5.00 (br s, 1H), 4.64 (s, 1H), 4.05 (br s, 1H), 3.88 - 3.83 (m, 3H), 3.62 (br t, *J* = 5.4 Hz, 1H), 3.38 (br s, 1H), 3.26 (br s, 1H).

### Example 27

### Synthesis of Compound 28

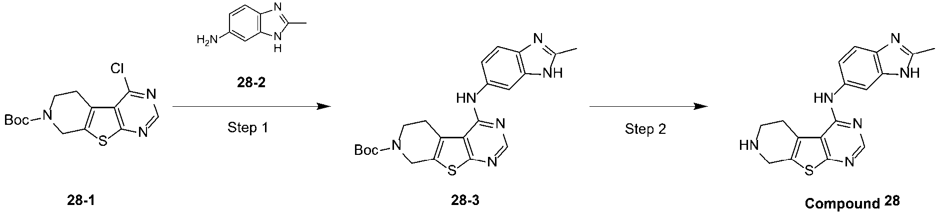

### Step 1: Preparation of Compound 28-3

Compound 28-2 (451 mg, 3.07 mmol, 1 *eq)* and Compound 28-1 (1.00 g, 3.07 mmol, 1.2 *eq)* were dissolved in DMSO (10 mL). Diisopropylethylamine (436 mg, 3.38 mmol, 588 µL, 1.1 *eq)* was added at 25°C. The reaction mixture was heated to 120°C and stirred for 3 hours. LCMS monitoring of the reaction showed that Compound 28-1 was nearly completely converted. The reaction mixture was cooled to room temperature, and water (50 mL) was added to quench the reaction. The mixture was filtered under reduced pressure, and the filter cake was collected and dried under vacuum to obtain the crude product of Compound 28-3. This crude product was suspended in ethyl acetate (30 mL) and stirred at 20°C for 2 hours. The mixture was filtered under reduced pressure, and the filter cake was collected and dried under vacuum to obtain Compound 28-3 (900 mg, 1.86 mmol, yield: 60.4%) as a pale purple solid. MS(ESI) m/z = 437.2 [M+H]⁺.

**¹H NMR:** (400 MHz, DMSO-*d*₆) δ (ppm) = 8.34 (s, 1H), 8.19 (s, 1H), 7.78 (d, *J* = 1.6 Hz, 1H), 7.41 (d, *J* = 8.6 Hz, 1H), 7.28 (dd, *J* = 8.6, 1.6 Hz, 1H), 4.71 - 4.63 (m, 2H), 3.69 (br t, *J* = 5.2 Hz, 2H), 3.23 (br s, 2H), 2.48 (s, 3H), 1.45 (s, 9H).

### Step 2: Preparation of Compound 28

Compound 28-3 (800 mg, 1.83 mmol, 1 *eq*) was dissolved in 1,4-dioxane (2 mL), and a HCl (gas)/dioxane solution (2 M, 9.16 mL, 10 *eq*) was added. The reaction mixture was stirred at 25°C for 1 hour. LCMS monitoring of the reaction showed that Compound 28-3 was nearly completely converted. The reaction mixture was concentrated under reduced pressure to obtain Compound 28 (600 mg, 1.78 mmol, yield: 97.5%) as a purple solid. MS(ESI) m/z = 336.9 [M+H]⁺.

**¹H NMR:** (400 MHz, DMSO-*d*₆) δ (ppm) = 14.99 (br s, 1H), 9.99 (br s, 2H), 8.85 (br s, 1H), 8.48 (s, 1H), 8.12 (s, 1H), 7.77 - 7.66 (m, 2H), 4.47 (br s, 2H), 3.54 - 3.54 (m, 1H), 3.54 - 3.51 (m, 2H), 3.49 - 3.43 (m, 2H), 2.80 (s, 3H).

### Example 28

### Synthesis of Compound 29

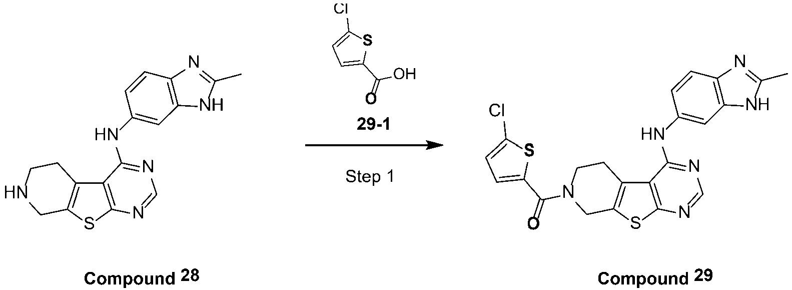

### Step 1: Preparation of Compound 29

Compound 28 (100 mg, 297 µmol, 1.2 *eq*) and Compound 29-1 (39.7 mg, 244 µmol, *1 eq*) were dissolved in DMF (2 mL). Diisopropylethylamine (94.7 mg, 733 µmol, 127 µL, 3 *eq)* and T₄P (264 mg, 366 µmol, 733 µL, 50% purity, 1.5 *eq*) were added. The above reaction mixture was stirred at 20°C for 1.5 hours. LCMS monitoring of the reaction showed that Compound 28 was nearly completely converted. Ammonia water (27%, 2 mL) was added to the reaction mixture to quench the reaction, followed by dilution with water (20 mL). A white solid precipitated and was collected by filtration under reduced pressure. This crude product was suspended in methanol (2 mL) and stirred at 20°C for 0.5 hours. After filtration under reduced pressure, the filter cake was collected and dried under vacuum to obtain Compound 29 (94.5 mg, 191 µmol, yield: 78.2%) as a white solid. MS(ESI) m/z = 480.9 [M+H]⁺.

**¹H NMR:** (400 MHz, DMSO-*d*₆) δ (ppm) = 12.13 (br d, *J =* 6.4 Hz, 1H), 8.35 (br d, *J=* 16.4 Hz, 1H), 8.21 (br d, *J* = 8.4 Hz, 1H), 7.84 - 7.73 (m, 1H), 7.50 (d, *J* = 4.0 Hz, 1H), 7.47 - 7.34 (m, 1H), 7.34 - 7.26 (m, 1H), 7.24 (d, *J* = 4.0 Hz, 1H), 5.00 (br s, 2H), 3.99 (br s, 2H), 3.39 (br s, 2H), 2.48 (br s, 3H).

### Example 29

### Synthesis of Compound 30

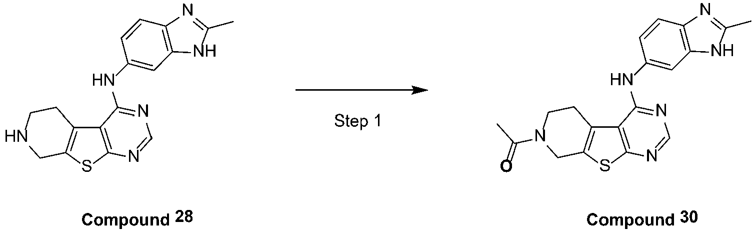

### Step 1: Preparation of Compound 30

Compound 28 (100 mg, 297 µmol, 1 *eq*) was dissolved in acetonitrile (2 mL), and sodium carbonate (47.3 mg, 445 µmol, 1.5 *eq*) and acetic anhydride (33.4 mg, 327 µmol, 30 µL, 1.1 *eq)* were added. The above reaction mixture was stirred at 20°C for 2 hours. LCMS monitoring of the reaction showed that Compound 28 was nearly completely converted. The reaction mixture was concentrated to obtain a crude product. This crude product was suspended in dichloromethane/methanol (5/1, 2 mL) and stirred at 20°C for 0.5 hours. The mixture was filtered under reduced pressure, and the filter cake was collected. The above filter cake was dissolved in methanol/water (1/2, 3 mL) and subjected to lyophilization to obtain Compound 30 (81.1 mg, 210 µmol, yield: 70.6%) as an off-white solid. MS(ESI) m/z = 378.9 [M+H]⁺.

**¹H NMR:** (400 MHz, DMSO-*d*₆) δ (ppm) = 8.34 (s, 1H), 8.09 (br s, 1H), 7.82 (s, 1H), 7.41 (d, *J=* 8.4 Hz, 1H), 7.31 (br d, *J* = 8.4 Hz, 1H), 4.79 (br s, 2H), 3.83 (br t, *J* = 5.6 Hz, 2H), 3.37 - 3.28 (m, 2H), 2.49 - 2.48 (m, 3H), 2.14 (s, 3H).

### Example 30

### Synthesis of Compound 31

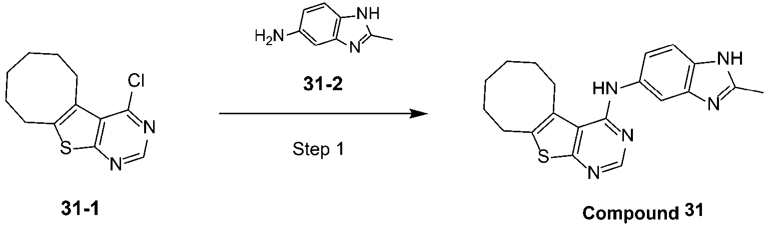

### Step 1: Preparation of Compound 31

Compound 31-1 (250 mg, 0.989 mmol, 1.0 *eq*)*,* Compound 31-2 (146 mg, 0.989 mmol, 1.0 *eq),* and TFA (15 µL, 0.198 mmol, 2.0 *eq*) were mixed in isopropanol (5.0 mL). The reaction mixture was heated to 85°C and stirred for 18 hours. The reaction mixture was cooled to room temperature and filtered under reduced pressure. The filter cake was collected and purified by preparative high-performance liquid chromatography (chromatographic column: YMC-Actus Triart C18 250*20 mm, mobile phase: 10%-95% MeCN in water with 0.1% FA) to obtain Compound 31 (56 mg, 0.154 mmol, yield: 15.5%) as a white solid. MS(ESI) m/z = 364.1 [M-H]⁺.

**¹H NMR:** (400 MHz, DMSO-*d*₆) δ (ppm) = 8.59 (s, 1H), 8.40 (s, 1H), 8.12 (d, *J* = 1.3 Hz, 1H), 7.73 (d, *J* = 8.8 Hz, 1H), 7.64 (dd, *J* = 8.8, 1.8 Hz, 1H), 3.26 - 3.23 (m, 2H), 2.99 - 2.95 (m, 2H), 2.79 (s, 3H), 1.70 - 1.66 (m, 4H), 1.53 - 1.48 (m, 2H), 1.19 - 1.14 (m, 2H).

### Example 31

### Synthesis of Compound 32

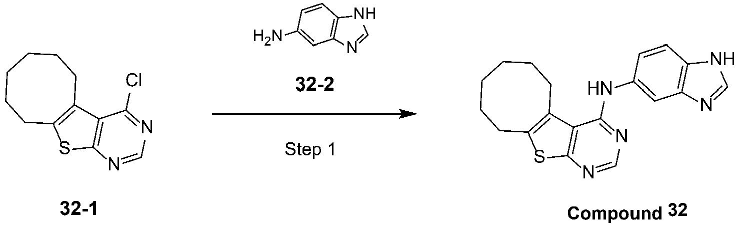

### Step 1: Preparation of Compound 32

Compound 32-1 (300 mg, 1.187 mmol, 1.0 *eq),* Compound 32-2 (158 mg, 1.187 mmol, 1.0 *eq),* and TFA (18 µL, 0.237 mmol, 2.0 *eq)* were mixed in isopropanol (6.0 mL). The reaction mixture was heated to 85°C and stirred for 18 hours. The reaction mixture was cooled to room temperature and filtered under reduced pressure. The filter cake was collected and purified by preparative high-performance liquid chromatography (chromatographic column: YMC-Actus Triart C18 250*20 mm, mobile phase: 10%-95% MeCN in water with 0.1% FA) to obtain Compound 32 (60 mg, 0.172 mmol, yield: 14.5%) as a white solid. MS(ESI) m/z = 350.2 [M-H]⁺.

**¹H NMR:** (400 MHz, DMSO-*d*₆) δ (ppm) = 9.33 (s, 1H), 8.61 (s, 1H), 8.42 (s, 1H), 8.21 (s, 1H), 7.80 (d, *J=* 8.8 Hz, 1H), 7.67 (dd, *J* = 8.8, 1.6 Hz, 1H), 3.27 - 3.23 (m, 2H), 3.00 - 2.95 (m, 2H), 1.70 - 1.64 (m, 4H), 1.53 - 1.48 (m, 2H), 1.19 - 1.15 (m, 2H).

### Example 32

### Synthesis of Compound 33

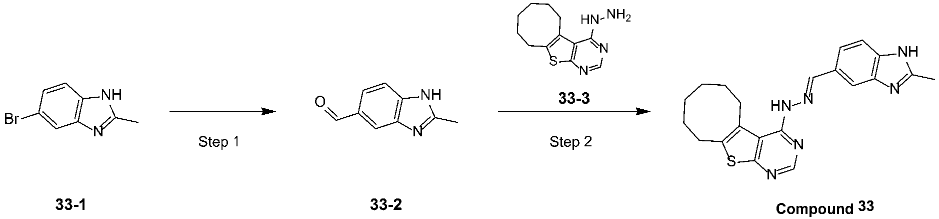

### Step 1: Preparation of Compound 33-2

Compound 33-1 (1.0 g, 4.74 mmol, 1.0 *eq*) was added to a solution of NaH (417 mg, 60% purity, 10.4 mmol, 2.2 *eq*) in tetrahydrofuran (25.0 mL) at 0°C. The reaction mixture was stirred for 30 minutes while maintaining the temperature at 0°C. The above reaction mixture was cooled to -78°C, and n-butyllithium (8.8 mL, 1.6 M in n-hexane, 14.2 mmol, 3.0 *eq)* was added dropwise. After the dropwise addition was completed, the reaction mixture was stirred at -78°C for an additional 1 hour. DMF (926 µL, 11.84 mmol, 2.5 *eq*) was added, and the final reaction mixture was stirred at -78°C for an additional 1 hour. The reaction was quenched with saturated aqueous ammonium chloride solution, then extracted twice with ethyl acetate. The organic phases were combined, dried over anhydrous sodium sulfate, filtered under reduced pressure, and the filtrate was concentrated under reduced pressure to obtain a crude product, which was purified by column chromatography (silica gel, 0-10% methanol in dichloromethane) to obtain Compound 33-2 (368 mg, 2.30 mmol, yield: 48.5%) as a yellow solid. MS(ESI) m/z = 161 [M+H]⁺.

### Step 2: Preparation of Compound 33

Compound 33-3 (150 mg, 0.61 mmol, 1.0 *eq)* was dissolved in EtOH (5.0 mL). At room temperature, Compound 33-2 (126 mg, 0.79 mmol, 1.3 *eq)* and acetic acid (2 drops) were added. The above reaction mixture was heated to 80°C and stirred for 2 hours. The reaction mixture was cooled and concentrated to obtain a residue, which was purified by preparative high-performance liquid chromatography (chromatographic column: YMC-Triart C18, 250*20 mm, 5 µm; mobile phase: 5-95% MeCN in water containing 0.1% NH₃HCO₃) to obtain Compound 33 (58 mg, 0.149 mmol, yield: 24.6%) as a yellow solid. MS(ESI) m/z = 391.1 [M+H]⁺.

**¹H NMR:** (400 MHz, CD₃OD) δ (ppm) = 8.48 (s, 1H), 7.96 (s, 1H), 7.83 (d, *J* = 8.4 Hz, 1H), 7.82 - 7.51 (br, 1H), 7.50 (d, *J* = 8.4 Hz, 1H), 3.28 - 3.20 (m, 2H), 2.97 - 2.85 (m, 2H), 2.59 (s, 3H), 1.82 - 1.67 (m, 4H), 1.58 - 1.50 (m, 2H), 1.37 - 1.31 (m, 2H).

### Example 33

### Synthesis of Compound 34

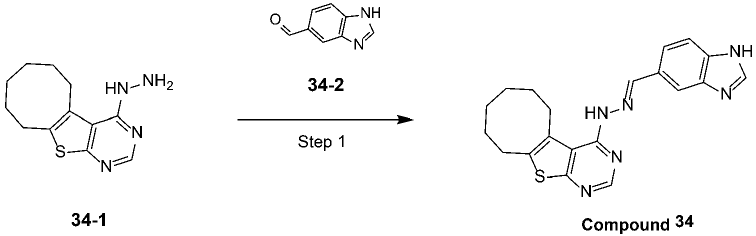

### Step 1: Preparation of Compound 34

Compound 34-1 (100 mg, 0.403 mmol, 1.0 *eq)* was dissolved in MtOH (5.0 mL). Compound 34-2 (159 mg, 0.403 mmol, 1.0 *eq*) was added at room temperature. The above reaction mixture was heated to 30°C and stirred for 12 hours. The reaction mixture was cooled and filtered under reduced pressure. The filter cake was collected and purified by preparative high-performance liquid chromatography (chromatographic column: YMC-Triart C18, 250*20 mm, 5 µm; mobile phase: 5-95% MeCN in water containing 0.1% NH₃HCO₃) to obtain Compound 34 (55 mg, 0.146 mmol, yield: 36.2%) as a yellow solid. MS(ESI) m/z = 377.1 [M+H]⁺.

**¹H NMR:** (400 MHz, DMSO-*d*₆) δ (ppm) = 12.59 (s, 1H), 11.85 (s, 1H), 8.51 (s, 1H), 8.32 - 7.85 (m, 3H), 7.80 (s, 1H), 7.63 (d, *J* = 41.5 Hz, 1H), 3.25 - 3.16 (m, 2H), 2.93 - 2.79 (m, 2H), 1.74 - 1.57 (m, 4H), 1.52 - 1.40 (m, 2H), 1.33 - 1.20 (m, 2H).

### Example 34

### Synthesis of Compound 35

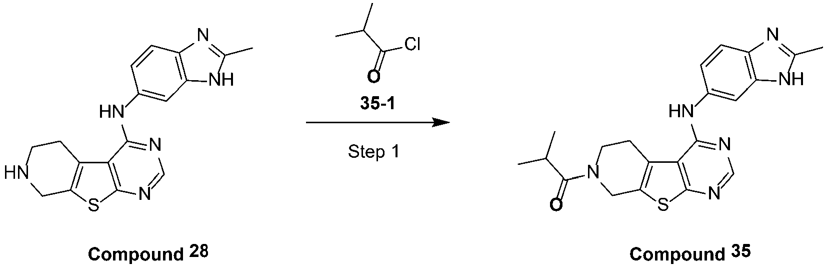

### Step 1: Preparation of Compound 35

The hydrochloride salt of Compound 28 (120 mg, 321 µmol, *1 eq)* and potassium carbonate (133 mg, 965 µmol, 3.0 *eq*) were dissolved in acetonitrile (2 mL), and Compound 35-1 (36.0 mg, 337 µmol, 35.4 µL, 1.05 *eq)* was added. The above reaction mixture was stirred at 25°C for 12 hours. LCMS monitoring of the reaction showed that Compound 28 was nearly completely converted. Methanol (2.0 mL) and water (0.1 mL) were added to the reaction mixture at 25°C, and the mixture was stirred for an additional 2 hours. The above solution was concentrated to obtain a crude product. This crude product was suspended in dichloromethane/methanol (10/1, 2 mL) and filtered under reduced pressure. The filter cake was rinsed twice with dichloromethane (10 mL), and the filtrate was collected and concentrated under reduced pressure to obtain a crude product. This crude product was suspended in methyl *tert*-butyl ether (20 mL) and stirred at 25°C for 30 minutes. After filtration, the filter cake was washed with methyl *tert*-butyl ether (20 mL) and collected. The solid was dried under vacuum. The above solid was then suspended in water (10.0 mL) and stirred at 25°C for 30 minutes. After filtration under reduced pressure, the filter cake was washed with water (10.0 mL). The collected filter cake was dissolved in MeOH (20.0 mL), and the solvent was removed under reduced pressure. The resulting solid was added to water (10.0 mL) to form a pink suspension, which was lyophilized to obtain Compound 35 (50.0 mg, 120 µmol, yield: 37.3%) as a pale pink solid. MS(ESI) m/z = 407.1 [M+H]⁺.

**¹H NMR:** (400 MHz, DMSO-*d*₆) δ (ppm) = 8.37 (s, 1 H), 8.26 (br s, 1 H), 7.85 (s, 1 H), 7.47 (d, *J* = 8.8 Hz, 1 H), 7.35 (br t, *J* = 7.6 Hz, 1 H), 4.74 - 4.94 (m, 2 H), 3.81 - 3.93 (m, 2 H), 3.22 (br d, *J* = 4.2 Hz, 2 H), 2.98 - 3.08 (m, 1 H), 2.53 (s, 3 H), 1.00 - 1.11 (m, 6 H).

### Example 35

### Synthesis of Compound 36

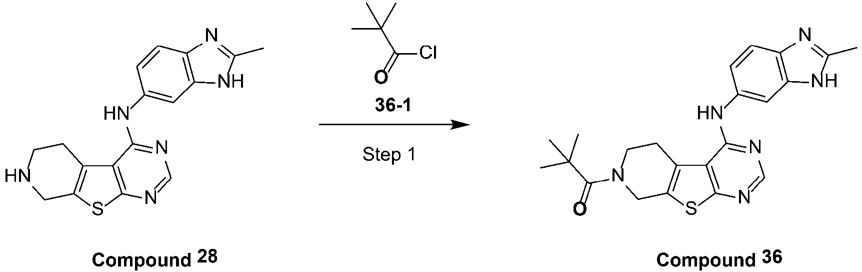

### Step 1: Preparation of Compound 36

The hydrochloride salt of Compound 28 (120 mg, 321 µmol, 1.0 *eq*) and potassium carbonate (133 mg, 965 µmol, 3.0 *eq*) were dissolved in acetonitrile (2 mL), and Compound 36-1 (116 mg, 965 µmol, 118 µL, 3.0 *eq*) was added. The above reaction mixture was stirred at 25°C for 12 hours. LCMS monitoring of the reaction showed that Compound 28 was nearly completely converted. Methanol (2.0 mL) and water (0.1 mL) were added to the reaction mixture at 25°C, and the mixture was stirred for an additional 2 hours. The above solution was concentrated to obtain a crude product. This crude product was suspended in dichloromethane/methanol (10/1, 10 mL), filtered under reduced pressure, and the filter cake was rinsed twice with dichloromethane (10 mL). The filtrate was collected and concentrated under reduced pressure to obtain a crude product. This crude product was suspended in methyl *tert-*butyl ether (20 mL) and stirred at 25°C for 30 minutes. The filtered filter cake was washed with methyl *tert*-butyl ether (20 mL), and the filter cake was collected. The solid was dried under vacuum, then suspended in water (10.0 mL) and stirred at 25°C for 30 minutes. After filtration under reduced pressure, the filter cake was washed with water (10.0 mL) and collected. The filter cake was added to methanol/water (1/3, 20.0 mL) to form a pink suspension, which was lyophilized to obtain a crude product. This crude product was suspended in water/ethyl acetate (1/1, 50.0 mL) and stirred at 25°C for 30 minutes. After filtration under reduced pressure, the filter cake was washed with ethyl acetate (20 mL) and collected. The filter cake was dried under vacuum to obtain Compound 36 (50.0 mg, 113 µmol, yield: 35.3%) as a pale pink solid. MS(ESI) m/z = 421.2 [M+H]⁺.

**¹H NMR:** (400 MHz, DMSO-*d*₆) δ (ppm) = 12.12 (br d, *J* = 1.6 Hz, 1 H), 8.29 - 8.40 (m, 1 H), 8.20 (br s, 1 H), 7.72 - 7.84 (m, 1 H), 7.22 - 7.48 (m, 2 H), 4.85 (br s, 2 H), 3.91 (br t, *J* = 5.2 Hz, 2 H), 3.28 (br s, 2 H), 2.48 (s, 3 H), 1.27 (s, 9 H).

### Example 36

### Synthesis of Compound 37

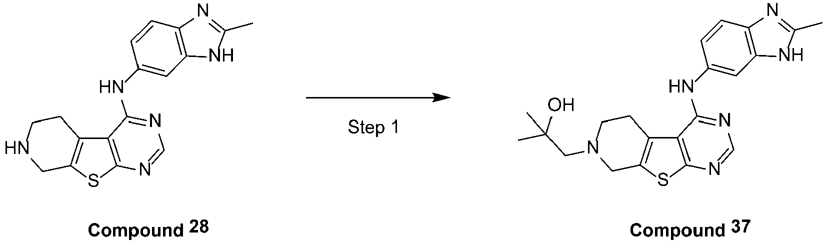

### Step 1: Preparation of Compound 37

The hydrochloride salt of Compound 28 (200 mg, 488 µmol, 1.0 *eq*) and 2,2-dimethyloxirane (141 mg, 1.95 mmol, 173 µL, 4.0 *eq*) were dissolved in NMP (3 mL). The reaction mixture was heated to 80°C under a nitrogen atmosphere and stirred for 12 hours. LCMS monitoring of the reaction showed that Compound 28 was nearly completely converted. The above reaction mixture was cooled to room temperature and filtered under reduced pressure. The filtrate was collected and purified by preparative high-performance liquid chromatography (chromatographic column: F-Prepulite XP tC 18 40*200 mm*7 µm; mobile phase: [water (NH₃H₂O+NH₄HCO₃)-ACN]; gradient: 6%-46% B, 20 minutes) to obtain Compound 37 (90.1 mg, 210 µmol, yield: 43.1%) as a white solid. MS(ESI) m/z = 409.0 [M+H]⁺.

**¹H NMR:** (400 MHz, DMSO-*d*₆) δ (ppm) = 12.14 (br s, 1H), 8.33 (s, 1H), 8.10 (s, 1H), 7.81 (s, 1H), 7.41 (br d, *J* = 8.4 Hz, 1H), 7.28 (br d, *J* = 8.0 Hz, 1H), 4.25 (s, 1H), 3.86 (s, 2H), 3.22 (br s, 2H), 3.02 - 2.89 (m, 2H), 2.48 (s, 3H), 2.45 (s, 2H), 1.15 (s, 6H).

### Example 37

### Synthesis of Compound 38

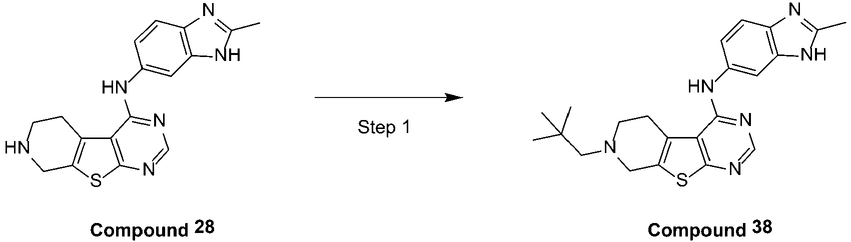

### Step 1: Preparation of Compound 38

The hydrochloride salt of Compound 28 (300 mg, 733 µmol, 1.0 *eq)* and 2,2-dimethylpropanal (63.1 mg, 733 µmol, 81.0 µL, 1.0 *eq)* were dissolved in methanol (5 mL). Sodium acetate (132 mg, 1.61 mmol, 2.2 *eq)* and sodium cyanoborohydride (101 mg, 1.61 mmol, 2.2 *eq)* were added. The above reaction mixture was heated to 50°C and stirred for 2 hours. TLC (dichloromethane/methanol = 10/1, UV) monitoring of the reaction showed that Compound 28 was nearly completely converted. The above reaction mixture was cooled to room temperature and filtered under reduced pressure. The filtrate was collected and purified by column chromatography (silica gel, dichloromethane/methanol = 10/1) to obtain Compound 38 (50.6 mg, 123 µmol, yield: 16.8%) as a white solid. MS(ESI) m/z = 407.2 [M+H]⁺.

**¹H NMR:** (400 MHz, DMSO-*d*₆) δ (ppm) = 12.14 (br s, 1H), 8.33 (s, 1H), 8.12 (s, 1H), 7.81 (br s, 1H), 7.40 (br d, *J* = 6.8 Hz, 1H), 7.28 (br d, *J* = 8.0 Hz, 1H), 3.82 (s, 2H), 3.23 (br s, 2H), 2.90 (br t, *J* = 5.6 Hz, 2H), 2.47 (s, 3H), 2.30 (s, 2H), 0.91 (s, 9H).

### Example 38

### Synthesis of Compound 39

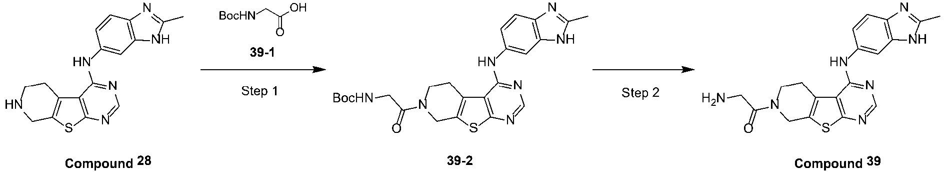

### Step 1: Preparation of Compound 39-2

The hydrochloride salt of Compound 28 (105 mg, 256 µmol, 1.0 *eq),* Compound 39-1 (53.9 mg, 308 µmol, 1.2 *eq),* and Compound T4P (50% in EtOAc, 92.4 mg, 256.51 µmol, 1.0 *eq)* were dissolved in DMF (2 mL). Diisopropylethylamine (33.1 mg, 256 µmol, 44.7 µL, 1.0 *eq)* was added. The above reaction mixture was stirred at 20°C for 2 hours. LCMS monitoring of the reaction showed that Compound 28 was nearly completely converted. The above reaction mixture was poured into water (10 mL), resulting in the precipitation of a large amount of solid. The mixture was filtered under reduced pressure, and the filter cake was collected. After drying under vacuum, the crude product of Compound 39-2 (120 mg) was obtained as a white solid and used directly in the next step without further purification. MS(ESI) m/z = 494.0 [M+H]⁺.

**¹H NMR:** (400 MHz, DMSO-*d*₆) δ (ppm) = 7.63 - 7.37 (m, 1H), 7.19 - 6.91 (m, 1H), 6.85 - 6.56 (m, 1H), 6.56 - 6.29 (m, 1H), 4.05 - 4.04 (m, 2H), 3.91 - 3.67 (m, 1H), 3.48 - 2.89 (m, 5H), 2.48 - 2.46 (m, 2H), 1.86 - 1.65 (m, 3H), 0.65 (br s, 9H).

### Step 2: Preparation of Compound 39

Compound 39-1 (100 mg, 176.52 µmol, 1.0 *eq*) was dissolved in methanol (0.5 mL), and a solution of HCl (gas) in dioxane (2 M, 435.64 µL, 4.94 *eq*) was added. The above reaction mixture was stirred at 20°C for 2 hours. LCMS monitoring of the reaction showed that Compound 39-1 was nearly completely converted. The reaction mixture was concentrated to obtain a crude product, which was diluted with methanol (10 mL). The pH was adjusted to approximately 8.0 using a 10% aqueous sodium hydroxide solution, yielding a clear brown solution. The solution was purified by preparative high-performance liquid chromatography (chromatographic column: F-Prepulite XP tC 18 40*200 mm*7 µm; mobile phase: [water (NH₃H₂O+NH₄HCO₃)-ACN]; gradient: 0%-32% B, 20 minutes) to obtain Compound 39 (50.6 mg, 123 µmol, yield: 34.9%) as a white solid. MS(ESI) m/z = 394.1 [M+H]⁺.

**¹H NMR:** (400 MHz, DMSO-*d*₆) δ (ppm) = 12.12 (br s, 1H), 8.34 (br d, *J* = 10.4 Hz, 1H), 8.27 - 8.09 (m, 1H), 7.88 - 7.65 (m, 1H), 7.57 - 7.16 (m, 2H), 4.83 - 4.72 (m, 2H), 4.05 - 3.66 (m, 3H), 3.58 - 3.39 (m, 3H), 3.23 (br d, *J* = 2.0 Hz, 2H), 2.48 (s, 3H).

### Example 39

### Synthesis of Compound 40

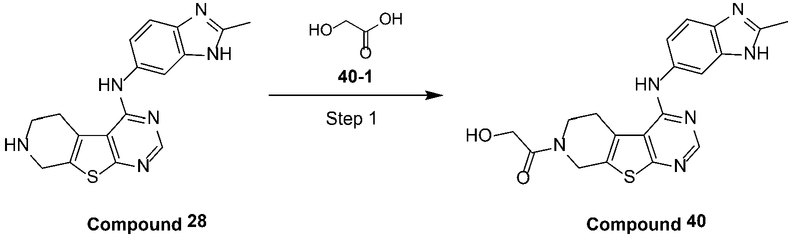

### Step 1: Preparation of Compound 40

The hydrochloride salt of Compound 28 (200 mg, 488 µmol, 1.0 *eq),* glycolic acid (44.6 mg, 586 µmol, 35.7 µL, 1.2 *eq),* and diisopropylethylamine (126 mg, 977 µmol, 170 µL, 2.0 *eq)* were dissolved in ethanol (2 mL). DMTMM (173 mg, 586 µmol, 1.2 *eq)* was added at 20°C, and the reaction mixture was stirred for 2 hours. LCMS monitoring of the reaction showed that Compound 28 was nearly completely converted. The above reaction mixture was concentrated under reduced pressure to obtain a residue, which was purified by preparative high-performance liquid chromatography (chromatographic column: F-Prepulite XP tC 18 40*200 mm*7 µm; mobile phase: [water (NH₃H₂O+NH₄HCO₃)-ACN]; gradient: 0%-34% B, 20 minutes) to obtain Compound 40 (41.0 mg, 99.6 µmol, yield: 20.3%) as a white solid. MS(ESI) m/z = 395.2 [M+H]⁺.

**¹H NMR:** (400 MHz, DMSO-*d*₆) δ (ppm) = 8.34 (d, *J* = 17.2 Hz, 1H), 8.27 - 8.13 (m, 1H), 7.85 - 7.69 (m, 1H), 7.49 - 7.34 (m, 1H), 7.32 - 7.18 (m, 1H), 4.87 (br t, *J* = 5.4 Hz, 1H), 4.83 - 4.63 (m, 2H), 4.33 - 4.13 (m, 2H), 3.93 - 3.67 (m, 2H), 3.24 (br d, *J=* 1.2 Hz, 2H), 2.47 (d, *J=* 1.6 Hz, 3H).

The compounds synthesized in the examples are summarized in Table 1 below.

**Table 1. Example Compound Information**

| Compound No. | Structural Formula | Compound Name |
|---|---|---|
| **Compound 1 Du101-1** | 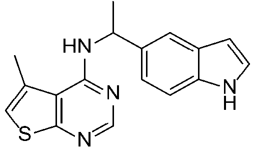 | *N*-(1-(1*H*-Indol-5-yl)ethyl)-5-methylthieno[2,3-*d*]pyrimidin-4-amine |
| **Compound 2 Du101-2** | 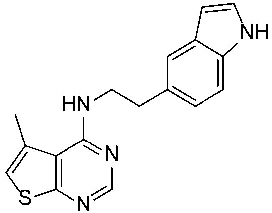 | *N*-(2-(1*H*-Indol-5-yl)ethyl)-5-methylthieno[2,3-*d*]pyrimidin-4-amine |
| **Compound 3 Du101-3** | 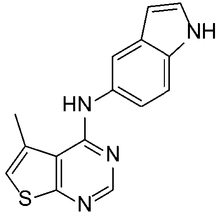 | *N*-(1*H*-Indol-5-yl)-methylthieno[2,3-*d*]pyrimidin-4-amine |
| **Compound 4 Du101-4** | 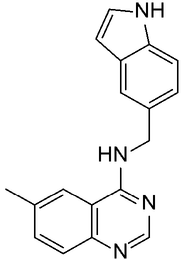 | *N*-((1*H*-Indol-5-yl)methyl)-6-methylquinazolin-4-amine |
| **Compound 5 Du101-5** | 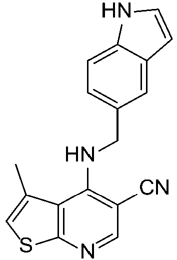 | 4-(((1*H*-Indol-5-yl)methyl)amino)-3-methylthieno[2,3-*d*]-5-carbonitrile |
| **Compound 6 Du101-6** | 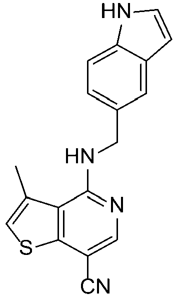 | 4-(((1*H*-Indol-5-yl)methyl)amino)-3-3-methylthieno[2,3-*c*]-7-carbonitrile |
| **Compound 7 Du101-7** | 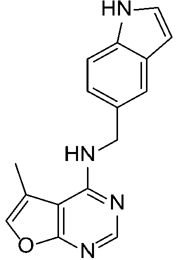 | *N*-(3-Methylthieno[2,3-*d*]-5-cyano)-5-methylfuro[2,3-*d*]pyrimidin-4-amine |
| **Compound 8 Du101-8** | 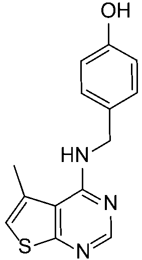 | 4-(((5-Methylthieno[2,3-*d*]pyrimidin-4-yl)aminomethyl)phenol |
| **Compound 9 Du101-10** | 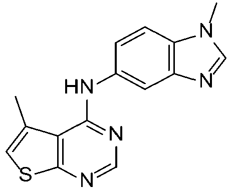 | 5-Methyl-*N*-(1-methyl-1*H-*benzo[*d*]imidazol-5-yl)thieno[2,3-*d*]pyrimidin-4-amine |
| **Compound 10 Du101-11** | 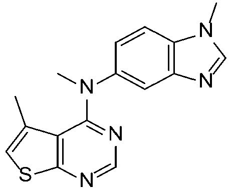 | *N*,5-Dimethyl-*N*-(1-methyl-1*H-*benzo[*d*]imidazol-5-yl)thieno[2,3-*d*]pyrimidin-4-amine |
| **Compound 11 Du101-12** | 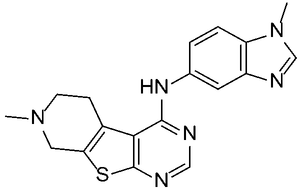 | 7-Methyl-*N*-(1-methyl-1*H-*benzo[*d*]imidazol-5-yl)-5,6,7,8-tetrahydropyrrolo[4',3':4,5]thieno[2,3-*d*]pyrimidin-4-amine |
| **Compound 12 Du101-13** | 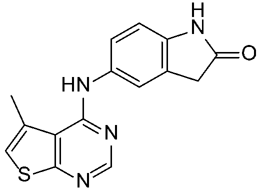 | 5-((5-Methylthieno[2,3-*d*]pyrimidin-4-yl)amino)indolone |
| **Compound 13 Du101-14** | 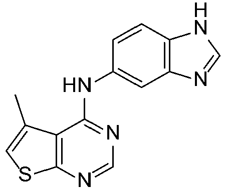 | *N*-(1*H*-Benzo[*d*]imidazol-5-yl)-5-methylthieno[2,3-*d*]pyrimidin-4-amine |
| **Compound 14 Du101-20** | 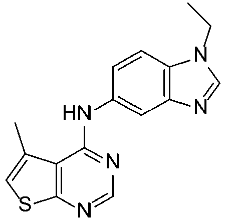 | *N*-(1-Ethyl-1*H*-benzo[*d*]imidazol-5-yl)-5-methylthieno[2,3-*d*]pyrimidin-4-amine |
| **Compound 15 Du101-16** | 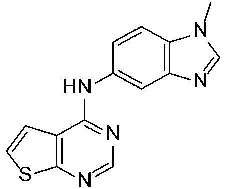 | N-(1-Methyl-1*H*-benzo[d]imidazol-5-yl)thieno[2,3-*d*]pyrimidin-4-amine |
| **Compound 16 Du101-17** | 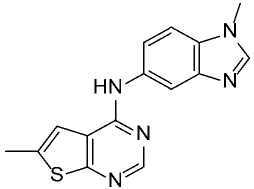 | 6-Methyl-*N*-(1-methyl-1*H-*benzo[*d*]imidazol-5-yl)thieno[2,3-*d*]pyrimidin-4-amine |
| **Compound 17 Du101-19** | 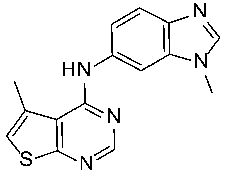 | 5-Methyl-*N*-(1-methyl-1*H-*benzo[*d*]imidazol-6-yl)thieno[2,3-*d*]pyrimidin-4-amine |
| **Compound 18 Du102-2** | 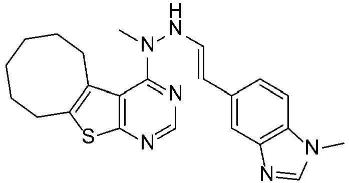 | (*E*)-4-(1-Methyl-2-(2-(1-methyl-1*H-*benzo[*d*]imidazol-5-yl)vinyl)hydrazinyl)-5,6,7,8,9,10-hexahydrocycloocta[4,5]thieno[2,3-*d*]pyrimidine |
| **Compound 19 Du102-3** | 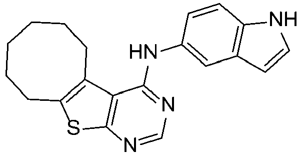 | *N*-(1*H*-indol-5-yl)-5,6,7,8.,9,10-hexahydrocycloocta[4,5]thieno[2,3-*d*]pyrimidin-4-amine |
| **Compound 20 Du102-4** | 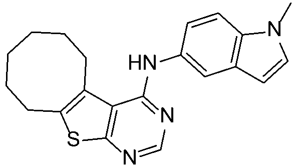 | *N*-(1-Methyl-1H-indol-5-yl)-5,6,7,8,9,10-hexahydrocycloocta[4,5]thieno[2,3-*d*]pyrimidin-4-amine |
| **Compound 21** | 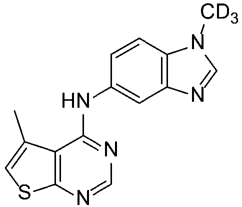 | 5-Methyl-*N*-(1-(methyl-*d*₃)-1*H-*benzo[*d*]imidazol-5-yl)thieno[2,3-*d*]pyrimidin-4-amine |
| **Compound 22** | 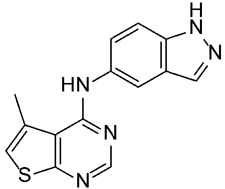 | *N*-(1*H*-Indazol-5-yl)-5-methylthieno[2,3-*d*]pyrimidin-4-amine |
| **Compound 23** | 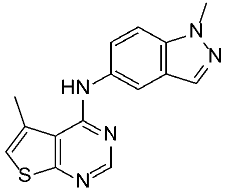 | 5-Methyl-*N-*(1-methyl-1*H* indazol-5-yl)thieno[2,3-*d*]pyrimidin-4-amine |
| **Compound 24** | 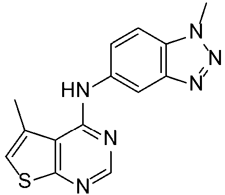 | 5-Methyl-*N*-(1-methyl-1*H-*benzo[*d*][1,2,3]triazol-5-yl)thieno[2,3-*d*]pyrimidin-4-amine |
| **Compound 25** | 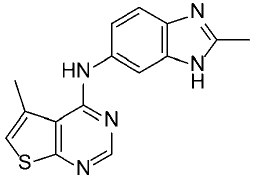 | 5-Methyl-*N*-(2-methyl-1*H-*benzo[*d*]imidazol-6-yl)thieno[2,3-*d*]pyrimidin-4-amine |
| **Compound 26** | 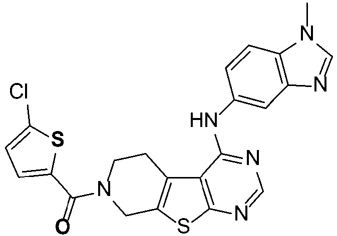 | (5-Chlorothiophen-2-yl)(4-((1-methyl-1*H*-benzo[*d*]imidazol-5-yl)amino)-5,8-dihydropyrido[4',3':4,5]thieno[2,3-*d*]pyrimidin-7(6*H*)-yl)methanone |
| **Compound 27** | 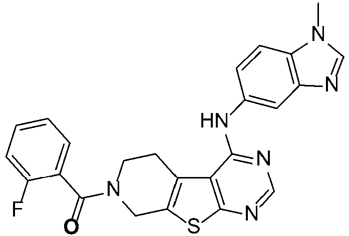 | (5-Fluorophenyl)(4-((1-methyl-1*H-*benzo[*d*]imidazol-5-yl)amino)-5,8-dihydropyrido[4',3':4,5]thieno[2,3-*d*]pyrimidin-7(6*H*)-yl)methanone |
| **Compound 28** | 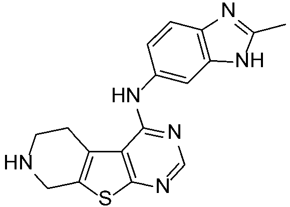 | *N*-(2-Methyl-1*H*-benzo[*d*]imidazol-6-yl)-5,6,7,8-tetrahydropyrido[4',3':4,5]thieno[2,3-*d*]pyrimidin-4-amine |
| **Compound 29** | 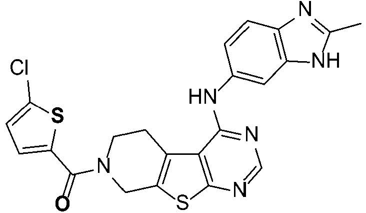 | (5-Chlorothiophen-2-yl)(4-((2-methyl-1*H*-benzo[*d*]imidazol-6-yl)amino)-5,8-dihydropyrido[4',3':4,5]thieno[2,3-d]pyrimidin-7(6*H*)-yl)methanone |
| **Compound 30** | 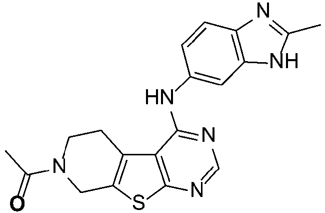 | 1-(4-((2-Methyl-1*H*-benzo[*d*]imidazol-6-yl)amino)-5,8-dihydropyrido[4',3':4,5]thieno[2,3-d]pyrimidin-7(6*H*)-yl)ethanone |
| **Compound 31** | 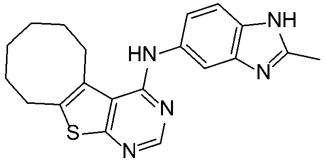 | *N*-(2-Methyl-1*H*-benzo[*d*]imidazol-5-yl)-5,6,7,8,9,10-hexahydrocycloocta[4,5]thieno[2,3-*d*]pyrimidin-4-amine |
| **Compound 32** | 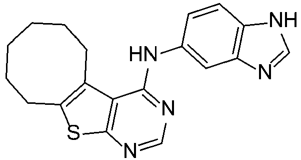 | *N*-(1*H*-Benzo[*d*]imidazol-5-yl)-5,6,7,8,9,10-hexahydrocycloocta[4,5]thieno[2,3-*d*]pyrimidin-4-amine |
| **Compound 33** | 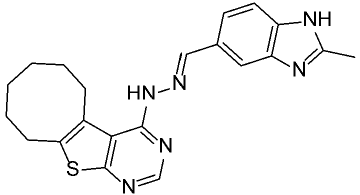 | (*E*)-4-(2-((2-Methyl-1H-benzo[*d*]imidazol-5-yl)methylene)hydrazinyl)-5,6,7,8,9,10-hexahydrocycloocta[4,5]thieno[2,3-*d*]pyrimidine |
| **Compound 34** | 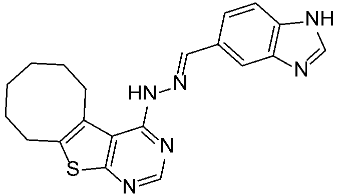 | (*E*)-4-(2-((1*H*-benzo[*d*]imidazol-5-yl)methylene)hydrazinyl)-5,6,7,8,9,10-hexahydrocycloocta[4,5]thieno[2,3-*d*]pyrimidine |
| **Compound 35** | 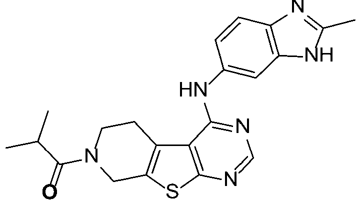 | 2-Methyl-1-(4-((2-methyl-1*H-*benzo[*d*]imidazol-6-yl)amino)-5,8-dihydropyrrolo[4',3':4,5]thieno[2,3-d]pyrimidin-7(6*H*)-yl)propan-1-one |
| **Compound 36** | 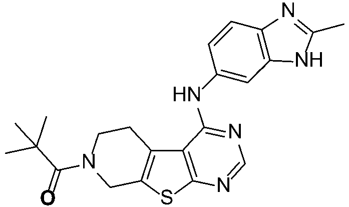 | 2,2-Dimethyl-1-(4-((2-methyl-1*H-*benzo[*d*]imidazol-6-yl)amino)-5,8-dihydropyrrolo[4',3':4,5]thieno[2,3-*d*]pyrimidin-7(6*H*)-yl)propan-1-one |
| **Compound 37** | 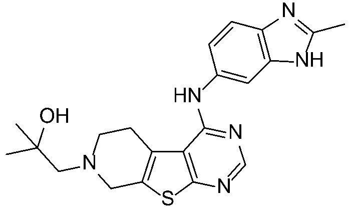 | 2-Methyl-1-(4-((2-methyl-1*H-*benzo[*d*]imidazol-6-yl)amino)-5,8-dihydropyrrolo[4',3':4,5]thieno[2,3-*d*]pyrimidin-7(6*H*)-yl)propan-1-ol |
| **Compound 38** | 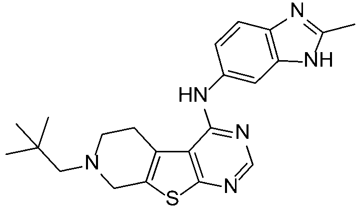 | *N*-(2-Methyl-1*H*-benzo[*d*]imidazol-6-yl)-7-neopentyl-5,6,7,8-tetrahydropyrrolo[4',3':4,5]thieno[2,3-*d*]pyrimidin-4-amine |
| **Compound 39** | 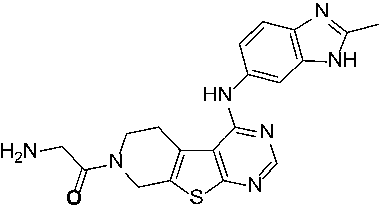 | 2-Amino-1-(4-((2-methyl-1*H-*benzo[*d*]imidazol-6-yl)amino)-5,8-dihydropyrrolo[4',3':4,5]thieno[2,3-*d*]pyrimidin-7(6*H*)-yl)ethanone |
| **Compound 40** | 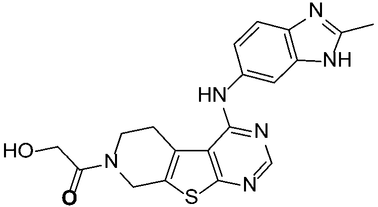 | 2-Hydroxy-1-(4-((2-methyl-1*H-*benzo[*d*]imidazol-6-yl)amino)-5,8-dihydropyrrolo[4',3':4,5]thieno[2,3-*d*]pyrimidin-7(6*H*)-yl)ethanone |

### Example 40. Solubility of Compounds 3 and 9 in DMSO and H₂O

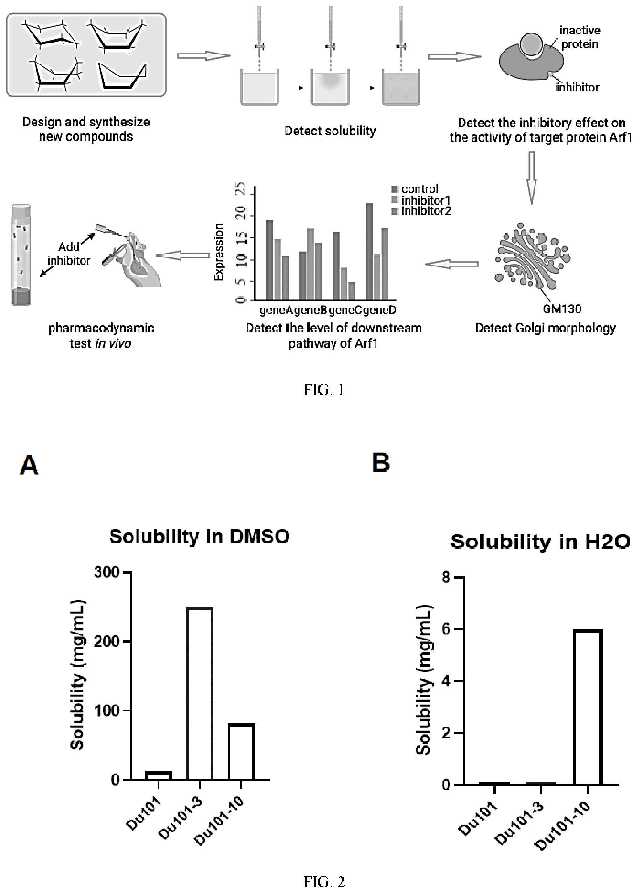

In this example, the solubility of Compounds 3 and 9 synthesized in the preparation examples was measured in DMSO and H₂O. The results are shown in FIG. 2. The results indicate that Compound 3 exhibits high solubility in DMSO but is poorly soluble in H₂O, while Compound 9 is soluble in DMSO and has high solubility in water.

### Example 41. Inhibition Test of Arf1 Activation

In this example, the inhibitory ability of Compounds 3 and 9 synthesized in the above preparation examples on Arf1 activation was tested. The Pierce Active Arf1 Pull-Down and Detection Kit (Thermo Scientific, Cat#16121) was used with the lysate of liver cancer Huh-7 cells to detect the activated Arf1. It is a complete kit that selectively enriches and detects GTP-bound Arf1 GTPase through specific protein interactions with the GGA3 protein-binding domain.

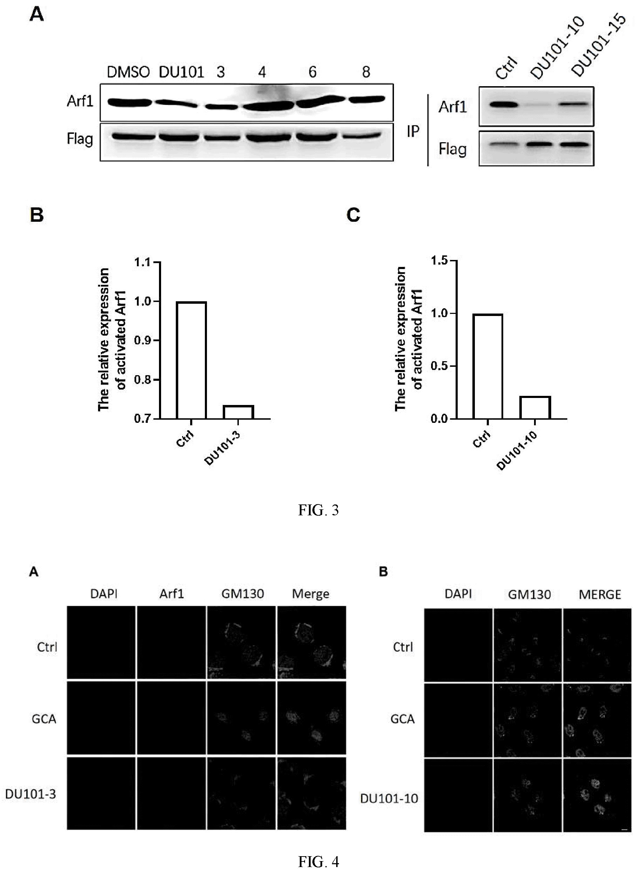

The test results are shown in FIG. 3, indicating that Compounds 3 and 9 of the present disclosure are effective inhibitors of the Arf1 activation.

### Example 42. Test for Golgi Morphology Abnormalities

In this example, Compounds 3 and 9 synthesized in the above preparation examples were tested for their ability to induce Golgi apparatus defects, as indicated by abnormal distribution of the Golgi marker GM130. GCA, a known Arf1 inhibitor, was used as a positive control.

Mouse neuroblastoma N2A (N2a, ATCC^{®}CCL-131^{™}) was cultured, treated, and subjected to immunofluorescence staining as previously reported (Wang *et al.*, 2021).

The test results are shown in FIG. 4, indicating that Compounds 3 and 9 of the present disclosure, similar to the known Arf1 inhibitor GCA, can effectively induce morphological changes in the Golgi apparatus.

### Example 43. Upregulation of Chemokine CCL5 Expression

In this example, the upregulation of chemokine CCL5 expression induced by the treatment of Compounds 3 and 9 synthesized in the above preparation examples was tested. Quantitative RT-PCR was used to measure the expression level of chemokine CCL5 in CT26 cells treated with DMSO, Compound 3, or Compound 9.

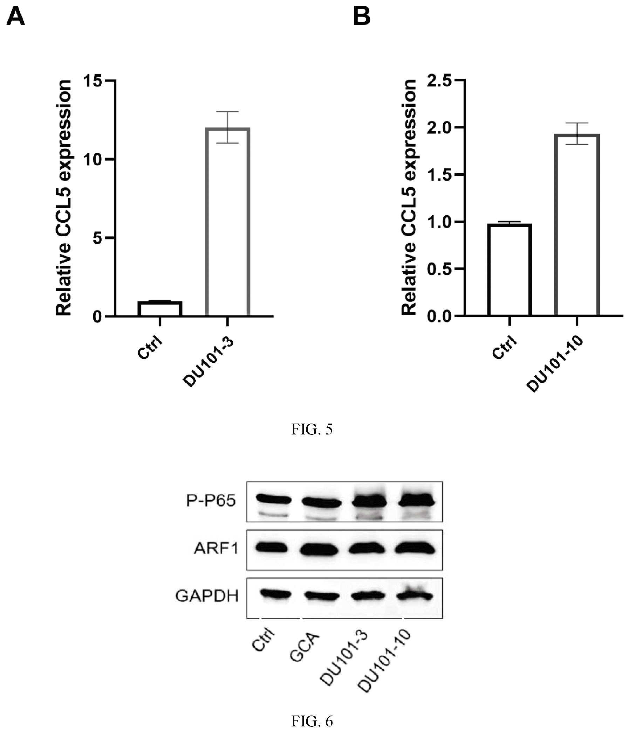

The test results are shown in FIG. 5, indicating that Compounds 3 and 9 of the present disclosure can effectively promote the expression of the chemokine CCL5.

### Example 44. Activation of NF-κB Signaling Pathway

In this example, Compounds 3 and 9 synthesized in the above preparation examples were tested for their ability to activate the NF-κB signaling pathway, as indicated by an increase of phosphorylated P65. GCA, a known Arf1 inhibitor, was used as a positive control.

In CT26 cells treated with DMSO, GCA, Compound 3, or Compound 9, the relevant protein levels were evaluated by Western blot analysis as previously described (Wang *et al.*, 2020).

The test results are shown in FIG. 6, indicating that Compounds 3 and 9 of the present disclosure, similar to the known Arf1 inhibitor GCA, increase the phosphorylation level of P65, a key molecule in the NF-κB signaling pathway.

### Example 45. Inhibition of Tumor Growth in Drosophila

In this example, the inhibitory effects of Compounds 3 and 9 synthesized in the above preparation examples on the growth of Drosophila orthotopic tumors were tested.

As previously described, renal stem cell tumor clusters were induced by expressing Ras in Drosophila renal stem cell (*Ras*^{V12}-PMML) clones (Singh *et al.*, 2016). The Drosophila carrying *Ras*^{V12}-PMML clones were cultured with normal food at room temperature for 4 days to allow tumor growth. They were then switched to food containing the indicated drugs for an additional 4 days, followed by feeding the Drosophila carrying *Ras*^{V12}-PMML with normal food containing DMSO or 5 µM Compound 3 or Compound 9. In the indicated groups, the number of esg>GFP + tumor cells per 5×10³ µm² area was quantified to characterize tumor size.

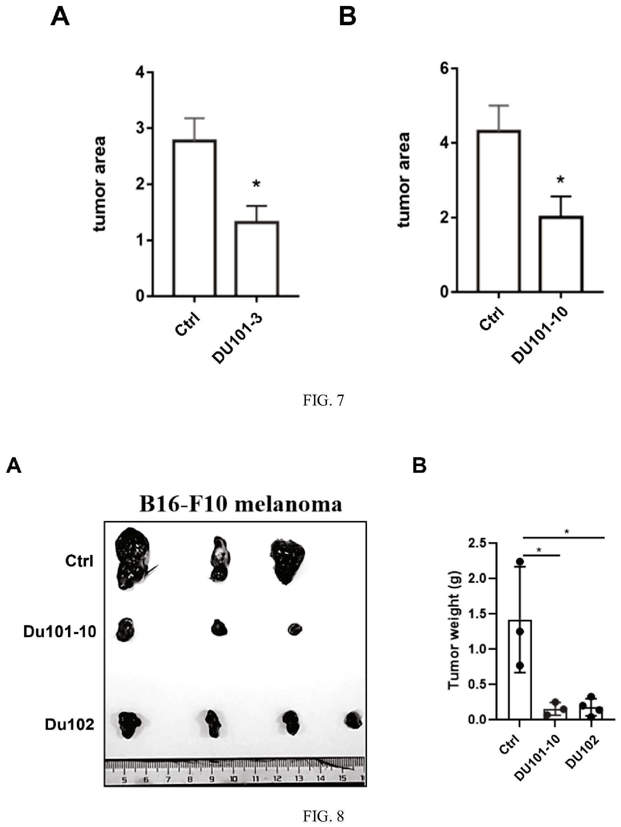

The test results are shown in FIG. 7, indicating that Compounds 3 and 9 of the present disclosure can effectively inhibit orthotopic tumor growth in Drosophila.

### Example 46. Inhibition of B16-F10 Melanoma in C57B/6 Mice

In this example, the inhibitory effect of Compound 9 synthesized in the above preparation example on melanoma tumor growth was tested.

Murine B16-F10 cells were inoculated at a dose of 5×10⁵ cells per mouse into the left abdomen of 6-week-old male mice. Approximately 7 days later, the mice were treated orally with ddH₂O or the novel Arf1 inhibitor for two consecutive weeks. The mice were randomly divided into three groups: the control group, the Compound 9 treatment group, and the DU102 treatment group. The mice were administered ddH₂O, 5 mg/kg of Compound 9 (dissolved in ddH₂O), or 5 mg/kg of DU102 (dissolved in a solvent composed of 5% DMSO + 95% corn oil, as a positive control) for two consecutive weeks. Tumor diameters were measured every 2-3 days using a digital caliper (ULINE, Cat^{#} H-7352), and the tumor volume was calculated using the formula ½ × longitudinal diameter (length) × maximum transverse diameter (width). When the maximum tumor volume reached 2000 mm³, the mice were euthanized.

The test results are shown in FIG. 8, indicating that Compound 9 of the present disclosure, similar to the positive control DU102, can effectively inhibit B16-F10 melanoma growth in C57B/6 mice.

### Example 47. Inhibition of CT26 Colon Cancer in BALB/c Mice

In this example, the inhibitory effects of Compounds 9, 11-3, 25, 26, 27, and DU102 on colon cancer growth were tested.

Murine CT26 cells were inoculated into the left abdomen of 6-week-old male mice at a dose of 5×10⁵ cells per mouse. Approximately 7 days later, the mice were randomly divided into two groups: the control group or the compound treatment group. They were gavaged daily with either the vehicle control (ddH₂O or 5% DMSO in corn oil) or 5 mg/kg of Compounds 9, 11-3, 25, 26, or 27 (dissolved in the corresponding vehicle) for two consecutive weeks. Tumor diameters were measured every 2-3 days using a digital caliper (ULINE, Cat^{#} H-7352), and the tumor volume was calculated using the formula ½ × longitudinal diameter (length) × maximum transverse diameter (width). When the maximum tumor volume reached 2000 mm³, the mice were euthanized.

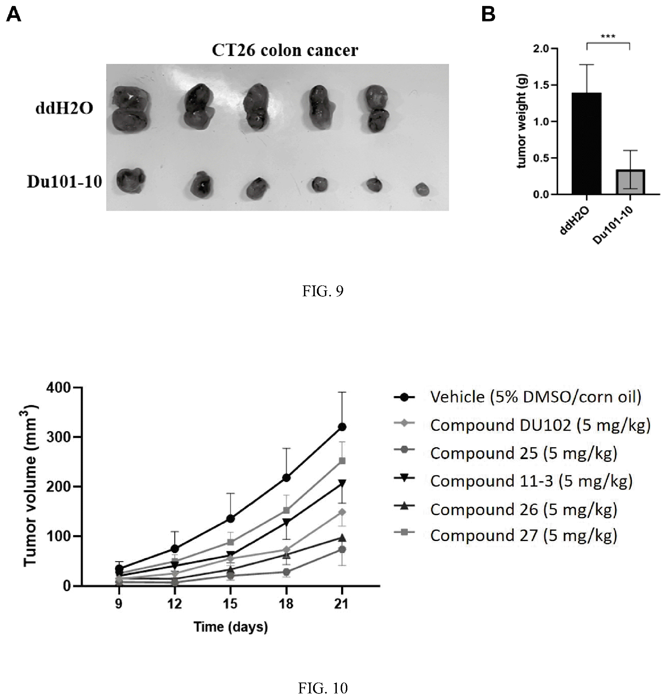

The test results are shown in FIG. 9 and FIG. 10, indicating that Compounds 9, 25, 26, and DU102 of the present disclosure can effectively inhibit CT26 colon cancer in BALB/c mice.

### Example 48. Inhibition of CT26 Colon Cancer in BALB/c Mice

In this example, the inhibitory effects of Compounds 28, 30, 31, 33, 34, and DU102 synthesized in the above preparation examples on the growth of colon cancer were tested.

Murine CT26 cells were inoculated into the left abdomen of 6-week-old male mice at a dose of 5×10⁵ cells per mouse. Approximately 7 days later, the mice were randomly divided into two groups: the control group or the compound treatment group. The mice were gavaged daily with either the vehicle control (ddH₂O or 5% DMSO in corn oil) or 10 mg/kg of Compounds 28, 30, 31, 33, 34, and DU102 (dissolved in the corresponding vehicle) for two consecutive weeks. Tumor diameters were measured every 2-3 days using a digital caliper (ULINE, Cat^{#} H-7352), and the tumor volume was calculated using the formula ½ × longitudinal diameter (length) × maximum transverse diameter (width). When the maximum tumor volume reached 3000 mm³, the mice were euthanized.

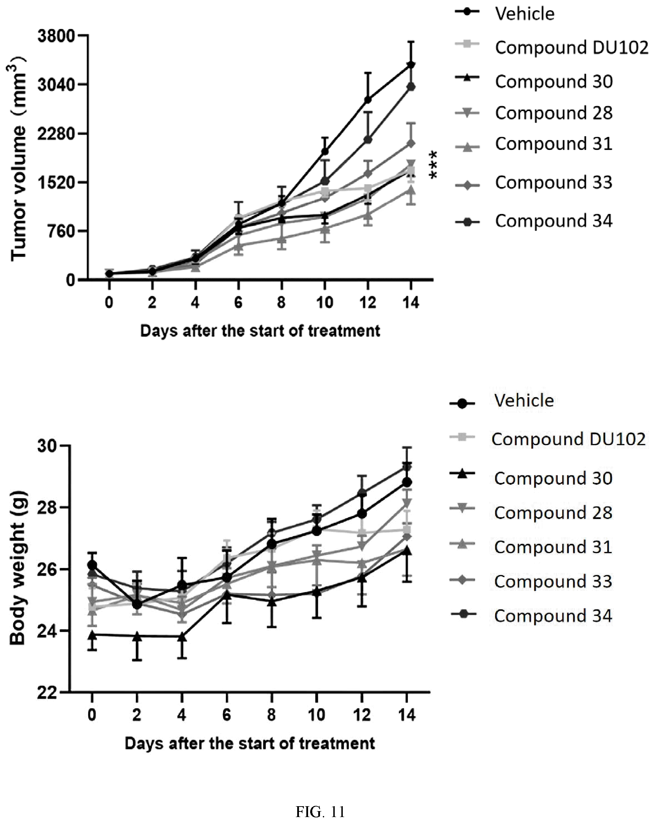

The test results are shown in FIG. 11, indicating that Compounds 28, 30, 31, 33, and DU102 of the present disclosure can effectively inhibit CT26 colon cancer in BALB/c mice. No significant reduction in body weight is observed during the administration period, suggesting that the aforementioned compounds of the present disclosure possess high safety.

### Example 49. Plasma Stability of Compound

At 37°C, 2.0 µM of the compound was co-incubated with plasma samples from five species (mouse, rat, dog, cynomolgus monkey, human) for 120 minutes. The concentration of the compound in the samples was detected by the liquid chromatography-tandem mass spectrometry (LC-MS/MS) method, and the half-life (T_{1/2}) was calculated.

The test results are shown in Table 2. After 120 minutes of incubation with Compound 9 (2.0 µM), the remaining amounts in mouse, rat, dog, and human plasma were 96.5%, 93.2%, 86.0%, and 104.6%, respectively, indicating high plasma stability.

**Table 2. Plasma Stability Results of Compound 9**

| Time Point (min) | 0 | 10 | 30 | 60 | 120 | T_{1/2} (min) |
|---|---|---|---|---|---|---|
| Mouse | 100.0 | 99.3 | 98.6 | 104.1 | 96.5 | |
| Rat | 100.0 | 102.1 | 93.5 | 99.3 | 93.2 | >289.1 |
| Dog | 100.0 | 99.0 | 94.0 | 98.8 | 86.0 | |
| Human | 100.0 | 99.8 | 108.9 | 113.3 | 104.6 | |

### Example 50. Permeability of Compound in Caco-2 Cells

The permeability of Compound 9 was evaluated using Caco-2 cells. The transport buffer used in this study was HBSS with 10.0 mM HEPES, pH 7.40±0.05. The test concentration was 2.00 µM, and a bidirectional test was performed on the test compound. Digoxin was repeatedly tested bidirectionally at 10.0 µM, while nadolol and metoprolol were repeatedly tested in the A-B direction at 2.00 µM. The final DMSO concentration was adjusted to less than 1%. The mixture was incubated at 37.0°C, 5% CO₂, saturated humidity, without shaking in a CO₂ incubator for 2 hours. All samples were mixed with acetonitrile containing internal standards and centrifuged at 3220 × g for 10 mins. The concentrations of the test compound and reference compound in the initial solution, donor solution, and acceptor solution were determined by the LC-MS/MS, using the analyte/internal standard peak area ratio.

The test results are shown in Table 3, indicating that Compound 9 of the present disclosure exhibits high cellular permeability and is unlikely to be a substrate of the efflux transporter P-gp.

**Table 3. Caco-2 Permeability Results of Compound 9**

| Compound | Average Papp (10⁻⁶ cm/s) | | Efflux Ratio (ER) |
|---|---|---|---|
| | A to B | B to A | |
| Compound 9 | 29.0 | 20.7 | 0.714 |

### Example 51. Pharmacokinetic Study of Compound in SD Rats

In this experiment, six male SD rats were divided into two groups based on similar body weight. These rats were administered Compound 9 via single intravenous injection and oral gavage, respectively. The intravenous injection dose was 2 mg/kg, while the oral gavage dose was 10 mg/kg. The vehicle for both intravenous and oral administration was a clear solution of 5% DMSO + 5% PEG400 + 90% water. The concentration for intravenous injection was 0.4 mg/mL, and for oral gavage was 1 mg/mL. K2-EDTA was used as the anticoagulant. The blood sampling time points were all at: 0.083, 0.25, 0.5, 1, 3, 6, 12, and 24 hours.

Blood samples of 0.02 mL were collected from the saphenous vein of all experimental animals, and the actual blood collection time was recorded. In this experiment, the deviation of the blood sampling time from the theoretical sampling time complied with the regulations (points within 1 hour after administration were within ±1 minute, and all others were within 5% of the theoretical time). After blood sample collection, the blood samples were immediately transferred to labeled centrifuge tubes containing K2-EDTA (0.5 M). The samples were then centrifuged (3000 g, 4°C, 15 minutes), and the plasma was collected. The plasma was transferred to a pre-cooled centrifuge tube, flash-frozen in dry ice, and stored in an ultra-low temperature freezer at -60°C or less until LC-MS/MS analysis was performed.

The test results are shown in Table 4, indicating that Compound 9 of the present disclosure is rapidly absorbed in rats, exhibits moderate apparent volume of distribution, and demonstrates high oral bioavailability.

**Table 4. Pharmacokinetic Data of Compound in Rats**

| **PK Parameters** | | **Compound 9** |
|---|---|---|
| Intravenous Injection | Dose (mg/kg) | 2 |
| | Half-Life T_{1/2} (h) | 0.183 |
| | Apparent Volume of Distribution V_{dss} (L/kg) | 0.647 |
| | Clearance Cl (mL/min/kg) | 39.8 |
| | Exposure AUC₀₋ₗₐₛₜ (ng*h/mL) | e |
| Oral Gavage | Dose (mg/kg) | 10 |
| | Maximum Concentration Cₘₐₓ (ng/mL) | 4767 |
| | Half-life T_{1/2} (h) | 0.570 |
| | Exposure AUC₀₋ₗₐₛₜ (ng*h/mL) | 6839 |
| | Bioavailability F (%) | 164% |

The foregoing description is considered merely illustrative of the principles of the present disclosure. Moreover, since many modifications and variations will be apparent to those skilled in the art, it is not intended to limit the present disclosure to the exact construction and processes described above. Accordingly, all suitable modifications and equivalents can be considered to fall within the scope of the present disclosure as defined by the appended claims.

All publications, patents, and patent applications cited herein are incorporated by reference in their entirety into the present disclosure.

### References

Aggarwal, P., Liu, Z., Cheng, G.Q., Singh, S.R., Shi, C., Chen, Y., Sun, L.V., and Hou, S.X. (2022). Disruption of the lipolysis pathway results in stem cell death through a sterile immunity-like pathway in adult Drosophila. Cell Rep. 39, 110958.
Baharom, F., Ramirez-Valdez, R.A., Tobin, K.K.S., Yamane, H., Dutertre, C.A., Khalilnezhad, A., Reynoso, G.V., Coble, V.L., Lynn, G.M., Mule, M.P., et al. (2021). Intravenous nanoparticle vaccination generates stem-like TCF1(+) neoantigen-specific CD8(+) T cells. Nat. Immunol. 22, 41-52.
Baldominos, P., Barbera-Mourelle, A., Barreiro, O., Huang, Y., Wight, A., Cho, J.W., Zhao, X., Estivill, G., Adam, I., Sanchez, X., et al. (2022). Quiescent cancer cells resist T cell attack by forming an immunosuppressive niche. Cell 185, 1694-1708.
Binnewies, M., Roberts, E.W., Kersten, K., Chan, V., Fearon, D.F., Merad, M., Coussens, L.M., Gabrilovich, D.I., Ostrand-Rosenberg, S., Hedrick, C.C., et al. (2018). Understanding the tumor immune microenvironment (TIME) for effective therapy. Nat. Med. 24, 541-550.
Bruni, D., Angell, H.K., and Galon, J. (2020). The immune contexture and Immunoscore in cancer prognosis and therapeutic efficacy. Nat. Rev. Cancer 20, 662-680.
Casalou, C., Faustino, A., and Barral, D.C. (2016). Arf proteins in cancer cell migration. Small GTPases 7, 270-282.
Chen, D.S., and Mellman, I. (2017). Elements of cancer immunity and the cancer-immune set point. Nature 541, 321-330.
de la Roche, M., Asano, Y., and Griffiths, G. M. (2016). Origins of the cytolytic synapse. Nat. Rev. Immunol. 16, 421-432.
Donaldson, J. G. and Jackson, C. L. (2011). ARF family G proteins and their regulators: roles in membrane transport, development and disease. Nat. Rev. Mol. Cell Biol. 12, 362-375.
D'Souza-Schorey, C., and Chavrier, P. (2006). ARF proteins: roles in membrane traffic and beyond. Nat. Rev. Mol. Cell Biol. 7, 347-358.
Eberhardt, C.S., Kissick, H.T., Patel, M.R., Cardenas, M.A., Prokhnevska, N., Obeng, R.C., Nasti, T.H., Griffith, C.C., Im, S.J., Wang, X., et al. (2021). Functional HPV-specific PD-1(+) stem-like CD8 T cells in head and neck cancer. Nature 597, 279-284.
Galletti, G., De Simone, G., Mazza, E.M.C., Puccio, S., Mezzanotte, C., Bi, T.M., Davydov, A.N., Metsger, M., Scamardella, E., Alvisi, G., et al. (2020). Two subsets of stem-like CD8(+) memory T cell progenitors with distinct fate commitments in humans. Nat. Immunol. 21, 1552-1562.
Gupta, Y.H., Khanom, A., and Acton, S.E. (2022). Control of Dendritic Cell Function Within the Tumour Microenvironment. Front. Immunol. 13, 733800.
Hashimoto, M., Araki, K., Cardenas, M. A., Li, P., Jadhav, R. R., Kissick, H. T., Hudson, W. H., McGuire, D. J., Obeng, R. C., Wieland, A., et al. (2022). PD-1 combination therapy with IL-2 modifies CD8+ T cell exhaustion program. Nature 610, 173-181.
Hass, R., von der Ohe, J., and Ungefroren, H. (2020). Impact of the Tumor Microenvironment on Tumor Heterogeneity and Consequences for Cancer Cell Plasticity and Stemness. Cancers 12, 3716.
Im, S.J., Hashimoto, M., Gerner, M.Y., Lee, J., Kissick, H.T., Burger, M.C., Shan, Q., Hale, J.S., Lee, J., Nasti, T.H., et al. (2016). Defining CD8+ T cells that provide the proliferative burst after PD-1 therapy. Nature 537, 417-421.
Jain, S., Annett, S.L., Morgan, M.P., and Robson, T. (2021). The Cancer Stem Cell Niche in Ovarian Cancer and Its Impact on Immune Surveillance. Int. J. Mol. Sci. 22, 4091.
Jansen, C.S., Prokhnevska, N., Master, V.A., Sanda, M.G., Carlisle, J.W., Bilen, M.A., Cardenas, M., Wilkinson, S., Lake, R., Sowalsky, A.G., et al. (2019). An intra-tumoral niche maintains and differentiates stem-like CD8 T cells. Nature 576, 465-470.
Jiao, S., Subudhi, S.K., Aparicio, A., Ge, Z., Guan, B., Miura, Y., and Sharma, P. (2019). Differences in Tumor Microenvironment Dictate T Helper Lineage Polarization and Response to Immune Checkpoint Therapy. Cell 179, 1177-1190.
Kaczmarek, B., Verbavatz, J. M. and Jackson, C. L. (2017). GBF1 and Arf1 function in vesicular trafficking, lipid homoeostasis and organelle dynamics. Biol. Cell 109, 391-399,
Krishna, S., Lowery, F.J., Copeland, A.R., Bahadiroglu, E., Mukherjee, R., Jia, L., Anibal, J.T., Sachs, A., Adebola, S.O., Gurusamy, D., et al. (2020). Stem-like CD8 T cells mediate response of adoptive cell immunotherapy against human cancer. Science 370, 1328-1334.
Kurtulus, S., Madi, A., Escobar, G., Klapholz, M., Nyman, J., Christian, E., Pawlak, M., Dionne, D., Xia, J., Rozenblatt-Rosen, O., et al. (2019). Checkpoint Blockade Immunotherapy Induces Dynamic Changes in PD-1(-)CD8(+) Tumor-Infiltrating T Cells. Immunity 50, 181-194 e186.
Lane, J. E., Walker, A. N., Kulharya, A., and Marzec, T. (2002). Cutaneous sclerosing extramedullary hematopoietic tumor in chronic myelogenous leukemia. J. Cutan. Pathol. 29, 608-612.
Lang, L., Shay, C., Zhao, X., and Teng, Y. (2017). Combined targeting of Arf1 and Ras potentiates anticancer activity for prostate cancer therapeutics. J. Exp. Clin. Cancer Res. 36, 112.
Lytle, N. K., Barber, A. G., and Reya, T. (2018). Stem cell fate in cancer growth, progression and therapy resistance. Nat. Rev. Cancer 18, 669-680.
Mo, F., Yu, Z., Li, P., Oh, J., Spolski, R., Zhao, L., Glassman, C.R., Yamamoto, T.N., Chen, Y., Golebiowski, F.M., et al. (2021). An engineered IL-2 partial agonist promotes CD8(+) T cell stemness. Nature 597, 544-548.
Müller, L., Tunger, A., Plesca, I., Wehner, R., Temme, A., Westphal, D., Meier, F., Bachmann, M., and Schmitz, M. (2020). Bidirectional Crosstalk Between Cancer Stem Cells and Immune Cell Subsets. Front. Immunol. 11, 140.
Prokhnevska, N., Cardenas, M. A., Valanparambil, R. M., Sobierajska, E., et al. (2023). CD8+ T cell activation in cancer comprises an initial activation phase in lymph nodes followed by effector differentiation within the tumor. Immunity 56, 107-124.
Rao, S. A., Gottesman, S. R., Nguyen, M. C., and Braverman, A. S. (2003). T cell lymphoma associated with myelofibrosis. Leuk. Lymphoma. 44, 715-718.
Robbins, P.F., Dudley, M.E., Wunderlich, J., El-Gamil, M., Li, Y.F., Zhou, J., Huang, J., Powell, D.J., Jr., and Rosenberg, S.A. (2004). Cutting edge: persistence of transferred lymphocyte clonotypes correlates with cancer regression in patients receiving cell transfer therapy. J. Immunol. 173, 7125-7130.
Schad, S.E., Chow, A., Mangarin, L., Pan, H., Zhang, J., Ceglia, N., Caushi, J.X., Malandro, N., Zappasodi, R., Gigoux, M., et al. (2022). Tumor-induced double positive T cells display distinct lineage commitment mechanisms and functions. J. Exp. Med. 219, e20212169.
Schmid, P., Cortes, J., Pusztai, L., McArthur, H., Kummel, S., Bergh, J., Denkert, C., Park, Y.H., Hui, R., Harbeck, N., et al. (2020). Pembrolizumab for Early Triple-Negative Breast Cancer. N. Engl. J. Med. 382, 810-821.
Sharma, P., Hu-Lieskovan, S., Wargo, J.A., and Ribas, A. (2017). Primary, Adaptive, and Acquired Resistance to Cancer Immunotherapy. Cell 168, 707-723.
Siddiqui, I., Schaeuble, K., Chennupati, V., Fuertes Marraco, S.A., Calderon-Copete, S., Pais Ferreira, D., Carmona, S.J., Scarpellino, L., Gfeller, D., Pradervand, S., et al. (2019). Intratumoral Tcf1(+)PD-1(+)CD8(+) T Cells with Stem-like Properties Promote Tumor Control in Response to Vaccination and Checkpoint Blockade Immunotherapy. Immunity 50, 195-211.
Singh, S.R., Zeng, X., Zhao, J., Liu, Y., Hou, G., Liu, H., and Hou, S.X. (2016). The lipolysis pathway sustains normal and transformed stem cells in adult Drosophila. Nature 538, 109-113.
Spranger, S., and Gajewski, T.F. (2018). Impact of oncogenic pathways on evasion of antitumour immune responses. Nat. Rev. Cancer 18, 139-147.
Thorsson, V., Gibbs, D.L., Brown, S.D., Wolf, D., Bortone, D.S., Ou Yang, T.H., Porta-Pardo, E., Gao, G.F., Plaisier, C.L., Eddy, J.A., et al. (2018). The Immune Landscape of Cancer. Immunity 48, 812-830.
Trumpp, A., and Haas, S. (2022). Cancer stem cells: The adventurous journey from hematopoietic to leukemic stem cells. Cell 185, 1266-1270.
Tumeh, P.C., Harview, C.L., Yearley, J.H., Shintaku, I.P., Taylor, E.J., Robert, L., Chmielowski, B., Spasic, M., Henry, G., Ciobanu, V., et al. (2014). PD-1 blockade induces responses by inhibiting adaptive immune resistance. Nature 515, 568-571.
Wang, G., Xu, J., Zhao, J., Yin, W., Liu, D., Chen, W., and Hou, S.X. (2020). Arf1-mediated lipid metabolism sustains cancer cells and its ablation induces anti-tumor immune responses in mice. Nat. Commun. 11, 220.
Wellenstein, M.D., and de Visser, K.E. (2018). Cancer-Cell-Intrinsic Mechanisms Shaping the Tumor Immune Landscape. Immunity 48, 399-416.
Wilfling, F., Thiam, A. R., Olarte, M. J., Wang, J., Beck, R., Gould, T. J. et al. (2014). Arf1/COPI machinery acts directly on lipid droplets and enables their connection to the ER for protein targeting. Elife 3, e01607.
Wu, T.D., Madireddi, S., de Almeida, P.E., Banchereau, R., Chen, Y.J., Chitre, A.S., Chiang, E.Y., Iftikhar, H., O'Gorman, W.E., Au-Yeung, A., et al. (2020). Peripheral T cell expansion predicts tumour infiltration and clinical response. Nature 579, 274-278.
Yost, K.E., Satpathy, A.T., Wells, D.K., Qi, Y., Wang, C., Kageyama, R., McNamara, K.L., Granja, J.M., Sarin, K.Y., Brown, R.A., et al. (2019). Clonal replacement of tumor-specific T cells following PD-1 blockade. Nat. Med. 25, 1251-1259.
Yu, J., Green, M.D., Li, S., Sun, Y., Journey, S.N., Choi, J.E., Rizvi, S.M., Qin, A., Waninger, J.J., Lang, X., et al. (2021). Liver metastasis restrains immunotherapy efficacy via macrophage-mediated T cell elimination. Nat. Med. 27, 152-164.
Zeng, X., Han, L., Singh, S.R., Liu, H., Neumuller, R.A., Yan, D., Hu, Y., Liu, Y., Liu, W., Lin, X., and Hou, S.X. (2015). Genome-wide RNAi screen identifies networks involved in intestinal stem cell regulation in Drosophila. Cell Rep. 10, 1226-1238.
Zhao, B., Zhao, H., and Zhao, J. (2020). Efficacy of PD-1/PD-L1 blockade monotherapy in clinical trials. Ther. Adv. Med. Oncol. 12, 1758835920937612.
Zheng, B., Wang, D., Qiu, X., Luo, G., Wu, T., Yang, S., Li, Z., Zhu, Y., Wang, S., Wu, R., et al. (2020). Trajectory and Functional Analysis of PD-1(high) CD4(+)CD8(+) T Cells in Hepatocellular Carcinoma by Single-Cell Cytometry and Transcriptome Sequencing. Adv. Sci. (Weinh) 7, 2000224.

## Claims

1. A compound of Formula (I), a stereoisomer thereof, an isotopic variant thereof, or a pharmaceutically acceptable salt or solvate of any one of the foregoing: 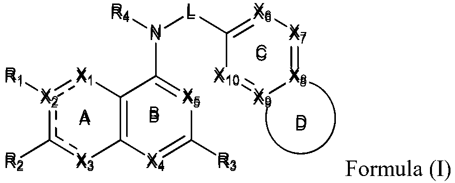 wherein
X₁ is absent or is CH;
X₂ is C or N;
X₃ is S, O, CH, or N;
- - - represents a single bond or a double bond;
X₄, X₅, X₆, X₇, X₈, X₉, and X₁₀ are each independently C(Rₓ) or N, wherein Rₓ is independently selected from the group consisting of H, D (deuterium), CN, OH, halogen, C₁₋₆ alkyl, C₁₋₆ haloalkyl, C₁₋₆ alkoxy, and C₁₋₆ haloalkoxy;
R₁ and R₂ are each independently absent or selected from the group consisting of H, D, CN, OH, halogen, C₁₋₆ alkyl optionally substituted with 1-3 Rₐ, and C₁₋₆ alkoxy optionally substituted with 1-3 R_{b}, or R₁ and R₂ together with the carbon atom to which they are attached form a C₅₋₈ carbocycle optionally substituted with 1-3 R_{c} or a 5- to 8-membered heterocycle optionally substituted with 1-3 R_{c}, wherein the 5- to 8-membered heterocycle optionally contains one or more heteroatoms selected from N, O, and S;
R₃ is selected from H, D, CN, OH, halogen, C₁₋₆ alkyl optionally substituted with 1-3 Rₐ, C₁₋₆ alkoxy optionally substituted with 1-3 R_{b}, and N(Rₙ)₂, wherein each Rₙ is independently H, D, C₁₋₆ alkyl optionally substituted with 1-3 Rₐ, or C₆₋₁₄ aryl optionally substituted with 1-3 R_{g};
R₄ is selected from H and C₁₋₆ alkyl optionally substituted with 1-3 Rₐ;
L is absent or is selected from C₁₋₆ hydrocarbylene optionally substituted with 1-3 R_{d} and C₁₋₆ heterohydrocarbylene optionally substituted with 1-3 Rₑ, wherein the heterohydrocarbylene optionally contains one or more heteroatoms selected from N, O, and S;
ring D is absent or is a C₅₋₆ carbocycle optionally substituted with 1-3 R_{f} or a 5- to 6-membered heterocycle optionally substituted with 1-3 R_{f},
each occurrence of Rₐ, R_{b}, R_{c}, R_{d}, Rₑ, R_{f}, and R_{g} is independently selected from the group consisting of D, halogen, C₁₋₆ alkyl, C₁₋₆ haloalkyl, C₁₋₆ alkoxy, C₁₋₆ haloalkoxy, -C(O)-C₁₋₆ alkyl, oxo (=O), and - NH₂.

2. A compound of Formula (I), a stereoisomer thereof, an isotopic variant thereof, or a pharmaceutically acceptable salt or solvate of any one of the foregoing: 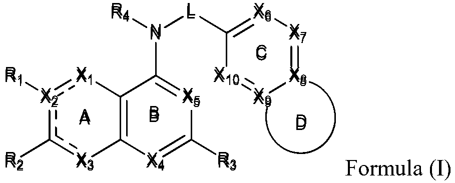 wherein
X₁ is absent or is CH;
X₂ is C or N;
X₃ is S, O, CH, or N;
- - - represents a single bond or a double bond;
X₄, X₅, X₆, X₇, X₈, X₉, and X₁₀ are each independently C(Rₓ) or N, wherein Rₓ is independently selected from the group consisting of H, D (deuterium), CN, OH, halogen, C₁₋₆ alkyl, C₁₋₆ haloalkyl, C₁₋₆ alkoxy, and C₁₋₆ haloalkoxy;
R₁ and R₂ are each independently absent or selected from the group consisting of H, D, CN, OH, halogen, C₁₋₆ alkyl optionally substituted with 1-3 Rₐ, and C₁₋₆ alkoxy optionally substituted with 1-3 R_{b}, or R₁ and R₂ together with the carbon atom to which they are attached form a C₅₋₈ carbocycle optionally substituted with 1-3 R_{c} or a 5- to 8-membered heterocycle optionally substituted with 1-3 R_{c}, wherein the 5- to 8-membered heterocycle optionally contains one or more heteroatoms selected from N, O, and S;
R₃ is selected from H, D, CN, OH, halogen, C₁₋₆ alkyl optionally substituted with 1-3 Rₐ, C₁₋₆ alkoxy optionally substituted with 1-3 R_{b}, and N(Rₙ)₂, wherein each Rₙ is independently H, D, C₁₋₆ alkyl optionally substituted with 1-3 Rₐ, or C₆₋₁₄ aryl optionally substituted with 1-3 R_{g};
R₄ is selected from H and C₁₋₆ alkyl optionally substituted with 1-3 Rₐ;
L is absent or is selected from C₁₋₆ hydrocarbylene optionally substituted with 1-3 R_{d} and C₁₋₆ heterohydrocarbylene optionally substituted with 1-3 Rₑ, wherein the heterohydrocarbylene optionally contains one or more heteroatoms selected from N, O, and S;
ring D is absent or is a C₅₋₆ carbocycle optionally substituted with 1-3 R_{f} or a 5- to 6-membered heterocycle optionally substituted with 1-3 R_{f},
each occurrence of Rₐ, R_{b}, R_{c}, Rₐ, Rₑ, R_{f}, and R_{g} is independently selected from the group consisting of D, halogen, C₁₋₆ alkyl, C₁₋₆ haloalkyl, C₁₋₆ alkoxy, C₁₋₆ haloalkoxy, C₁₋₆ hydroxyalkyl, C₁₋₆ aminoalkyl, -C(O)-C₁₋₆ alkyl, -C(O)-C₁₋₆ haloalkyl, -C(O)-C₁₋₆ hydroxyalkyl, -C(O)-C₁₋₆ aminoalkyl, -C(O)-optionally substituted C₅₋₁₄ aryl, -C(O)-optionally substituted C₅₋₁₄ heteroaryl, oxo (=O), and -NH₂, wherein the optionally substituted C₅₋₁₄ aryl and optionally substituted C₅₋₁₄ heteroaryl are optionally substituted with 1-3 substituents selected from the group consisting of D, halogen, -OH, and -NH₂.

3. The compound of Formula (I), the stereoisomer thereof, the isotopic variant thereof, or the pharmaceutically acceptable salt or solvate of any one of the foregoing according to claim 2: 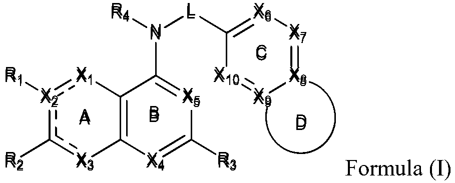 wherein
X₁ is absent or is CH;
X₂ is C or N;
X₃ is S, O, CH, or N;
--- represents a single bond or a double bond;
X₄, X₅, X₆, X₇, X₈, X₉, and X₁₀ are each independently C(Rₓ) or N, wherein Rₓ is independently selected from the group consisting of H, D (deuterium), CN, OH, halogen, C₁₋₆ alkyl, C₁₋₆ haloalkyl, C₁₋₆ alkoxy, and C₁₋₆ haloalkoxy;
R₁ and R₂ are each independently absent or selected from the group consisting of H, D, CN, OH, halogen, C₁₋₆ alkyl optionally substituted with 1-3 Rₐ, and C₁₋₆ alkoxy optionally substituted with 1-3 R_{b}, or R₁ and R₂ together with the carbon atom to which they are attached form a C₅₋₈ carbocycle optionally substituted with 1-3 R_{c} or a 5- to 8-membered heterocycle optionally substituted with 1-3 R_{c}, wherein the 5- to 8-membered heterocycle optionally contains one or more heteroatoms selected from N, O, and S;
R₃ is selected from H, D, CN, OH, halogen, C₁₋₆ alkyl optionally substituted with 1-3 Rₐ, C₁₋₆ alkoxy optionally substituted with 1-3 R_{b}, and N(Rₙ)₂, wherein each Rₙ is independently H, D, C₁₋₆ alkyl optionally substituted with 1-3 Rₐ, or C₆₋₁₄ aryl optionally substituted with 1-3 R_{g};
R₄ is selected from H and C₁₋₆ alkyl optionally substituted with 1-3 Rₐ;
L is absent or is selected from C₁₋₆ hydrocarbylene optionally substituted with 1-3 R_{d} and C₁₋₆ heterohydrocarbylene optionally substituted with 1-3 Rₑ, wherein the heterohydrocarbylene optionally contains one or more heteroatoms selected from N, O, and S;
ring D is absent or is a C₅₋₆ carbocycle optionally substituted with 1-3 R_{f} or a 5- to 6-membered heterocycle optionally substituted with 1-3 R_{f};
each occurrence of Rₐ, R_{b}, R_{c}, R_{d}, Rₑ, R_{f}, and R_{g} is independently selected from the group consisting of C₁₋₆ hydroxyalkyl, C₁₋₆ aminoalkyl, -C(O)-C₁₋₆ haloalkyl, -C(O)-C₁₋₆ hydroxyalkyl, -C(O)-C₁₋₆ aminoalkyl, -C(O)-optionally substituted C₅₋₁₄ aryl, and -C(O)-optionally substituted C₅₋₁₄ heteroaryl, wherein the optionally substituted C₅₋₁₄ aryl and optionally substituted C₅₋₁₄ heteroaryl are optionally substituted with 1-3 substituents selected from the group consisting of D, halogen, -OH, and -NH₂.

4. The compound of Formula (I), the stereoisomer thereof, the isotopic variant thereof, or the pharmaceutically acceptable salt or solvate of any one of the foregoing according to any one of claims 1-3, wherein ring A is selected from the following ring structures:

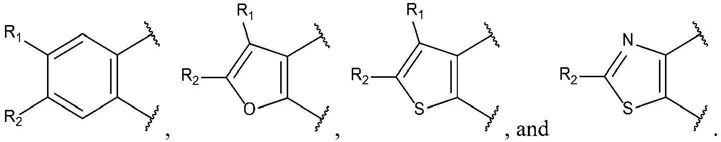

5. The compound of Formula (I), the stereoisomer thereof, the isotopic variant thereof, or the pharmaceutically acceptable salt or solvate of any one of the foregoing according to any one of claims 1-4, wherein R₁ and R₂ are each independently absent or selected from the group consisting of H, D, CN, OH, halogen, C₁₋₄ alkyl optionally substituted with 1-3 Rₐ, and C₁₋₄ alkoxy optionally substituted with 1-3 R_{b}.

6. The compound of Formula (I), the stereoisomer thereof, the isotopic variant thereof, or the pharmaceutically acceptable salt or solvate of any one of the foregoing according to any one of claims 1-4, wherein R₁ and R₂ together with the carbon atom to which they are attached form a C₅₋₈ carbocycle or 5- to 8-membered heterocycle selected from the following ring structures: 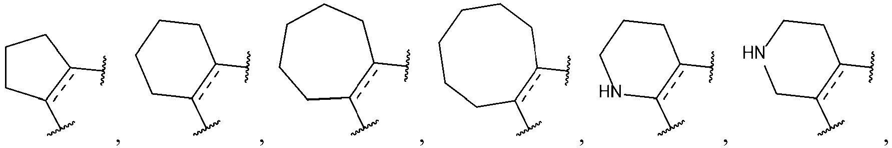 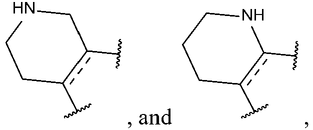 the C₅₋₈ carbocycle or 5- to 8-membered heterocycle is optionally substituted with 1-3 R_{c}.

7. The compound of Formula (I), the stereoisomer thereof, the isotopic variant thereof, or the pharmaceutically acceptable salt or solvate of any one of the foregoing according to any one of claims 1-6, wherein ring B is selected from the following structures:

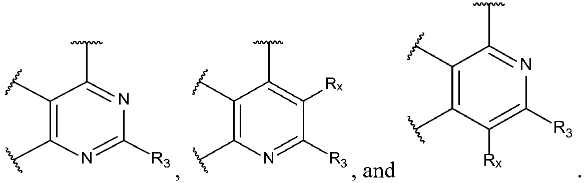

8. The compound of Formula (I), the stereoisomer thereof, the isotopic variant thereof, or the pharmaceutically acceptable salt or solvate of any one of the foregoing according to any one of claims 1-7, wherein ring A and ring B together form a ring structure selected from:

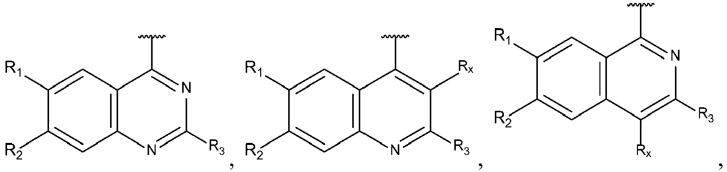

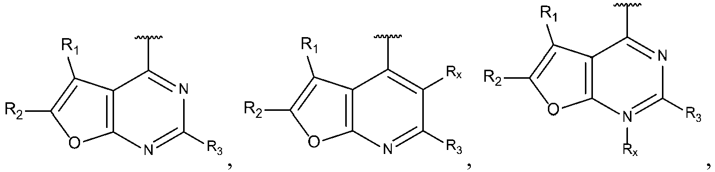

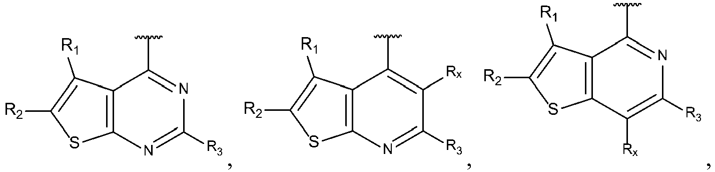

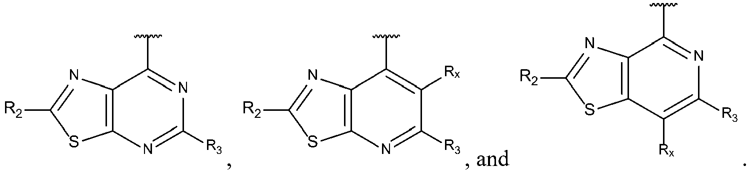

9. The compound of Formula (I), the stereoisomer thereof, the isotopic variant thereof, or the pharmaceutically acceptable salt or solvate of any one of the foregoing according to any one of claims 1-8, wherein R₁ and R₂ are each independently absent or selected from the group consisting of H, D, CN, OH, halogen, C₁₋₄ alkyl optionally substituted with 1-3 Rₐ, and C₁₋₄ alkoxy optionally substituted with 1-3 R_{b}.

10. The compound of Formula (I), the stereoisomer thereof, the isotopic variant thereof, or the pharmaceutically acceptable salt or solvate of any one of the foregoing according to any one of claims 1-8, wherein R₁ and R₂ together with the carbon atom to which they are attached form a C₅₋₈ carbocycle or 5- to 8-membered heterocycle selected from the following ring structures: 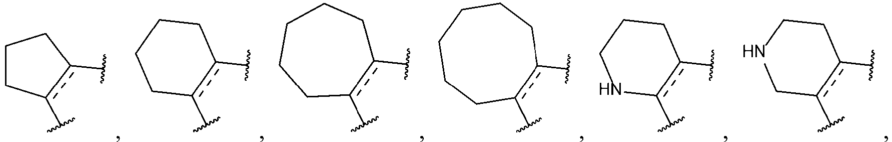 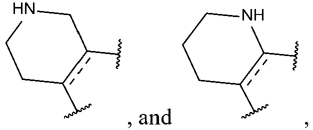 the C₅₋₈ carbocycle or 5- to 8-membered heterocycle is optionally substituted with 1-3 R_{c}.

11. The compound of Formula (I), the stereoisomer thereof, the isotopic variant thereof, or the pharmaceutically acceptable salt or solvate of any one of the foregoing according to any one of claims 1-10, wherein X₆, X₇, X₈, X₉, and X₁₀ are each independently C(Rₓ).

12. The compound of Formula (I), the stereoisomer thereof, the isotopic variant thereof, or the pharmaceutically acceptable salt or solvate of any one of the foregoing according to any one of claims 1-11, wherein ring C is a benzene ring optionally substituted with 1-5 Rₓ such as 1-3 Rₓ or a pyridine ring optionally substituted with 1-4 Rₓ such as 1-2 Rₓ.

13. The compound of Formula (I), the stereoisomer thereof, the isotopic variant thereof, or the pharmaceutically acceptable salt or solvate of any one of the foregoing according to any one of claims 1-12, wherein ring D is the following ring structure optionally substituted with 1-3 R_{f}: 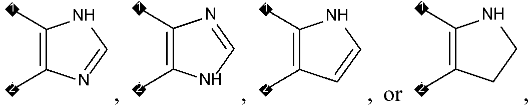 wherein "1" indicates the connection direction to X₈ of ring C, and "2" indicates the connection direction to X₉ of ring C.

14. The compound of Formula (I), the stereoisomer thereof, the isotopic variant thereof, or the pharmaceutically acceptable salt or solvate of any one of the foregoing according to any one of claims 1-12, wherein ring D is the following ring structure optionally substituted with 1-3 R_{f}: 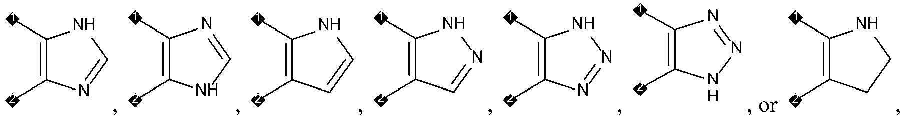 wherein "1" indicates the connection direction to X₈ of ring C, and "2" indicates the connection direction to X₉ of ring C.

15. The compound of Formula (I), the stereoisomer thereof, the isotopic variant thereof, or the pharmaceutically acceptable salt or solvate of any one of the foregoing according to claim 14, wherein ring D is the following ring structure optionally substituted with 1-3 R_{f}: 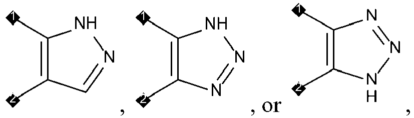 wherein "1" indicates the connection direction to X₈ of ring C, and "2" indicates the connection direction to X₉ of ring C.

16. The compound of Formula (I), the stereoisomer thereof, the isotopic variant thereof, or the pharmaceutically acceptable salt or solvate of any one of the foregoing according to any one of claims 1-15, wherein ring C and ring D together form the following fused ring structure: 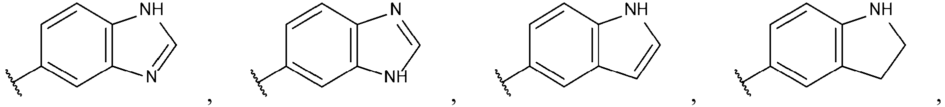 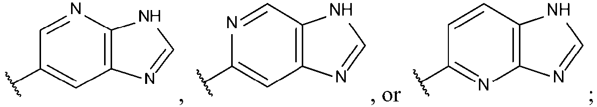 ring C is optionally substituted with 1-3 Rₓ, and ring D is optionally substituted with 1-3 R_{f}.

17. The compound of Formula (I), the stereoisomer thereof, the isotopic variant thereof, or the pharmaceutically acceptable salt or solvate of any one of the foregoing according to any one of claims 1-16, wherein ring C and ring D together form the following fused ring structure: 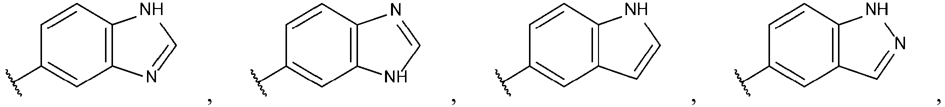 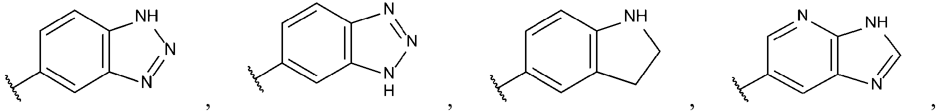 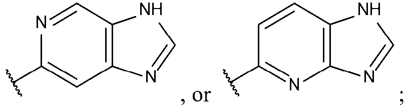 ring C is optionally substituted with 1-3 Rₓ, and ring D is optionally substituted with 1-3 R_{f}.

18. The compound of Formula (I), the stereoisomer thereof, the isotopic variant thereof, or the pharmaceutically acceptable salt or solvate of any one of the foregoing according to claim 17, wherein ring C and ring D together form the following fused ring structure: 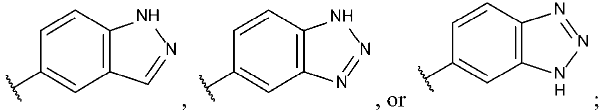 ring C is optionally substituted with 1-3 Rₓ, and ring D is optionally substituted with 1-3 R_{f}.

19. The compound of Formula (I), the stereoisomer thereof, the isotopic variant thereof, or the pharmaceutically acceptable salt or solvate of any one of the foregoing according to any one of claims 1-18, wherein when present, L is selected from the group consisting of -CH₂-, -C(CH₃)-, -CH₂CH₂-, - NH-CH=CH-, and -N=CH₂-.

20. The compound of Formula (I), the stereoisomer thereof, the isotopic variant thereof, or the pharmaceutically acceptable salt or solvate of any one of the foregoing according to any one of claims 1-19, wherein the compound of Formula (I) is selected from the following compounds: 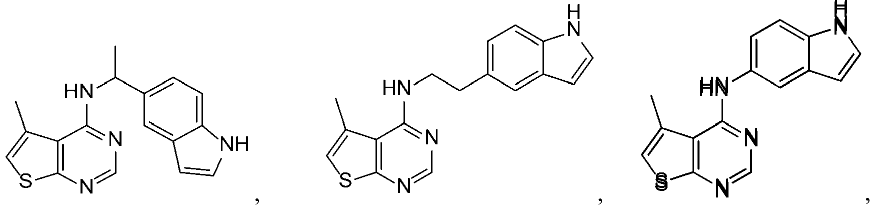 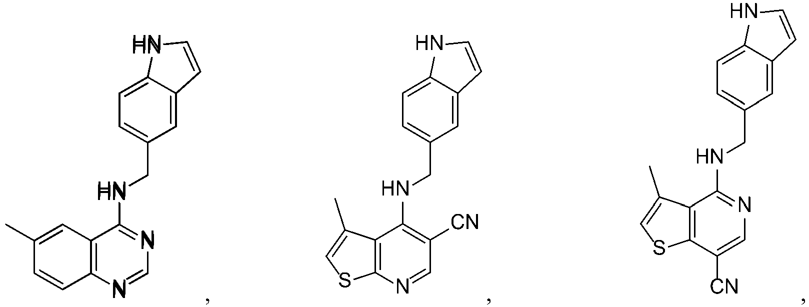 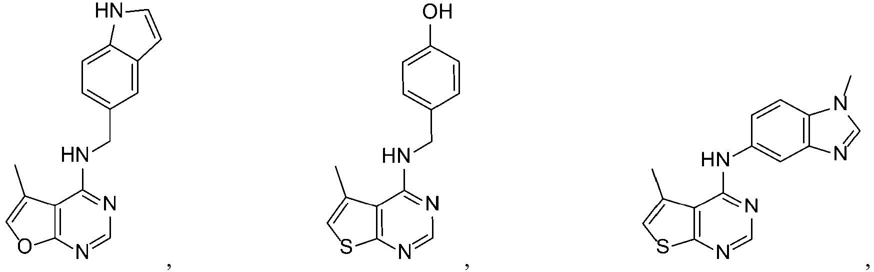 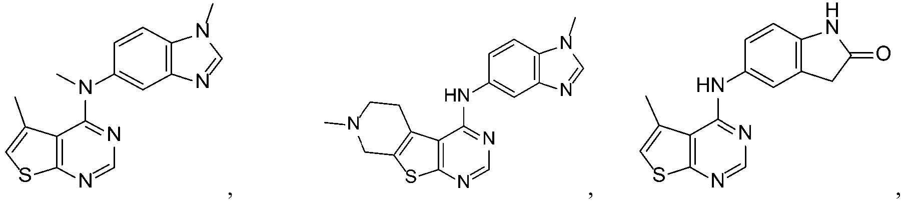 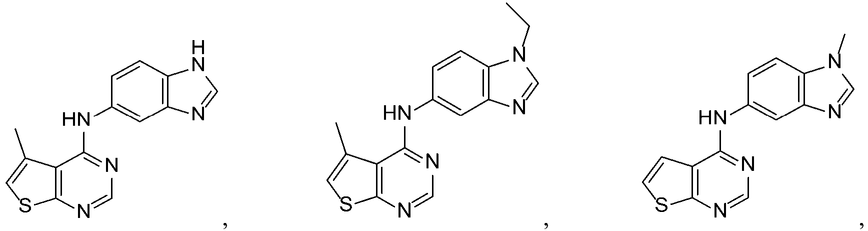 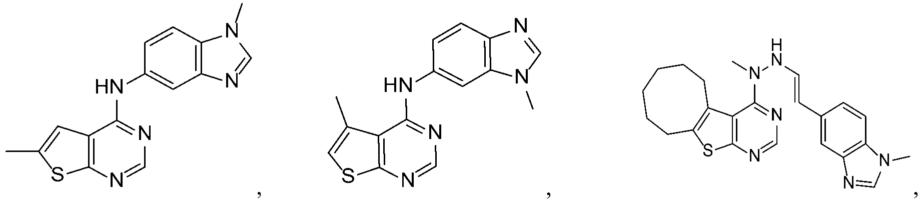 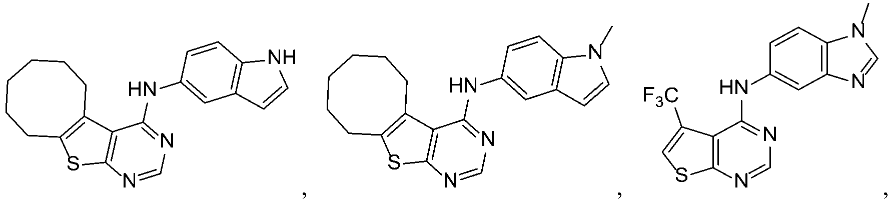 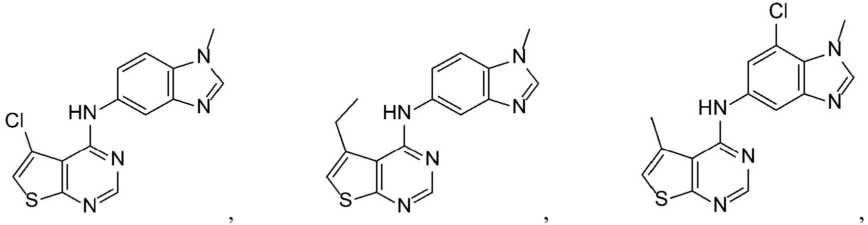 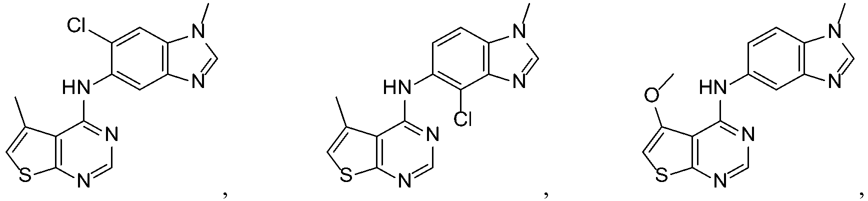 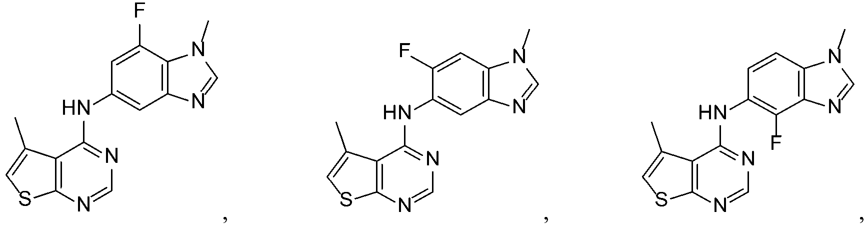 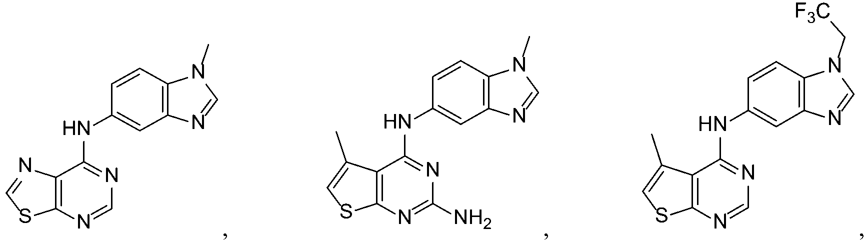 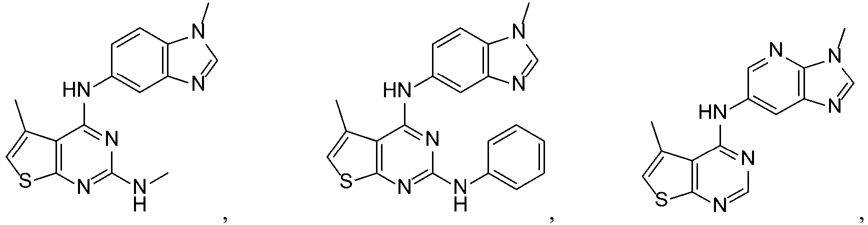 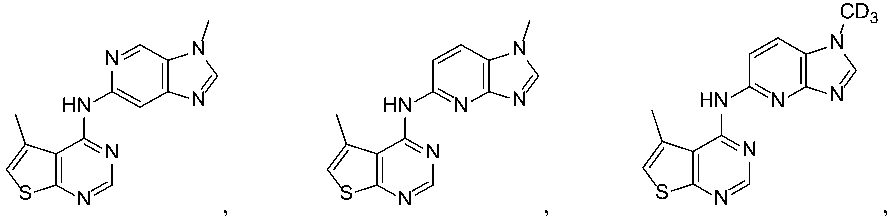 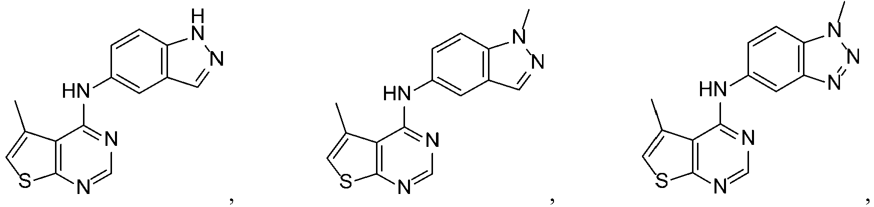 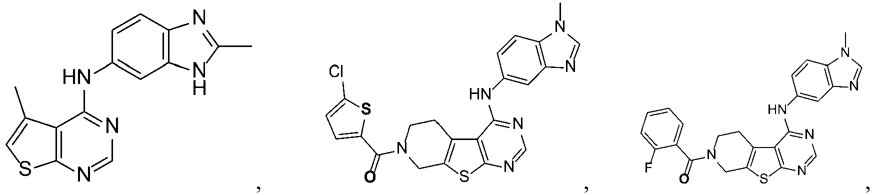 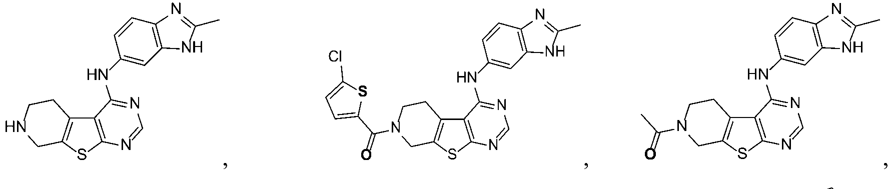 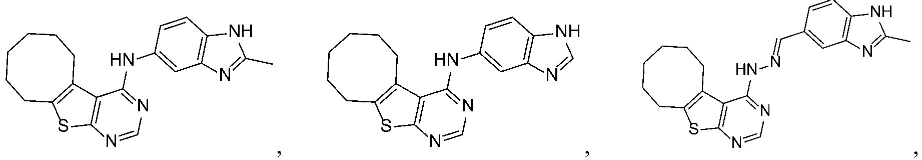 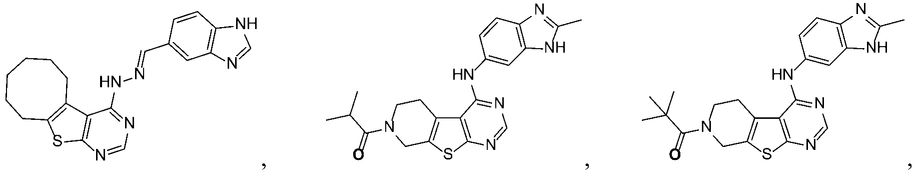 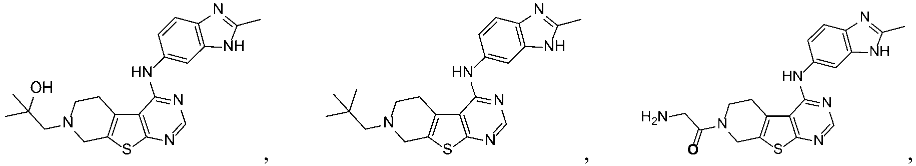 and

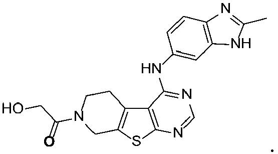

21. A preparation method for the compound of Formula (I), the stereoisomer thereof, the isotopic variant thereof, or the pharmaceutically acceptable salt or solvate of any one of the foregoing according to any one of claims 1-20, wherein the preparation method comprises the following synthetic route: 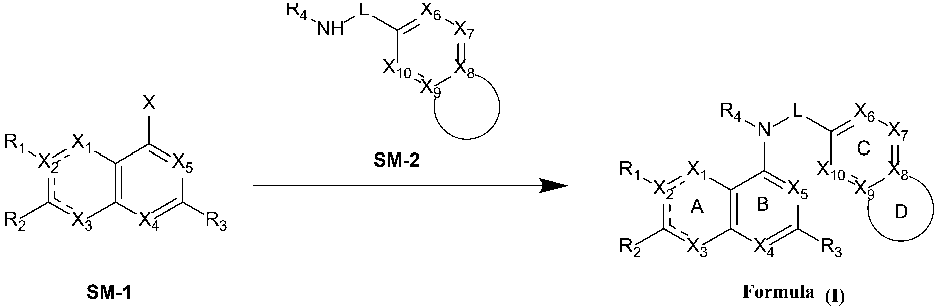 wherein X in SM-1 represents a leaving group selected from a halogen, an activated hydroxyl group, and the like, and SM-1 and SM-2 react under the action of a suitable base such as TEA, DIPEA, and pyridine or a suitable acid such as TFA, with the preferred reaction temperature under base-catalyzed conditions being 20-40°C and the preferred reaction temperature under acid-catalyzed conditions being 70-90°C.

22. A preparation method for the compound of Formula (I), the stereoisomer thereof, the isotopic variant thereof, or the pharmaceutically acceptable salt or solvate of any one of the foregoing according to any one of claims 1-20, wherein the preparation method comprises the following synthetic route: 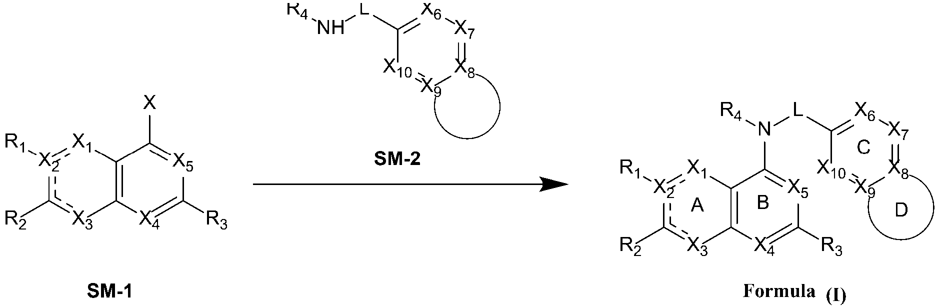 wherein X in SM-1 represents a leaving group selected from a halogen, an activated hydroxyl group, and the like, and SM-1 and SM-2 react under the action of a suitable base such as TEA, DIPEA, and pyridine or a suitable acid such as TFA and HCl, with the preferred reaction temperature under base-catalyzed conditions being 20-120°C and the preferred reaction temperature under acid-catalyzed conditions being 70-90°C.

23. A pharmaceutical composition comprising the compound of Formula (I), the stereoisomer thereof, the isotopic variant thereof, or the pharmaceutically acceptable salt or solvate of any one of the foregoing according to any one of claims 1-20, and a pharmaceutically acceptable carrier or excipient.

24. A method for inhibiting intracellular Arf1 pathway activity in a cell, comprising administering an effective amount of the compound of Formula (I), the stereoisomer thereof, the isotopic variant thereof, or the pharmaceutically acceptable salt or solvate of any one of the foregoing according to any one of claims 1-20 to the cell, thereby reducing Arf1 pathway activity in the cell.

25. The method according to claim 24, wherein the cell is a progenitor cell, a stem cell, a cancer stem cell, or a cancer cell.

26. The method according to claim 24 or 25, wherein the method induces cell death at the cellular level.

27. The method according to any one of claims 24-26, wherein the method is performed *in vitro.*

28. The method according to any one of claims 24-26, wherein the method is performed *in vivo.*

29. A use of the compound of Formula (I), the stereoisomer thereof, the isotopic variant thereof, or the pharmaceutically acceptable salt or solvate of any one of the foregoing according to any one of claims 1-20 in the manufacture of an agent for inhibiting intracellular Arf1 pathway activity in a cell.

30. The use according to claim 29, wherein the cell is a progenitor cell, a stem cell, a cancer stem cell, or a cancer cell.

31. The use according to claim 29 or 30, wherein the agent induces cell death at the cellular level.

32. The use according to any one of claims 29-31, wherein the agent is administered to the cell *in vitro.*

33. The use according to any one of claims 29-31, wherein the agent is administered to the cell *in vivo.*

34. A method for treating or preventing a disease associated with Arf1 pathway activity in a subject, comprising administering an effective amount of the compound of Formula (I), the stereoisomer thereof, the isotopic variant thereof, or the pharmaceutically acceptable salt or solvate of any one of the foregoing according to any one of claims 1-20 to the subject.

35. The method according to claim 34, wherein the disease associated with Arf1 pathway activity is cancer or a tumor.

36. The method according to claim 35, wherein the cancer or the tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

37. The method according to claim 34, wherein the disease associated with Arf1 pathway activity is selected from the group consisting of autoimmune diseases, inflammatory diseases, inflammatory bowel disease, arthritis, autoimmune demyelinating disorders, Alzheimer's disease, amyotrophic lateral sclerosis, stroke, ischemia-reperfusion injury, multiple sclerosis, other neurodegenerative diseases, and the like.

38. The method according to any one of claims 34-37, wherein the subject is a mammal, such as a human.

39. The method according to any one of claims 34-38, wherein the method further comprises coadministering at least one anti-PD-1 antibody.

40. The method according to claim 39, wherein the compound of Formula (I), the stereoisomer thereof, the isotopic variant thereof, or the pharmaceutically acceptable salt or solvate of any one of the foregoing according to any one of claims 1-20 is administered simultaneously with or sequentially in any order with at least one immune checkpoint inhibitor, such as an anti-PD-1 antibody.

41. The compound of Formula (I), the stereoisomer thereof, the isotopic variant thereof, or the pharmaceutically acceptable salt or solvate of any one of the foregoing according to any one of claims 1-20 for use in treating or preventing a disease associated with Arf1 pathway activity in a subject.

42. The compound of Formula (I), the stereoisomer thereof, the isotopic variant thereof, or the pharmaceutically acceptable salt or solvate of any one of the foregoing according to any one of claims 1-20 for use in treating or preventing a disease associated with Arf1 pathway activity in a subject according to claim 41, wherein the disease associated with Arf1 pathway activity is cancer or a tumor.

43. The compound of Formula (I), the stereoisomer thereof, the isotopic variant thereof, or the pharmaceutically acceptable salt or solvate of any one of the foregoing according to any one of claims 1-20 for use in treating or preventing a disease associated with Arf1 pathway activity in a subject according to claim 42, wherein the cancer or the tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

44. The compound of Formula (I), the stereoisomer thereof, the isotopic variant thereof, or the pharmaceutically acceptable salt or solvate of any one of the foregoing according to any one of claims 1-20 for use in treating or preventing a disease associated with Arf1 pathway activity in a subject according to claim 41, wherein the disease associated with Arf1 pathway activity is selected from the group consisting of autoimmune diseases, inflammatory diseases, inflammatory bowel disease, arthritis, autoimmune demyelinating disorders, Alzheimer's disease, amyotrophic lateral sclerosis, stroke, ischemia-reperfusion injury, multiple sclerosis, other neurodegenerative diseases, and the like.

45. The compound of Formula (I), the stereoisomer thereof, the isotopic variant thereof, or the pharmaceutically acceptable salt or solvate of any one of the foregoing according to any one of claims 1-20 for use in treating or preventing a disease associated with Arf1 pathway activity in a subject according to any one of claims 41-44, wherein the subject is a mammal, such as a human.

46. The compound of Formula (I), the stereoisomer thereof, the isotopic variant thereof, or the pharmaceutically acceptable salt or solvate of any one of the foregoing according to any one of claims 1-20 for use in treating or preventing a disease associated with Arf1 pathway activity in a subject according to any one of claims 41-45, wherein the compound of Formula (I), the stereoisomer thereof, the isotopic variant thereof, or the pharmaceutically acceptable salt or solvate of any one of the foregoing according to any one of claims 1-20 is administered in combination with at least one immune checkpoint inhibitor, such as an anti-PD-1 antibody.

47. The compound of Formula (I), the stereoisomer thereof, the isotopic variant thereof, or the pharmaceutically acceptable salt or solvate of any one of the foregoing according to any one of claims 1-20 for use in treating or preventing a disease associated with Arf1 pathway activity in a subject according to claim 46, wherein the compound of Formula (I), the stereoisomer thereof, the isotopic variant thereof, or the pharmaceutically acceptable salt or solvate of any one of the foregoing according to any one of claims 1-20 is administered simultaneously with or sequentially in any order with at least one immune checkpoint inhibitor, such as an anti-PD-1 antibody.

48. A use of the compound of Formula (I), the stereoisomer thereof, the isotopic variant thereof, or the pharmaceutically acceptable salt or solvate of any one of the foregoing according to any one of claims 1-20 in the manufacture of a medicament for treating or preventing a disease associated with Arf1 pathway activity in a subject.

49. The use according to claim 48, wherein the disease associated with Arf1 pathway activity is cancer or a tumor.

50. The use according to claim 49, wherein the cancer or the tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

51. The use according to claim 48, wherein the disease associated with Arf1 pathway activity is selected from the group consisting of autoimmune diseases, inflammatory diseases, inflammatory bowel disease, arthritis, autoimmune demyelinating disorders, Alzheimer's disease, amyotrophic lateral sclerosis, stroke, ischemia-reperfusion injury, multiple sclerosis, other neurodegenerative diseases, and the like.

52. The use according to any one of claims 48-51, wherein the subject is a mammal, such as a human.

53. A kit for use in treating or preventing a disease associated with Arf1 pathway activity in a subject, comprising the compound of Formula (I), the stereoisomer thereof, the isotopic variant thereof, or the pharmaceutically acceptable salt or solvate of any one of the foregoing according to any one of claims 1-20, or the pharmaceutical composition according to claim 23; a container; and optionally a package insert or label indicating the treatment.

54. The kit for use according to claim 53, wherein the disease associated with Arf1 pathway activity is cancer or a tumor.

55. The kit for use according to claim 54, wherein the cancer or the tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

56. The kit for use according to claim 53, wherein the disease associated with Arf1 pathway activity is selected from the group consisting of autoimmune diseases, inflammatory diseases, inflammatory bowel disease, arthritis, autoimmune demyelinating disorders, Alzheimer's disease, amyotrophic lateral sclerosis, stroke, ischemia-reperfusion injury, multiple sclerosis, other neurodegenerative diseases, and the like.

57. The kit for use according to any one of claims 53-56, wherein the subject is a mammal, such as a human.

58. The kit for use according to any one of claims 53-57, wherein the kit further comprises at least one immune checkpoint inhibitor, such as an anti-PD-1 antibody.

59. A kit for use in diagnosing a disease associated with Arf1 pathway activity in a subject, comprising at least one compound of Formula (I), the stereoisomer thereof, the isotopic variant thereof, or the pharmaceutically acceptable salt or solvate of any one of the foregoing according to any one of claims 1-20 as a test agent.

60. The kit for use according to claim 59, wherein the test agent detects at least one indicative biomarker of the presence of a disease associated with Arf1 pathway activity.

61. The kit for use according to claim 59 or 60, wherein the test agent is capable of detecting Arf1 GTPase activity, serving as a reporter gene for detecting lipolytic activity, detecting lipid droplet formation, detecting autophagy activity, or acting as an upstream or downstream surrogate modulator of Arf1 activity or function.

62. The kit for use according to any one of claims 59-61, wherein the disease associated with Arf1 pathway activity is cancer or a tumor.

63. The kit for use according to claim 62, wherein the cancer or the tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

64. The kit for use according to any one of claims 59-61, wherein the disease associated with Arf1 pathway activity is selected from the group consisting of autoimmune diseases, inflammatory diseases, inflammatory bowel disease, arthritis, autoimmune demyelinating disorders, Alzheimer's disease, amyotrophic lateral sclerosis, stroke, ischemia-reperfusion injury, multiple sclerosis, other neurodegenerative diseases, and the like.

65. The kit for use according to any one of claims 59-64, wherein the subject is a mammal, such as a human.

66. The compound of Formula (I), the stereoisomer thereof, the isotopic variant thereof, or the pharmaceutically acceptable salt or solvate of any one of the foregoing according to any one of claims 1-20 for use in the manufacture of a therapeutic vaccine for blocking tumor development.

67. The compound of Formula (I), the stereoisomer thereof, the isotopic variant thereof, or the pharmaceutically acceptable salt or solvate of any one of the foregoing according to any one of claims 1-20 for use in the manufacture of a therapeutic vaccine for blocking tumor development according to claim 66, wherein the tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

68. A use of the compound of Formula (I), the stereoisomer thereof, the isotopic variant thereof, or the pharmaceutically acceptable salt or solvate of any one of the foregoing according to any one of claims 1-20 in the manufacture of a therapeutic vaccine for blocking tumor development.

69. The use according to claim 68, wherein the tumor is selected from the group consisting of breast cancer, head and neck cancer, lung cancer, ovarian cancer, pancreatic cancer, colorectal cancer, prostate cancer, renal cell carcinoma, melanoma, hepatocellular carcinoma, cervical cancer, sarcoma, brain tumor, gastric cancer, multiple myeloma, leukemia, lymphoma, and the like.

70. A method for preparing a therapeutic vaccine for blocking tumor development, comprising contacting the compound of Formula (I), the stereoisomer thereof, the isotopic variant thereof, or the pharmaceutically acceptable salt or solvate of any one of the foregoing according to any one of claims 1-20 with a cancer stem cell, thereby converting the cancer stem cell into a therapeutic vaccine.

71. A vaccination method, comprising administering the therapeutic vaccine prepared by the method according to claim 70 to a subject.

72. The vaccination method according to claim 71, wherein the subject is a mammal, such as a human.

73. A method for killing cells and inducing an anti-tumor immune response, comprising inhibiting at least one Arf1 in cells, particularly the activity of the COPI/Arf1-lipolysis-β-oxidation pathway, via an Arf1 pathway inhibitor.

74. The method according to claim 73, wherein the cell is a progenitor cell, a stem cell, a cancer stem cell, or a cancer cell.

75. The method according to claim 73 or 74, wherein the Arf1 pathway inhibitor is selected from the group consisting of a small molecule Arf1 inhibitor, an RNAi agent targeting Arf1, an antisense agent targeting Arf1, a peptidomimetic Arf1 inhibitor, and a DNA aggregation deoxynucleotide inhibitor containing a target protein Arf1.

76. The method according to claim 75, wherein the small molecule Arf1 inhibitor is the compound of Formula (I), the stereoisomer thereof, the isotopic variant thereof, or the pharmaceutically acceptable salt or solvate of any one of the foregoing according to any one of claims 1-20.
